(19) Europäisches Patentamt
European Patent Office
Office européen des brevets

(11) **EP 3 562 592 B1**

(12) **EUROPEAN PATENT SPECIFICATION**

(45) Date of publication and mention
of the grant of the patent:
**23.04.2025   Bulletin 2025/17**

(21) Application number: **17887706.4**

(22) Date of filing: **22.12.2017**

(51) International Patent Classification (IPC):
**B01L 3/00** *(2006.01)*        **B01L 7/00** *(2006.01)*
**C12M 1/00** *(2006.01)*        **C12M 1/26** *(2006.01)*
**C12Q 1/686** *(2018.01)*

(52) Cooperative Patent Classification (CPC):
(C-Sets available)
**B01L 3/50273; B01L 3/502715; B01L 7/5255;
C12Q 1/686;** B01L 2400/0481              (Cont.)

(86) International application number:
**PCT/US2017/068311**

(87) International publication number:
**WO 2018/125835 (05.07.2018 Gazette 2018/27)**

(54) **FAST PCR WITH MOLECULAR CROWDING**

SCHNELLE PCR MIT MOLEKULAREM CROWDING

PCR RAPIDE AVEC ENCOMBREMENT MOLÉCULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.12.2016   US 201662440037 P**

(43) Date of publication of application:
**06.11.2019   Bulletin 2019/45**

(60) Divisional application:
**25163974.6**

(73) Proprietor: **BioFire Defense, LLC
Salt Lake City, UT 84107 (US)**

(72) Inventors:
• **PASKO, Christopher Paul**
  **Salt Lake City, Utah 84105 (US)**
• **PORITZ, Mark Aaron**
  **Salt Lake City, Utah 84103 (US)**
• **WERNEREHL, Aaron**
  **Salt Lake City, Utah 84115 (US)**

(74) Representative: **Henderson, Helen Lee
Withers & Rogers LLP
2 London Bridge
London SE1 9RA (GB)**

(56) References cited:
**WO-A1-2008/018839        WO-A1-2016/196827
WO-A2-2013/177429        US-A1- 2011 076 674
US-A1- 2015 118 715        US-B2- 8 568 982**

• **SASAKI YOSHIHARU ET AL: "Effect of molecular
crowding on DNA polymerase activity",
BIOTECHNOLOGY JOURNAL, WILEY-VCH
VERLAG, WEINHEIM, DE, vol. 1, no. 4, 1 April
2006 (2006-04-01), pages 440 - 446,
XP002490677, ISSN: 1860-6768, DOI: 10.1002/
BIOT.200500032**
• **WITTWER C T ET AL: "Minimizing the time
required for DNA amplification by efficient heat
transfer to small samples", ANALYTICAL
BIOCHEMISTRY, ACADEMIC PRESS,
AMSTERDAM, NL, vol. 186, no. 2, 1 May 1990
(1990-05-01), pages 328 - 331, XP024823234,
ISSN: 0003-2697, [retrieved on 19900501], DOI:
10.1016/0003-2697(90)90090-V**

Remarks:
The complete document including Reference
Table(s) and the Sequence Listing(s) can be
downloaded from the EPO website

(52) Cooperative Patent Classification (CPC): (Cont.)

C-Sets
**C12Q 1/686, C12Q 2527/125, C12Q 2527/153,
C12Q 2565/629**

**Description**

CROSS-REFERENCE TO RELATED APPLICATIONS

**BACKGROUND**

Polymerase chain reaction (PCR) is a technique widely used in molecular biology. It derives its name from one of its key components, a DNA polymerase used to amplify a piece of DNA by in vitro enzymatic replication. As PCR progresses, the DNA generated (the amplicon) is itself used as a template for replication. This sets in motion a chain reaction in which the DNA template is exponentially amplified. With PCR, it is possible to amplify a single or few copies of a piece of DNA across several orders of magnitude, generating millions or more copies of the DNA piece. PCR employs a thermostable polymerase, dNTPs, and a pair of primers.

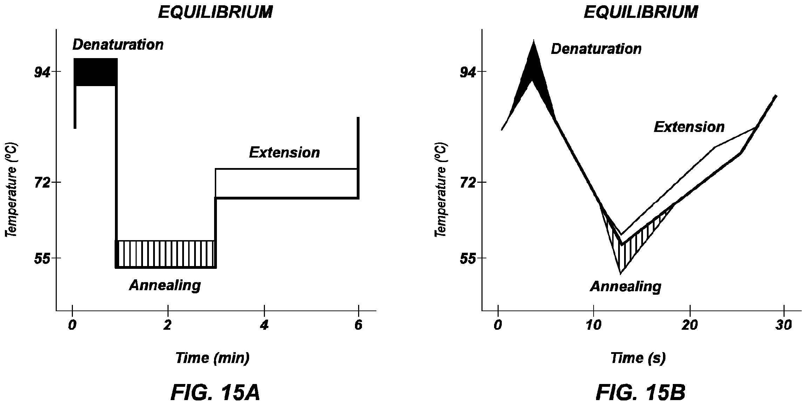

PCR is conceptually divided into 3 reactions, each usually assumed to occur over time at each of three temperatures. Such an "equilibrium paradigm" of PCR is easy to understand in terms of three reactions (denaturation, annealing, and extension) occurring at 3 temperatures over 3 time periods each cycle. However, this equilibrium paradigm does not fit well with physical reality. Instantaneous temperature changes do not occur; it takes time to change the sample temperature. Furthermore, individual reaction rates vary with temperature, and once primer annealing occurs, polymerase extension immediately follows. More accurate, particularly for rapid PCR, is a kinetic paradigm where reaction rates and temperature are always changing. Holding the temperature constant during PCR is not necessary as long as the products denature and the primers anneal. Under the kinetic paradigm of PCR, product denaturation, primer annealing, and polymerase extension may temporally overlap and their rates continuously vary with temperature. Under the equilibrium paradigm, a cycle is defined by 3 temperatures each held for a time period, whereas the kinetic paradigm requires transition rates and target temperatures. Illustrative time/temperature profiles for the equilibrium and kinetic paradigms are shown in Figs. 15a-15b. However, it is understood that these temperature profiles are illustrative only and that in some implementations of PCR, the annealing and extension steps are combined so that only 2 temperatures are needed.

Paradigms are not right or wrong, but they vary in their usefulness. The equilibrium paradigm is simple to understand and lends itself well to the engineering mindset and instrument manufacture. The kinetic paradigm is more relevant to biochemistry, rapid cycle PCR, and melting curve analysis.

When PCR was first popularized in the late 1980s, the process was slow. A typical protocol was 1 minute for denaturation at 94°C, 2 minutes for annealing at 55°C, and 3 minutes for extension at 72°C. When the time for transition between temperatures was included, 8 minute cycles were typical, resulting in completion of 30 cycles in 4 hours. Twenty-five percent of the cycling time was spent in temperature transitions. As cycling speeds increased, the proportion of time spent in temperature transitions also increased and the kinetic paradigm became more and more relevant. During rapid cycle PCR, the temperature is usually changing. For rapid cycle PCR of short products (<100 bps), 100% of the time may be spent in temperature transition and no holding times are necessary. For rapid cycle PCR of longer products, a temperature hold at an optimal extension temperature may be included.

In isolation, the term "rapid PCR" is both relative and vague. A 1 hour PCR is rapid compared to 4 hours, but slow compared to 15 minutes. Furthermore, PCR protocols can be made shorter if one starts with higher template concentrations or uses fewer cycles. A more specific measure is the time required for each cycle. Thus, "rapid cycle PCR" (or "rapid cycling") was defined in 1994 as 30 cycles completed in 10-30 minutes (1), resulting in cycles of 20-60 seconds each. This actual time of each cycle is longer than the sum of the times often programmed for denaturation, annealing and extension, as time is needed to ramp the temperatures between each of these stages. Initial work in the early 1990s established the feasibility of rapid cycling using capillary tubes and hot air for temperature control. Over the years, systems have become faster, and the kinetic requirements of denaturation, annealing, and extension have become clearer.

In one early rapid system, a heating element and fan from a hair dryer, a thermocouple, and PCR samples in capillary tubes were enclosed in a chamber (2). The fan created a rapid flow of heated air past the thermocouple and capillaries. By matching the thermal response of the thermocouple to the sample, the temperature of the thermocouple closely tracked the temperature of the samples, even during temperature changes. Although air has a low thermal conductivity, rapidly moving air against the large surface area exposed by the capillaries was adequate to cycle the sample between denaturation, annealing, and extension temperatures. Electronic controllers monitored the temperature, adjusted the power to the heating element, and provided the required timing and number of cycles. For cooling, the controller activated a solenoid that opened a portal to outside air, introducing cooling air to the otherwise closed chamber.

Temperatures could be rapidly changed using the capillary/air system. Using a low thermal mass chamber, circulating air, and samples in glass capillaries, PCR products >500 bp were visualized on ethidium bromide stained gels after only 10 minutes of PCR (30 cycles of 20 seconds each) (3). Product yield was affected by the extension time and the concentration of polymerase. With 30 second cycle times (about 10 seconds between 70 and 80°C for extension), the band intensity increased as the polymerase concentration was increased from 0.1 to 0.8 Units per 10 $\mu$l reaction. It is noted that polymerase Unit definitions can be confusing. For native Taq polymerase, 0.4 U/10 $\mu$l is about 1.5 nM under typical rapid cycling conditions (50).

Rapid protocols use momentary or "0" second holds at the denaturation and annealing temperatures. That is, the temperature-time profiles show temperature spikes for denaturation and annealing, without holding the top and bottom temperatures. Denaturation and annealing can occur very quickly.

Rapid and accurate control of temperature allowed analytical study of the required temperatures and times for PCR. For an illustrative 536 bp fragment of human genomic DNA (β-globin), denaturation temperatures between 91°C and 97°C were equally effective, as were denaturation times from <1 second to 16 seconds. However, it was found that denaturation times longer than 16 seconds actually decreased product yield. Specific products in good yield were obtained with annealing temperatures of 50-60°C, as long as the time for primer annealing was limited. That is, best specificity was obtained by rapid cooling from denaturation to annealing and an annealing time of <1 second. Yield was best at extension temperatures of 75-79°C, and increased with extension time up to about 40 seconds.

Conclusions from this early work were: 1) denaturation of PCR products is very rapid with no need to hold the denaturation temperature, 2) annealing of primers can occur very quickly and annealing temperature holds may not be necessary, and 3) the required extension time depends on PCR product length and polymerase concentration. Also, rapid cycle PCR is not only faster, but better in terms of specificity and yield (4, 5) as long as the temperature was controlled precisely. PCR speed is not limited by the available biochemistry, but by instrumentation that does not control the sample temperature closely or rapidly.

However, most current laboratory PCR instruments perform poorly with momentary denaturation and annealing times, and many don't even allow programming of "0" second holding periods. Time delays from thermal transfer through the walls of conical tubes, low surface area-to-volume ratios, and heating of large samples force most instruments to rely on extended times at denaturation and annealing to assure that the sample reaches the desired temperatures. With these time delays, the exact temperature vs time course becomes indefinite. The result is limited reproducibility within and high variability between commercial products (6). Many instruments show marked temperature variance during temperature transitions (7, 8). Undershoot and/or overshoot of temperature is a chronic problem that is seldom solved by attempted software prediction that depends on sample volume. Such difficulties are compounded by thermal properties of the instrument that may change with age.

Over time, conventional heat block instruments have become faster, with incremental improvements in "thin wall" tubes, more conductive heat distribution between samples, low thermal mass blocks and other "fast" modifications. Nevertheless, it is unusual for these systems to cycle rapidly enough to complete a cycle in less than 60 seconds. A few heat block systems can achieve <60 second cycles, usually restricted to 2-temperature cycling between a limited range of temperatures. By flattening the sample container, rapid cycling can be achieved by resistive heating and air cooling (9), or by moving the sample in a flexible tube between heating zones kept at a constant temperature (U.S. Patent No. 6,706,617).

Commercial versions of the air/capillary system for PCR have been available since 1991 (1) and for real-time PCR since 1996 (10, 11). Rapid cycling capabilities of other instruments are often compared against the air/capillary standard that first demonstrated 20-60 second cycles. Oddly enough, there has been a trend to run the capillary/air systems slower over the years, perhaps reflecting discomfort with "0" second denaturation and annealing times by many users. Also, heat-activated enzymes require long activation periods, often doubling run times even when "fast" activation enzymes are used. Another compromise away from rapid cycling is the use of plastic capillaries. These capillaries are not thermally matched to the instrument, so 20 second holds at denaturation and annealing are often required to reach the target temperatures (12).

Some progress in further decreasing the cycle times for PCR has occurred in microsystems, where small volumes are naturally processed (13, 14). However, even with high surface area-to-volume sample chambers, cycles may be long if the heating element has a high thermal mass and is external to the chamber (15). With thin film resistive heaters and temperature sensors close to the samples, 10-30 minute amplification can be achieved (16, 17).

While cooling of low thermal mass systems is usually by passive thermal diffusion and/or by forced air, several interesting heating methods have been developed. Infrared radiation can be used for heating (18) with calibrated infrared pyrometry for temperature monitoring (19). Alternatively, thin metal films on glass capillaries can serve as both a resistive heating element and a temperature sensor for rapid cycling (20). Finally, direct Joule heating and temperature monitoring of the PCR solution by electrolytic resistance is possible and has been implemented in capillaries (21). All of the above methods transfer heat to and from fixed samples.

Instead of heat transfer to and from stationary samples, the samples can be physically moved to different temperature baths, or through channels with fixed temperature zones. Microfluidic methods have become popular, with the PCR fluid passing within channels through different segments kept at denaturation, annealing, and extension temperatures. Continuous flow PCR has been demonstrated within serpentine channels that pass back and forth through 3 temperature zones (22) and within loops of increasing or decreasing radius that pass through 3 temperature sectors (23). A variant with a serpentine layout uses stationary thermal gradients instead of isothermal zones, to more closely fit the kinetic paradigm of PCR (24). To limit the length of the microchannel necessary for PCR, some systems shuttle samples back and forth between temperature zones by bi-directional pressure-driven flow (25), pneumatics (26), or electrokinetic forces (27). Instead of linear shuttling of samples, a single circular channel can be used with sample movement driven as a magnetic ferrofluid (28) or by convection (29). One potential advantage of microsystem PCR, including continuous flow methods, is cycling speed.

Although some microsystems still require >60 second cycles, many operate in the 20-60 second cycle range of rapid cycle PCR (13, 30). Minimum cycle times ranging from 16-37 seconds have been reported for infrared heating (18, 19). Metal coated capillaries have achieved 40 second PCR cycles (20), while direct electrolytic heating has amplified with 21 second cycles (20). Minimum cycle times reported for closed loop convective PCR range from 24-42 seconds (29, 31). Several groups have focused on reducing PCR cycle times to <20 seconds, faster than the original definition of rapid cycle PCR that was first demonstrated in 1990. Thin film resistive heating of stationary samples has reduced cycle times down to 17 seconds for 25 $\mu$l samples (32) and 8.5 seconds for 100 nl samples (17). Continuous flow systems have achieved 12-14 second cycles with thermal gradient PCR (24) and sample shuttling (26), while a ferrofluid loop claims successful PCR with 9 second cycles (28). Continuous flow systems through glass and plastic substrates have achieved cycle times of 6.9 seconds (22) and 5.2 seconds (23) for various size PCR products. Alternating hot and cool water conduction through an aluminum substrate amplified 1 $\mu$l droplets under oil with 5.25 second cycles (33). Similarly, water conduction through a porous copper block amplified 5 $\mu$l samples with 4.6 second cycles (34). A continuous flow device of 1 $\mu$l reaction plugs augmented by vapor pressure achieved 3 second cycles (35). Additionally, there are reports that claim to amplify an 85 bp fragment of the Stx bacteriophage of E. coli in capillaries with 2.7 second cycles by immersion of the capillaries in gallium sandwiched between Peltier elements (36). Alternatively, PCR amplification in capillaries cycled by pressurized hot and cool gases obtained 2.6 second cycles (48).

Table 1 summarizes work to minimize PCR cycle times to less than the 20 second cycles that originally defined "Rapid PCR". Over the past 20 years, new prototype instruments have been developed that incrementally improve cycling speed. However, practical PCR performance (efficiency and yield) is often poor. As a general rule, as cycles become increasingly shorter, claims for successful PCR correlate with lower complexity targets (bacteria, phage, multicopy plasmids, or even PCR products) that are used at higher starting concentrations (see, e.g., U.S. Patent No. 6,210,882, wherein 5 ng of amplicon was used as the starting sample). Indeed, none of the studies listed in Table 1 with <20 second cycles used complex eukaryotic DNA such as human DNA. The starting copy number of template molecules is often very high (e.g., 180,000,000 copies of lambda phage/$\mu$l), so that little amplification is needed before success is claimed. Furthermore, the lack of no template controls in many studies raises questions regarding the validity of positive results, especially in an environment with high template concentrations. One instrument-oriented report focuses extensively on the design and modeling of the thermal cycling device, with a final brief PCR demonstration using a high concentration of a low complexity target. Heating and cooling rates (up to 175°C/s) have been reported based on modeling and measurements without PCR samples present (17).

Table 1

| Fastest Cycle Time (s) | [Template] (Copies/μl) | Template Form | Total [Primers] (nM) | Polymerase | [Polymerase] (nM) | Product Length (bp) | Quantification | Trend | Method | No Template Control? | Reference |
|---|---|---|---|---|---|---|---|---|---|---|---|
| 20 | 1,600 | Human DNA | 1000 | 0.08 U/μl Taq | 3 | 536 | Faint Gel Band | Increases with [Polymerase] | Capillary Air Cycling | No | 3 |
| 12 | 40,000 | Lambda phage | 400 | 0.2U/μl Taq | 7.5 | 500 | Capillary Electrophoresis | ? | IR Heating, Pressurized Air Cooling | No | 56 |
| 12 | 1,000,000 | 230 bp PCR product | 1000 | 0.5 U/μl Taq | 19 | 230 | Good gel band | Dependent on cycle # and copy # | Continuous Flow | Yes | 55 |
| 9.25 | 4,700-470,000 | 18S rDNA (human genomic) | 1800 | Taq Gold | ? | 187 | ? | ? | IR Heating of droplets in oil | No | 54 |
| 9 | 18,000,000 | Lambda phage | 2000 | 0.025 U/μl Taq | 0.94 | 500 | OK gel band | Intensity increases with cycle time | Continuous Flow with a Ferrous Particle Plug | No | 28 |
| 8.5 | ? | cDNA | 1800 | ? | ? | 82 | 80% efficiency | Decreasing efficiency at faster cycles | Micromachined cantilever | ? | 17 |
| 7.0 | 10,000,000 | 1 KB PCR product | 2000 | 0.25 U/μl Taq | 9.4 | 176 | 7% of control | 50% at 15 s cycles | Continuous Flow | Yes | 22 |
| 6.3 | 10,000 | Plasmids (B. anthracis) | 1200 | 0.05 U/μl Ex Taq HS | ? | 134 | 55% of control | ? | Plug Continuous Flow | Yes | 53 |
| 5.2/9.7 | 180,000,000 | Lambda phage | 400 | 0.07 U/μl Taq | 2.6 | 500/997 | Faint gel bands | Dependent on cycle times | Continuous Flow | No | 23 |
| 5.25 | 1,400,000 | B. subtilis (bacterial DNA) | 500 | 0.025 U/μl KOD plus | ? | 72 | 90% efficiency (SYBR) | Single run | Water pumped against aluminum plate | Yes | 33 |
| 4.6 | 34,000 | E. herbicola (bacterial DNA) | 800 | 0.04 U/μl KAPA2G | 4 | 58/160 | Faint gel bands | Yield increases with # cycles | Water pumped through porous copper | No | 31 |

EP 3 562 592 B1

(continued)

| Fastest Cycle Time (s) | [Template] (Copies/μl) | Template Form | Total [Primers] (nM) | Polymerase | [Polymerase] (nM) | Product Length (bp) | Quantification | Trend | Method | No Template Control? | Reference |
|---|---|---|---|---|---|---|---|---|---|---|---|
| 4.2 | 50[1] | B. subtilis (bacterial DNA) | ? | KOD plus | ? | 72 | Cq = 33 (SYBR) | Higher copy # reduces Cq | IR laser | ?[2] | 51 |
| 3.0 | 10,000 | Plasmids (B. anthracis) | 1200 | 0.05 U/μl Ex Taq HS | ? | 134 | 15% of control | 80% at 7.5 s cycles | Constant flow with vapor pressure | Yes (5% signal) | 35 |
| 2.7 | ? | stx phage (E. coli) | ?[3] | KOD | ? | 85 | Barely visible band | Decreasing yield from 3.06 s to 2.69 s cycles | Gallium transfer from Peltiers to capillaries | No | 36 |
| 2.6 | ?[4] | stx phage (E. coli) | ?[5] | 0.5 U/μl Taq | 19 | 85 | Very dim band | Constant from 2.8 to 2.6 s cycles | Pressurized gas and capillaries | No | 48 |

[1]Presumed single copy in a 20 nl droplet with Cq of 33 under SYBR Green monitoring, but no gel or melting analysis to confirm PCR product identity. [2]A "Blank" sample was run, but it is not clear if this was a no template control.

[3]Article says [primer] is 0.5 mmol, patent application (US 2009/0275014 A1) says [primer] is 0.01 - 0.5 μM.

[4]Two pg E. coli DNA/μl, but copy number of phage in the DNA preparation is unknown.

[5]Dissertation says 0.5 μmol/10 μl (50 mM), patent (US 6,472,186) says 50 pmol/10 μl (5 μM).

EP 3 562 592 B1

One way to decrease cycle time is to introduce variations to the PCR protocol to ease the temperature cycling requirements. Longer primers with higher Tms allow higher annealing temperatures. By limiting the product length and its Tm, denaturation temperatures can be lowered to just above the product Tm. In combination, higher annealing and lower denaturation temperatures decrease the temperature range required for successful amplification. Reducing 3-step cycling (denaturation, annealing, and extension) to 2-steps (denaturation and a combined annealing/extension step) also simplifies the temperature cycling requirements. Both decreased temperature range and 2-step cycling are typical for the studies in Table 1 with cycle times <20 seconds. Two-step cycling can, however, compromise polymerase extension rates if the combined annealing/extension step is performed at temperatures lower than the 70 to 80°C temperature optimum where the polymerase is most active. Polymerase extension rates are log-linear with temperature until about 70-80°C, with a reported maximum of 60-120 bp/s (50).

Even with protocol variations, amplification efficiency and yield are often poor when cycle times are <20 seconds when compared to control reactions (22, 23). These efforts towards faster PCR appear dominated by engineering with little focus on the biochemistry. As cycle times decrease from 20 seconds towards 2 seconds, PCR yield decreases and finally disappears, reflecting a lack of robustness even with simple targets at high copy number.

The instrumentation in various references disclosed in Table 1 may be suitable for extremely fast PCR, if reaction conditions are compatible. As disclosed herein, a focus on increased concentrations of primers, polymerase, and Mg$^{++}$ allows for "extreme PCR" (PCR with <20 second cycles (30 cycles in <10 min)), while retaining reaction robustness and yield. Also as disclosed herein, a focus on increased concentrations of primers and polymerase are achieved by use of molecular crowders.

BRIEF SUMMARY

Described are methods are provided for amplifying a target nucleic acid in a biological sample, the methods comprising the steps of adding a thermostable polymerase, primers configured for amplification of the target nucleic acid, and a molecular crowder to the biological sample to create an amplification mixture; and amplifying the target nucleic acid by polymerase chain reaction by thermally cycling the amplification mixture between at least a denaturation temperature and an elongation temperature through a plurality of amplification cycles using an extreme temperature cycling profile wherein each cycle is completed in a cycle time less than 40 seconds per cycle. In various illustrative embodiments, the molecular crowder is provided in an amount that is at least 3%, 5%, 7.5% or more w/v of the amplification mixture. One illustrative molecular crowder is a Ficoll or mixture of Ficolls of different molecular weights. WO2008018839 describes the use of Ficoll in amplification reactions.

A method for amplifying a target nucleic acid in a biological sample is provided, the method comprising the steps of adding a thermostable polymerase, primers configured for amplification of the target nucleic acid, and a molecular crowder to the biological sample to create an amplification mixture; and amplifying the target nucleic acid by polymerase chain reaction by thermally cycling the amplification mixture between at least a denaturation temperature and an elongation temperature through a plurality of amplification cycles using an extreme temperature cycling profile wherein each cycle is completed in a cycle time less than 30 seconds per cycle. In various illustrative embodiments, the molecular crowder is provided in an amount between 5% and 50% w/v of the amplification mixture, the primers are each provided at a concentration of at least 0.5 $\mu$M in the amplification mixture, and the polymerase is provided at a concentration of at least 0.4U/$\mu$L of the amplification mixture. In various illustrative embodiments, the cycle time is no more than 10 seconds.

A container for conducting a reaction is provided, the container comprising a flexible material defining a plurality of fluidly connected reaction zones fluidly connected by channels, the fluidly connected reaction zones including at least a first-stage PCR reaction zone; the container comprising an amplification mixture for first-stage PCR in the first-stage PCR reaction zone, wherein the amplification mixture includes a thermostable polymerase, primers configured for amplification of a target nucleic acid, and a molecular crowder provided in an amount that is at least 3% w/v of the amplification mixture.

A mixture for amplifying a target nucleic acid is provided, the mixture comprising a thermostable polymerase and a molecular crowder, wherein the molecular crowder is used in an amount that is at least 3% w/v of an amplification mixture for the target nucleic acid. In various embodiments the mixture comprises at least one primer pair for amplifying the target nucleic acid.

In one more aspect of this disclosure a method for decreasing cycle time for a PCR mixture having a known cycling protocol is provided, the method comprising adding a molecular crowder to the PCR mixture, thermocycling the PCR mixture at a cycling time that is 5% to 50% faster than the known cycling protocol.

**BRIEF DESCRIPTION OF THE DRAWINGS**

In order to describe the manner in which certain advantages and features of the present disclosure can be obtained, a description of the disclosure will be rendered by reference to specific embodiments thereof which are illustrated in the appended drawings. Understanding that these drawings depict only typical embodiments of the disclosure and are not therefore to be considered to be limiting of its scope, the disclosure will be described and explained with additional specificity and detail through the use of the accompanying drawings in which:

Fig. 1A shows a schematic for performing extreme PCR.

Fig. 1B is an illustrative device for performing extreme PCR with real-time capabilities for monitoring one sample tube in a water bath.

Fig. 1C is an illustrative device for performing extreme PCR with three-temperature cycling.

Fig. 1D is a close up view of the optics of the device in Fig. 1B that also shows the temperature reference capillary.

Fig. 2A is a graph that superimposes the location of the sample holder (-----) of Fig. 1B with the temperature of the sample ( _____ ).

Fig. 2B is a temperature graph of extreme PCR using the device shown in Fig. 1B.

Fig. 2C is a temperature graph of rapid cycle PCR using a carousel LightCycler (Roche) shown for comparison against Fig. 2B.

Fig. 3A shows derivative melting curves of extreme PCR products (-----) and rapid cycle PCR products (-··-), with negative controls for extreme (-) and rapid (- - -) cycling, amplified using the temperature profile of Fig. 2B.

Fig. 3B is a 2% SeaKem LE agarose gel of the same samples of Fig. 3A, lanes 1 and 8 are size markers, lanes 2 and 3 are products resulting from 30 sec extreme PCR, lane 4 is a no template control for 30 sec extreme PCR, lanes 5 and 6 are products resulting from 12 min PCR, and lane 7 is the no template control for 12 min PCR.

Fig. 3C shows an extreme PCR temperature trace (-----) that amplified the same products shown in Fig. 3A and 3B, along with real-time monitoring ( _____ ) of the same reaction.

Fig. 4A shows an extreme PCR temperature trace that increases the extension rate by temperature control.

Fig. 4B shows a magnified portion of Fig. 4A, superimposing the location of the sample holder (-) of Fig. 1B with the temperature of the sample (-----).

Fig. 4C is a negative derivative melting curve (-dF/dT) of a 58 bp amplicon of IRL10RB, wherein AA (-), AG (- - -), and GG (-----) genotypes are shown.

Fig. 5A is a three dimensional graph plotting polymerase concentration vs. primer concentration vs. concentration of PCR product, using extreme PCR.

Fig. 5B is the extreme PCR temperature trace used in Fig. 5A.

Fig. 5C shows negative derivative melting curves of the 4 $\mu$M KlenTaq polymerase (KT POL) products from Fig. 5A.

Fig. 5D is an agarose gel showing results of extreme PCR using varying polymerase concentrations at 10 $\mu$M primer concentrations from Fig. 5A.

Fig. 6A is a temperature trace of extreme PCR performed in a 19 gauge stainless steel tube.

Fig. 6B is a gel of the PCR products produced by the extreme temperature cycles of Fig. 6A.

Fig. 7A is an extreme PCR temperature trace with a long (1 second) combined annealing/extension step.

Fig. 7B is a three dimensional graph plotting polymerase concentration vs. primer concentration vs. concentration of PCR product, using extreme PCR for a 102 bp product.

Fig. 8A shows an extreme PCR temperature profile used to amplify a 226 bp product, using a one second combined annealing/extension step.

Fig. 8B shows an extreme PCR temperature profile used to amplify a 428 bp product, using a four second combined annealing/extension step.

Fig. 8C shows the real time results obtained from Fig. 8A and a similar temperature trace using a 2 second annealing/extension step, including no template controls for each.

Fig. 8D shows the real time results obtained from Fig. 8B and a similar temperature trace using a 5 second annealing/extension step, including no template controls for each.

Fig. 9A shows amplification curves of a 45 bp fragment of KCNE1 at different starting concentrations.

Fig. 9B is a plot of Cq versus $\log_{10}$ (initial template copies) of the data from Fig. 9A. Reactions were performed in quintuplicate.

Figs. 9C-9D are similar to Figs. 9A-9B, except showing amplification of a 102 bp fragment of NQO1.

Fig. 10A is a three dimensional graph plotting polymerase concentration vs. primer concentration vs. concentration of PCR product, using extreme PCR for a 300 bp product (20 cycles, 4.9 seconds per cycle).

Fig. 10B shows fluorescence versus cycle number plots for PCR amplification of a 500 bp synthetic template using KAPA2G FAST polymerase and 1-5 second extension times.

Fig. 10C is a plot of extension length vs. minimum extension time for several KlenTaq polymerase concentrations and KAPA2G FAST polymerase.

Figs. 11A-11E show fluorescence versus cycle number plots for PCR amplification of products of size: 100 bp (Fig. 11A), 200 bp (Fig. 11B), 300 bp (Fig. 11C), 400 bp (Fig. 11D), and 500 bp (Fig. 11E).

Fig. 12A shows negative derivative melting curves of a 60 bp fragment of AKAP10 after 35 cycles of extreme PCR, using varying magnesium concentrations.

Fig. 12B is a gel of the PCR products shown in the negative derivative melting curves of Fig. 12A.

Fig. 13A shows negative derivative melting curves of a 60 bp fragment of AKAP10 after 35 cycles, using varying cycle times with 5 mM $Mg^{++}$. Cycle times were 0.32 seconds (- -), 0.42 seconds (——··——), 0.52 seconds (—— – ——), and 0.62 seconds (-----). Cycle times included a 0.1 to 0.4 second hold in a 60°C bath.

Fig. 13B is a gel of the PCR products shown in the negative derivative melting curves of Fig. 13A.

Fig. 14A shows negative derivative melting curves of a 60 bp fragment of AKAP10, as amplified on three different instruments: (1) extreme PCR, (2) LightCycler, and (3) CFX96 (Bio-Rad).

Fig. 14B is a gel of the PCR products shown in the negative derivative melting curves of Fig. 14A.

Figs. 15A-15B show illustrative profiles for an equilibrium paradigm (Fig. 15A) and a kinetic paradigm (Fig. 15B) of PCR. Solid black represents denaturation, striped represents annealing, and solid white represents extension of the nucleic acids during thermal cycling.

Fig. 16 shows a sample vessel ("pouch") used in various examples herein. The pouch is suitable for use on the FilmArray® Instrument (BioFire Diagnostics, LLC).

Fig. 17 shows results for three yeast assays in a test pouch of Fig. 16, wherein each $\times$ is the Cp for a run at standard conditions, each is the Cp for a run at fast conditions, each $\triangle$ is the Cp for a run at standard conditions with molecular crowders, and each $\bigcirc$ is the Cp for a run at fast conditions with molecular crowders.

Fig. 18 is similar to Fig. 17, but showing the results for eleven assays and two controls, wherein the same symbols are used represent the same conditions.

Figs. 19A-19K show the same eleven assays as in Fig. 18, with different concentrations of the molecular crowders, with 0.5μM primers ($\bigcirc$), 2.5μM primers ($\square$), and standard conditions ($\times$).

Fig. 20 shows seven of the same assays with 0.5μM primers and various mixtures of molecular crowders. The Cp was normalized to an average for each target, where each data point is the mean Cp for each mixture subtracted from the mean Cp of three pouch runs. Since the data are centered at zero, negative values performed better than average (earlier Cp) and positive values performed worse (later Cp).

Fig. 21 shows the Cp of 30 assays in a prototype biothreat panel, with standard cycling conditions without molecular crowders ($\square$), with fast cycling conditions without molecular crowders ($\square$), and fast cycling conditions with three different ratios of Ficolls: 6% Ficoll 70 / 21% Ficoll 400 ($\times$), 30% Ficoll 70 / 2% Ficoll 400 ($\triangle$), and 21% Ficoll 70 / 6% Ficoll 400 (+).

Fig. 22 shows the effects of polymerase concentration on four of the assays from the pouch of Fig. 18. All pouches were run at fast cycling conditions, using 1x KlenTaq without molecular crowders, 1x KlenTaq with molecular crowders, 10x KlenTaq without molecular crowders, and 10x KlenTaq with molecular crowders.

## DETAILED DESCRIPTION

As used herein, the terms "a," "an," and "the" are defined to mean one or more and include the plural unless the context is inappropriate. Ranges may be expressed herein as from "about" one particular value, and/or to "about" another particular value. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 5%. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

The word "or" as used herein means any one member of a particular list and also includes any combination of members of that list.

As used herein, the transitional phrase "consisting essentially of" means that the scope of a claim is to be interpreted to encompass the specified materials or steps recited in the claim, "and those that do not materially affect the basic and novel characteristic(s)" of the claimed invention. *See, In re Herz,* 537 F.2d 549, 551-52, 190 U.S.P.Q. 461, 463 (CCPA 1976) (emphasis in the original); *see also* MPEP § 2111.03. Thus, the term "consisting essentially of" when used in a claim of this invention is not intended to be interpreted to be equivalent to "comprising".

As used herein, the term "fluidly connected," is synonymous with "fluidically connected," "fluid coupled," and "in fluid communication," and refers to a connection between components that allows for a fluid to pass therebetween.

By "sample" is meant an animal; a tissue or organ from an animal; a cell (either within a subject, taken directly from a subject, or a cell maintained in culture or from a cultured cell line); a cell lysate (or lysate fraction) or cell extract; a solution containing one or more molecules derived from a cell, cellular material, or viral material (e.g. a polypeptide or nucleic acid); or a solution containing a naturally or non-naturally occurring nucleic acid, which is assayed as described herein. A sample may also be any body fluid or excretion (for example, but not limited to, blood, urine, stool, saliva, tears, bile) that contains cells, cell components, or nucleic acids.

The phrase "nucleic acid" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acids of the invention can also include nucleotide analogs (e.g., BrdU, dUTP, 7-deaza-dGTP), and non-phosphodiester internucleoside linkages (e.g., peptide nucleic acid (PNA) or thiodiester linkages). In particular, nucleic acids can include, without limitation, DNA, RNA, cDNA, gDNA, ssDNA, dsDNA or any combination thereof.

By "probe," "primer," or "oligonucleotide" is meant a single-stranded DNA or RNA molecule of defined sequence that can base-pair to a second DNA or RNA molecule that contains a complementary sequence (the "target"). The stability of the resulting hybrid depends upon the length, GC content, nearest neighbor stacking energy, and the extent of the base-pairing that occurs. The extent of base-pairing is affected by parameters such as the degree of complementarity between the probe and target molecules and the degree of stringency of the hybridization conditions. The degree of hybridization stringency is affected by parameters such as temperature, salt concentration, and the concentration of organic molecules such as formamide, and is determined by methods known to one skilled in the art. Probes, primers, and oligonucleotides may be detectably-labeled, either radioactively, fluorescently, or non-radioactively, by methods well-known to those skilled in the art. dsDNA binding dyes (dyes that fluoresce more strongly when bound to double-stranded DNA than when bound to single-stranded DNA or free in solution) may be used to detect dsDNA. It is understood that a "primer" is specifically configured to be extended by a polymerase, whereas a "probe" or "oligonucleotide" may or may not be so configured.

By "specifically hybridizes" is meant that a probe, primer, or oligonucleotide recognizes and physically interacts (that is, base-pairs) with a substantially complementary nucleic acid (for example, a sample nucleic acid) under high stringency conditions, and does not substantially base pair with other nucleic acids.

By "high stringency conditions" is meant typically occuring at about melting temperature (Tm) minus 5°C (i.e. 5° below the Tm of the probe). Functionally, high stringency conditions are used to identify nucleic acid sequences having at least 80% sequence identity.

In an illustrative embodiment, methods and kits are provided for PCR with <20 second cycle times, with some embodiments using <10 second, <5 second, <2 second, <1 second, and <0.5 second cycle times. With these cycle times, a 30 cycle PCR is completed in <10 min, <5 min, <2.5 min, <1 min, <30 seconds, and <15 seconds, respectively. As PCR speeds become increasingly faster, the primer or polymerase concentrations, or both, are increased, thereby retaining PCR efficiency and yield.

Compromising any of the 3 component reactions of PCR (primer annealing, polymerase extension, and template denaturation) can limit the efficiency and yield of PCR. For example, if primers anneal to only 95% of the template, the PCR efficiency cannot be greater than 95%, even if 100% of the templates are denatured and 100% of the primed templates are extended to full length products. Similarly, if extension is only 95% efficient, the maximum possible PCR efficiency is only 95%. In order for the PCR product concentration to double each cycle, all the components must reach 100% completion. Denaturation, annealing and extension will be considered sequentially in the following paragraphs.

Inadequate denaturation is a common reason for PCR failure, in slow (>60 second cycles), rapid (20-60 second cycles), and extreme (<20 second cycles) PCR temperature cycling. The goal is complete denaturation each cycle, providing quantitative template availability for primer annealing. Initial denaturation of template before PCR, particularly genomic DNA, usually requires more severe conditions than denaturation of the amplification product during PCR. The original optimization of rapid cycle PCR (4) was performed after boiling the template, a good way to assure initial denaturation of genomic DNA. Incomplete initial denaturation can occur with high Tm targets, particularly those with flanking regions of high stability (37). This can compromise quantitative PCR, illustratively for genomic insertions or deletions, particularly if minor temperature differences during denaturation affect PCR efficiency (37-39). If prior boiling or restriction digestion (37) is not desired, and higher denaturation temperatures compromise the polymerase, adjuvants that lower product Tm can be used to help with denaturation.

Although 94°C is often used as a default target temperature for denaturation, it is seldom optimal. PCR products melt over a 40°C range, depending primarily on GC content and length (43). Low denaturation target temperatures have both a speed and specificity advantage when the PCR product melts low enough that a lower denaturation temperature can be used. The lower the denaturation temperature, the faster the sample can reach the denaturation temperature, and the faster PCR can be performed. Added specificity arises from eliminating all potential products with higher denaturation temperatures, as these potential products will remain double-stranded and will not be available for primer annealing. To amplify high Tm products, the target temperature may need to be increased above 94°C. However, most current heat stable polymerases start to denature above 97°C and the PCR solution may boil between 95°C (or lower) and 100°C, depending on the altitude, so there is not much room to increase the temperature. Lowering the monovalent salt and $Mg^{++}$ concentration lowers product Tm. Similarly, incorporating dUTP and/or 7-deaza-dGTP also lowers product Tm, but may decrease polymerase extension rates. Most proprietary PCR "enhancers" are simple organics that lower product Tm, enabling denaturation (and amplification) of high Tm products. Most popular among these are DMSO, betaine, glycerol, ethylene glycol, and formamide. In addition to lowering Tm, some of these additives also raise the boiling point of the PCR mixture (particularly useful at high altitudes). As the concentration of enhancer increases, product Tms decrease, but polymerase inhibition may increase.

Denaturation, however, need not be rate limiting even under extreme cycling conditions, because DNA unwinding is first order and very fast (10-100 msec), even when the temperature is only slightly above the product Tm. Denaturation occurs so rapidly at 2-3°C above the Tm of the amplification product that it is difficult to measure, but complete denaturation of the amplicon probably occurs in less than 0.1 second. If the product melts in multiple domains, the target denaturation temperature should be 2-3°C above the highest melting domain. As long as the sample reaches this temperature, denaturation is very fast, even for long products. Using capillaries and water baths (40), complete denaturation of PCR products over 20 kB occured in less than one second (52). Product Tms and melting domains are illustratively determined experimentally with DNA dyes and high resolution melting (41). Although Tm estimates can be obtained by software predictions (42), their accuracy is limited. Furthermore, observed Tms strongly depend on local reaction conditions, such as salt concentrations and the presence of any dyes and adjuvants. Thus, observed Tms are usually better matched to the reaction conditions.

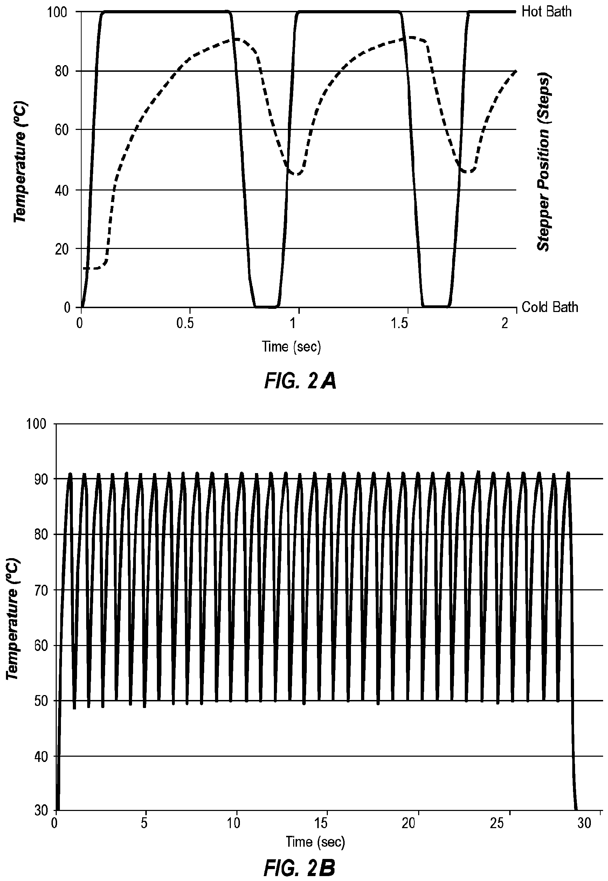

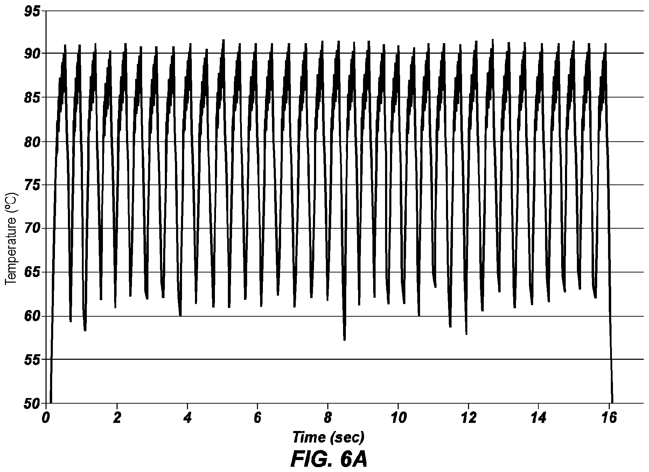

Without any effect on efficiency, the approach rate to denaturation can be as fast as possible, for example 200-400°C/s, as shown in Fig. 2A and Fig. 6A. At these rates, only about 0.1-0.2 seconds are required to reach denaturation temperatures. However, a slower rate as the target temperature is approached decreases the risk of surpassing the target temperature and avoids possible polymerase inactivation or boiling of the solution. One illustrative method to achieve a slower approach temperature is to submerge the sample in a hot bath that exceeds the target temperature by 5-10°C. The temperature difference between the target and bath temperatures determines the exponential approach curve that automatically slows as the difference decreases. By continuously monitoring the temperature, the next phase (cooling toward annealing) is triggered when the denaturation target is achieved. In summary, complete product denaturation in PCR requires <0.2 s at temperatures 2-3°C above the highest melting domain temperature of the product and the denaturation temperature can be approached as rapidly as possible, illustratively at 40-400°C/second. Since denaturation is first order, its rate depends only on the product concentration, and the efficiency (or percentage of the product that is denatured) is independent of the product concentration.

Incomplete and/or misdirected primer annealing can result in poor PCR. Low efficiency results if not all template sites are primed. Furthermore, if priming occurs at undesired sites, alternative products may be produced. The goal is essentially complete primer annealing to only the desired sites each cycle, providing quantitative primed template for polymerase extension.

Rapid PCR protocols with 20-60 second cycles suggest an annealing time of <1 second at 5°C below the Tm with 500 nM primers (52). Primer concentrations for instruments attempting <20 second cycles range from 200-1,000 nM each (Table 1). These concentrations are similar to those used in conventional PCR (>60 second cycles), where long annealing times are used. Lowering the primer concentration is often used to improve specificity, and increasing the primer concentration is seldom considered due to concerns regarding nonspecific amplification. However, with rapid cycling, improved specificity has been attributed to shorter annealing times (5). If this trend is continued, one would expect that very short annealing times of extreme PCR should tolerate high primer concentrations. To promote annealing, an annealing temperature 5°C below the primer Tm is recommended for 20-60 second cycles. Tms are best measured experimentally by melting analysis using saturating DNA dyes and oligonucleotides under the same buffer conditions used for amplification. The primer is combined with its complementary target with a 5'-extension as a dangling end, to best approximate the stability of a primer annealed to its template, and melting analysis is performed.

In contrast to denaturation, annealing efficiency depends on the primer concentration. Primer annealing can become limiting at very fast cycle speeds. Primer annealing is a second order reaction dependent on both primer and target concentrations. However, during most of PCR, the primer concentration is much higher than the target concentration and annealing is effectively pseudo-first order and dependent only on the primer concentration. In this case, the fraction of product that is primed (the annealing efficiency) depends only on the primer concentration, not the product concentration, so that higher primer concentrations should allow for shorter annealing times. Furthermore, without being bound to theory, it is believed that the relationship is linear. As the annealing time becomes shorter and shorter, increased primer concentrations become necessary to maintain the efficiency and yield of PCR. For example, rapid cycling allows about 1-3 seconds for annealing at temperatures 5°C below primer Tm (3). If this annealing time (at or below Tm-5°C) is reduced 10-fold in extreme PCR, a similar priming efficiency would be expected if the primer concentration were increased 10-fold. As the available annealing time becomes increasingly shorter, the primer concentration should be made increasingly higher by approximately the same multiple. Typical rapid PCR protocols use 500 nM each primer. If the annealing time in extreme PCR is reduced 3 to 40-fold, the primer concentrations required to obtain the same priming efficiency are 1,500 - 20,000 nM each primer. This is equivalent to 3,000 - 40,000 nM total primers, higher than any primer concentration in Table 1. This suggests that one reason for poor efficiency in prior attempts at <20 second cycling is poor annealing efficiency secondary to inadequate primer concentrations. In extreme PCR, the primer concentrations are increased to 1.5-20 $\mu$M each to obtain excellent annealing efficiency despite annealing times of 0.05 - 0.3 seconds. Ever greater primer concentrations can be contemplated for ever shorter annealing times, using increased primer concentrations to offset decreased annealing times to obtain the same annealing efficiency. It is noted that most commercial instruments require a hold time of at least 1 second, while a few instruments allow a hold time of "0" seconds, but no commercial instrument allows a hold time of a fractional second. For some illustrative examples of extreme PCR, hold times in increments of 0.1 or 0.01 seconds may be desirable.

Another way to increase the annealing rate and shorten annealing times without compromising efficiency is to increase the ionic strength, illustratively by increasing the $Mg^{++}$ concentration. Annealing rates are known in the art to increase with increasing ionic strength, and divalent cations are particularly effective for increasing rates of hybridization, including primer annealing.

Illustratively, the approach rate to the annealing target temperature may be as fast as possible. For example, at 200-800°C/s (Figs. 2a and 6a), annealing temperatures can be reached in 0.05-0.2 seconds. Rapid cooling also minimizes full length product rehybridization. To the extent that duplex amplification product forms during cooling, PCR efficiency is reduced because primers cannot anneal to the duplex product. Although this is rare early in PCR, as the product concentration increases, more and more duplex forms during cooling. Continuous monitoring with SYBR® Green I suggests that such product reannealing can be a major cause of the PCR plateau (44).

Polymerase extension also requires time and can limit PCR efficiency when extension times are short. Longer products are known to require longer extension times during PCR and a final extension of several minutes is often appended at the end of PCR, presumably to complete extension of all products. The usual approach for long products is to lengthen the time for extension. Using lower extension temperatures further increases required times, as in some cases of 2-step cycling where primer annealing and polymerase extension are performed at the same temperature.

Essentially complete extension of the primed template each cycle is required for optimal PCR efficiency. Most polymerase extension rates increase with temperature, up to a certain maximum. For Taq polymerase, the maximum is about 100 nucleotides/s at 75-80°C and it decreases about 4-fold for each 10°C that the temperature is reduced (50). For a 536 bp beta-globin product, 76°C was found optimal in rapid cycle PCR (4). Faster polymerases have recently been introduced with commercial claims that they can reduce overall PCR times, suggesting that they may be able to eliminate or shorten extension holding times for longer products.

As an alternative or complement to faster polymerase extension rates, it has been found that increasing the concentration of polymerase reduces the required extension time. Given a standard Taq polymerase concentration in PCR (0.04 U/μl) or 1.5 nM (49) with 500 nM of each primer, if each primer is attached to a template, there is only enough polymerase to extend 0.15% of the templates at a time, requiring recycling of the polymerase over and over again to new primed templates in order to extend them all. By increasing the concentration of polymerase, more of the available primed templates are extended simultaneously, decreasing the time required to extend all the templates, presumably not by faster extension rates, but by extending a greater proportion of the primed templates at any given time.

To a first approximation, for small PCR products (<100 bp), the required polymerization time appears to be directly proportional to the polymerization rate of the enzyme (itself a function of temperature) and the polymerase concentration. The required time is also inversely proportional to the length of the template to be extended (product length minus the primer length). By increasing the polymerase activity 20-300 fold over the standard activity of 0.04 U/μl in the PCR, extreme PCR with <20 second cycles can result in high yields of specific products. That is, activities of 0.8 - 12 U/μl (1-16 μM of KlenTaq) enable two-step extreme PCR with combined annealing/extension times of 0.1-1.0 second. The highest polymerase activity used previously was 0.5 U/μl (Table 1). For two-step PCR that is used in illustrative examples of extreme PCR, a combined annealing/extension step at 70-75°C is advantageous for faster polymerization rates. Furthermore, because it simplifies temperature cycling, two-step PCR is typically used in illustrative examples of extreme cycling (<20 second cycles) and both rapid annealing and rapid extension must occur during the combined annealing/extension step. Therefore, both increased primer concentrations and increased polymerase concentrations are used in illustrative examples, resulting in robust PCR under extreme two-temperature cycling. Illustratively, primer concentrations of 1.5-20 μM each and polymerase concentrations of 0.4 - 12 U/μl of any standard polymerase (0.5-16 μM of KlenTaq) are necessary with combined annealing/extension times of 0.05 - 5.0 seconds at 50-75°C, as illustrated in the Examples to follow. Because there is only one PCR cycling segment for both annealing and extension, extreme PCR conditions require enhancement of both processes, illustratively by increasing the concentrations of both the primers and the polymerase.

Extreme three-temperature cycling is also envisioned, where the annealing and extension steps are kept separate at different temperatures. In this case, the time allotted to annealing and extension steps can be individually controlled and tailored to specific needs. For example, if only the annealing time is short (0.05 - 0.2 seconds) and the extension time is kept comparatively long (illustratively for 1, 2, 5, 10 or 15 seconds), only the primer concentrations need to be increased for efficient PCR. Alternatively, if the extension time is short (< 1 sec within 70-80°C), but the annealing time is long, it is believed that only the polymerase concentration needs to be increased to obtain efficient PCR. It is understood that efficient PCR has an illustrative efficiency of at least 70%, more illustratively of at least 80%, and most illustratively of at least 90%, with >95% efficiency achievable in many instances.

For products longer than 100 bp, efficient extension using extreme PCR may need a combination of high polymerase concentration and increased extension time. If the polymerase is in excess, the minimum time illustratively should be the extension length (defined as the product length minus the primer length) in bases divided by the polymerase extension rate in bases/second. However, as previously noted, the polymerase is usually only saturating in the beginning of PCR, before the concentration of template increases to greater than the concentration of polymerase. One way to decrease cycle time is to use two-temperature PCR near the temperature maximum of the polymerase, typically 70-80°C. The required extension time can be determined experimentally using real-time PCR and monitoring the quantification cycle or Cq. For example, at a polymerase extension rate of 100 bases/second at 75°C, a 200 bp product would be expected to require about 2 seconds if the concentration of polymerase is in excess. Similarly, a 400 bp product would be expected to require about 4 seconds using this same polymerase as long as its concentration is greater than the template being extended. If the polymerase is not in excess, adding more polymerase allows more templates to be extended at the same time, decreasing the required extension time in proportion to the concentration of polymerase.

The utility of any DNA analysis method depends on how fast it can be performed, how much information is obtained, and how difficult it is to do. Compared to conventional cloning techniques, PCR is fast and simple. Rapid cycle and extreme PCR focus on continued reduction of the time required. Real-time PCR increases the information content by acquiring data each cycle. Melting analysis can be performed during or after PCR to monitor DNA hybridization continuously as the temperature is increased.

Returning to the equilibrium and kinetic paradigms of PCR (Fig. 15A-15B), extreme PCR of products <100 bps exemplifies a good application of the kinetic model. Temperatures are always changing and rates of denaturation, annealing, and extension depend on temperature, so an adequate assessment of PCR can only be obtained by integrating the rates of the component reactions across temperature. For products greater than 100 bp, longer extension times may be necessary, and components of both the kinetic and equilibrium models are appropriate.

When the reaction conditions are configured according to at least one embodiment herein, it has been found that PCR can be performed at very fast rates, illustratively with some embodiments in less than one minute for complete amplification, with cycle times of less than two seconds. Illustratively, various combinations of increased polymerase and primer concentrations are used for this extreme PCR. Without being bound to any particular theory, it is believed that an excess concentration of primers will allow for generally complete primer annealing, thereby increasing PCR efficiency. Also without being bound to any particular theory, it is believed that an increase in polymerase concentration improves PCR efficiency by allowing more complete extension. Increased polymerase concentration favors binding to the annealed primer, and also favors rebinding if a polymerase falls off prior to complete extension. The examples below show that extreme PCR has been successful, even when starting with complex eukaryotic genomic DNA and single-copy targets.

Although KlenTaq was used in the Examples to follow, it is believed that any thermostable polymerase of similar activity will perform in a similar manner in extreme PCR, with allowances for polymerase extension rates. For example, Herculase, Kapa2G FAST, KOD Phusion, natural or cloned *Thermus aquaticus* polymerase, Platinum Taq, GoTaq and Fast Start are commercial preparation of polymerases that should enable extreme PCR when used at the increased concentrations presented here, illustratively adjusted for differences in enzyme activity rates.

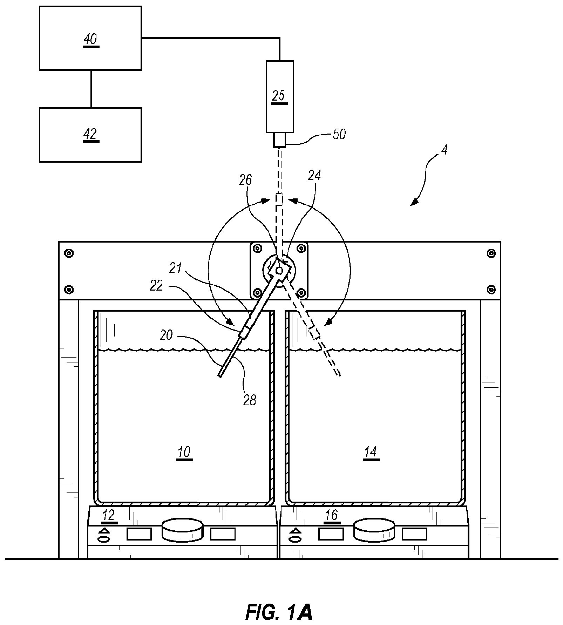

Because no current commercial PCR instrument allows for two second cycle times, a system 4 was set up to test proof of concept for extreme PCR. However, it is understood that the system 4 is illustrative and other systems that can thermocycle rapidly are within the scope of this disclosure. As shown in Fig. 1A, a hot water bath 10 of 95.5°C (the temperature of boiling water in Salt Lake City, UT, the location where the present examples were performed), and a cool water bath 14 of 30-60°C are used to change the temperature of 1-5 μl samples contained in a sample container 20. The illustrative water baths 10, 14 are 4.5 quart stainless steel dressing jars (Lab Safety Supply, #41634), although 500 ml glass beakers were used in some examples, and are heated on electric hotplates 12, 16 with magnetic stirring (Fisher Scientific Isotemp Digital Hotplates (#11-300-49SHP). However, it is understood that other embodiments may be used to heat and cool the samples. In the embodiment shown in Fig. 1A, the sample container 20 is a composite glass/plastic reaction tube (BioFire Defense #1720, 0.8 mm ID and 1.0 mm OD). However, in other examples, hypodermic needles (Becton Dickenson #305187, 0.042" ID, 0.075" OD) and composite stainless steel/plastic reaction tubes constructed from stainless steel tubing (Small Parts, 0.042" ID/0.075" OD, 0.035" ID/0.042" OD, or 0.0265" ID/0.035" OD) and fit into the plastic tops of the BioFire tubes were used as the sample container 20. While other sample containers are within the scope of this invention, it is desirable that the sample containers have a large surface area to volume ratio and have a fast heat transfer rate. For certain embodiments, the open end of the metal tubing was sealed by heating to a red-white color using a gas flame and compressing in a vise. For real-time PCR, tubes that are optically clear or have an optically clear portion are desirable. Samples were spun down to the bottom of each tube by brief centrifugation.

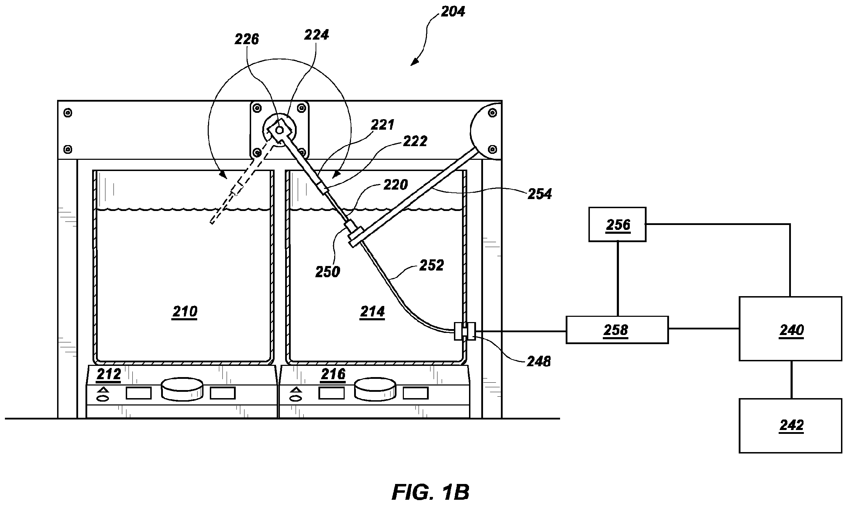

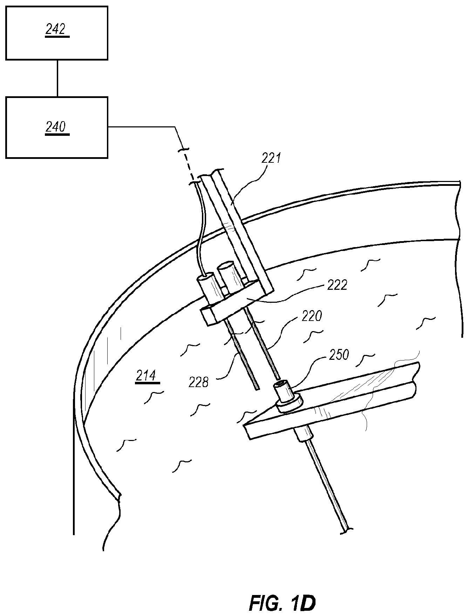

The sample container 20 is held by a tube holder 22 attached to a stepper motor shaft 26 by arm 21. The tube holder 22 was machined from black Delrin plastic to hold 2-5 sample containers 20 (only one sample container 20 is visible in Fig. 1A, but a row of such sample containers 20 may be present) so that the reaction solutions were held at a radius of 6.5 - 7.5 cm. While not visible in Fig. 1A, a thermocouple (Omega type T precision fine wire thermocouple #5SRTC-TT-T-40-36, 36" lead, 0.003' diameter with Teflon insulation) may be used to measure temperature. With reference to Fig. 1D, which shows a similar tube holder and arm of Fig. 1B with like numbers representing similar components, a tube holder 222 designed to hold two sample containers is present, with one location in tube holder 222 occupied by a thermocouple 228. It is understood that any number of sample containers 20 or 220 may be used in any of the embodiments described herein, with or without a thermocouple, as shown in Fig. 1D. Thermocouple amplification and linearization is performed with an Analog Devices AD595 chip (not shown). The thermocouple voltage was first calculated from the AD595 output as Type T voltage = (AD595 output/247.3) - 11 $\mu$V. Then, the thermocouple voltage was converted to temperature using National Institute of Standards and Technology coefficients for the voltage/temperature correlation of Type T thermocouples. The analog signal was digitized (PCIe-6363 acquisition board) and processed by LabView software (version 2010, National Instruments) installed on CPU 40 and viewed on user interface 42. Stepper motion illustratively is triggered dynamically at 87-92°C and 60-75°C or may be held in each water bath for a computer-controlled period of time. Thirty to fifty cycles are typically performed.

The stepper motor 24 (Applied Motion Products, #HT23-401, 3V,3A) is positioned between the water baths 10 and 14 so that all sample containers 20 in the tube holder 22 could flip between each water bath 10 and 14, so that the portion of each sample container 20 containing samples are completely submerged. The stepper motor 24 is powered illustratively by a 4SX-411 nuDrive (National Instruments, not shown) and controlled with a PCI-7344 motion controller and NI-Motion Software (version 8.2, National Instruments) installed on CPU 40. Stepper motor 24 rotates between water baths 10 and 14 in about 0.1 second. Fig. 2A shows a sample temperature trace (-----) juxtaposed over a trace of the position of the sample container 20 (-), for a run where stepper motion was triggered at 90°C and 50°C. As can be seen in Fig. 2A, there is some overshoot to a temperature lower than 50°C, presumably due to the time required to move the sample container 20 out of water bath 14. Thus, as discussed above, it may be desirable to trigger stepper motor 24 at a somewhat higher temperature. In the examples below, the temperatures given are for the sample temperature reached, not the trigger temperature. The maximum heating rate calculated from Fig. 2A is 385°C/s and maximum cooling rate 333°C/s. Illustratively, extreme PCR may be performed with ramp rates of at least 200°C/s. In other embodiments, the ramp rate may be 300°C/s or greater.

In some examples, system 4 is also configured for real-time monitoring. As shown in Fig. 1A, for real time monitoring, a fiber optics tip 50 of optics block 25 is mounted above sample container 20, such that when sample container 20 is being moved from hot water bath 10 to the cold water bath by stepper motor 24, sample container 20 passes by the fiber optics tip 50, with or without a hold in this monitoring position. In this illustrative embodiment, fiber optics tip is provided in air above the water baths. Thermocycling device 4 may be controlled by CPU 40 and viewed on user interface 42

Fig. 1B shows an embodiment similar to Fig. 1A. Hot plates 212 and 216 are provided for controlling temperature of hot water bath 210 and cold water bath 214. A stepper motor 224 is provided for moving sample container 220 and thermocouple 228 (shown in Fig. 1D), by moving arm 221 and tube holder 222, which is illustratively made of aluminum. However, in this embodiment, the tip 250 of the fiber optics cable 252 is held in water bath 214 by positioning block 254. Fiber optics cable 252 enters water bath 214 through port 248 and provides signal to optics block 225. Thermocycling device 204 may be controlled by CPU 240 and viewed on user interface 242.

Light from an Ocean Optics LLS-455 LED Light Source 256 was guided by fiber optics cable 252 (Ocean Optics P600-2-UV-VIS, 600 $\mu$m fiber core diameter) into a Hamamatsu Optics Block 258 with a 440 +/-20 nm excitation interference filter, a beamsplitting 458 nm dichroic and a 490 +/- 5 nm emission filter (all from Semrock, not shown). Epifluorescent illumination of the capillary was achieved with another fiber optic cable (not shown) placed approximately 1-2 mm distant from and in-line with the one sample capillary when positioned in the cooler water bath. Emission detection was with a Hamamatsu PMT 62.

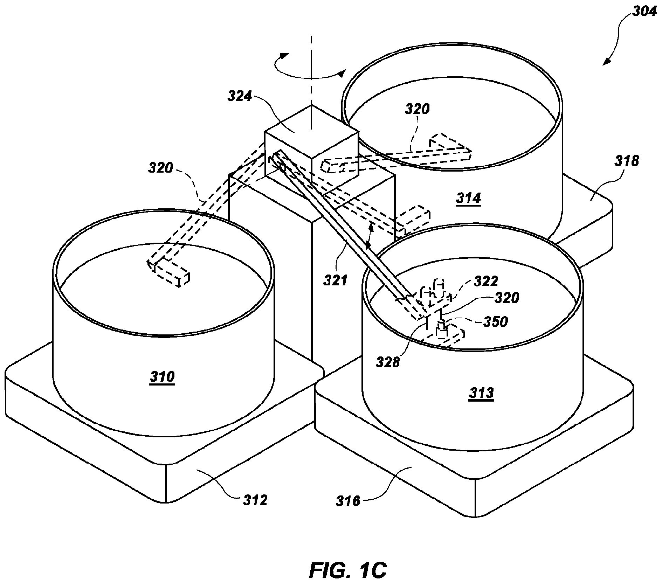

Fig. 1C shows an illustrative system 304 for three-temperature PCR. A hot water bath 310 of 95.5°C, a cool water bath 314 of 30-60°C, and a medium water bath 313 of 70-80°C are used to change the temperature of 1-5 $\mu$l samples contained in a sample container 320, and are heated on three electric hotplates 312, 316, and 318 with magnetic stirring. The sample container 320 is held by a tube holder 322 attached to a stepper motor 324 by arm 321. Thermocouple 328 is also held by tube holder 322. Arm 321 may be raised as stepper motor 324 rotates. A fiber optics tip 350 is illustratively provided in medium water bath 313, although it is understood that it may be placed in air, as with Fig. 1A. Due to the set-up of this illustrative embodiment, it was not possible to place the three water baths, 310, 313, and 314 equidistant from one another. Accordingly, the largest space was placed between hot water bath 310 and cool water bath 314, as cooling of the sample between these baths is desirable, whereas the sample moves between the other water baths to be heated. Because two stepper motors are used simultaneously (one to raise the capillary out of the water and one to transfer between water baths) the angular motion of each can be minimized to decrease the time of movement between baths. In the 2 water bath system, the required angular motion of the stepper to transfer the sample between baths is greater than 270 degrees. However, in the 3 water bath system, the stepper motor that raises the samples needs to traverse less than 45 degrees while the stepper moving the samples between water baths needs to move only 90 degrees or less. The water baths can also be configured as sectors of a circle (pie-shaped wedges) to further limit the angular movement required. Minimizing the angular movement decreases the transfer time between water baths. Transfer times less than 100 msec or even less than 50 msec are envisioned. Other components of this system 304 are similar to the systems 4, 204 shown in Figs. 1a-b and are not shown in Fig. 1C. Extension to a 4 water bath system is also envisioned. Uses for the fourth water bath include an ice water bath to ensure a cold start to limit the amount of extension before initial PCR denaturation, and a water bath at 37-56°C for reverse transcription prior to PCR (RT-PCR). If both a cold start and a reverse transcription were needed, a 5 water bath system could be used.

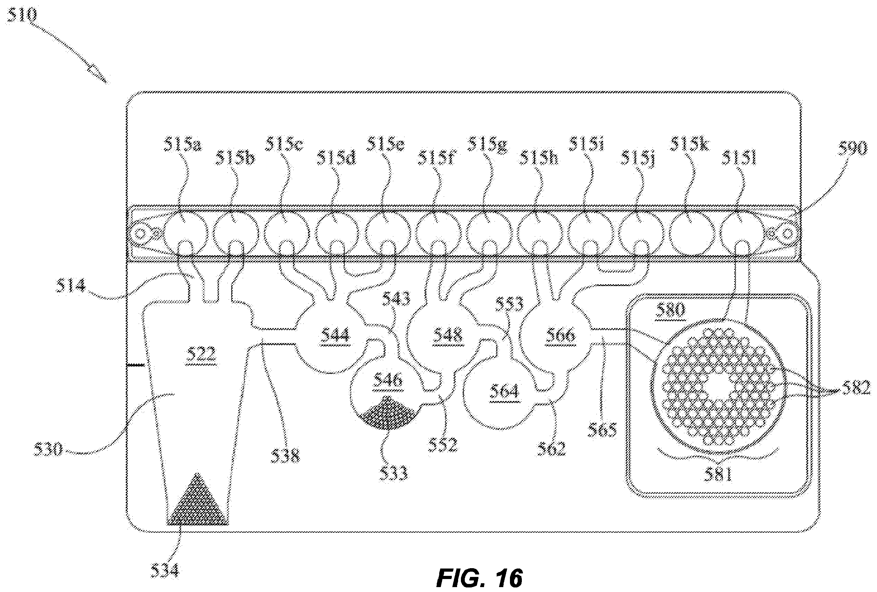

Fig. 16 shows an illustrative pouch 510 that may be used in various examples below, or may be reconfigured for various embodiments. Pouch 510 is similar to Fig. 15 of U.S. Patent No. 8,895,295, with like items numbered the same. Fitment 590 is provided with entry channels 515a through 515l, which also serve as reagent reservoirs or waste reservoirs. Illustratively, reagents may be freeze dried in fitment 590 and rehydrated prior to use. Blisters 522, 544, 546, 548, 564, and 566, with their respective channels 514, 538, 543, 552, 553, 562, and 565 are similar to blisters of the same number of Fig. 15 of U.S. Patent No. 8,895,295. Second-stage reaction zone 580 of Fig. 16 is similar to that of U.S. Patent Application No. 8,895,295, but the second-stage wells 582 of high density array 581 are arranged in a somewhat different pattern. The more circular pattern of high density array 581 of Fig. 16 eliminates wells in corners and may result in more uniform filling of second-stage wells 582. As shown, the high density array 581 is provided with 102 second-stage wells 582. Pouch 510 is suitable for use in the FilmArray® instrument (BioFire Diagnostics, LLC, Salt Lake City, UT). However, it is understood that the pouch embodiment is illustrative only.

While other containers may be used, illustratively, pouch 510 is formed of two layers of a flexible plastic film or other flexible material such as polyester, polyethylene terephthalate (PET), polycarbonate, polypropylene, polymethylmethacrylate, and mixtures thereof that can be made by any process known in the art, including extrusion, plasma deposition, and lamination. Metal foils or plastics with aluminum lamination also may be used. Other barrier materials are known in the art that can be sealed together to form the blisters and channels. If plastic film is used, the layers may be bonded together, illustratively by heat sealing. Illustratively, the material has low nucleic acid binding capacity.

For embodiments employing fluorescent monitoring, plastic films that are adequately low in absorbance and auto-fluorescence at the operative wavelengths are preferred. Such material could be identified by testing different plastics, different plasticizers, and composite ratios, as well as different thicknesses of the film. For plastics with aluminum or other foil lamination, the portion of the pouch that is to be read by a fluorescence detection device can be left without the foil. For example, if fluorescence is monitored in second-stage wells 582 of the second-stage reaction zone 580 of pouch 510, then one or both layers at wells 582 would be left without the foil. In the example of PCR, film laminates composed of polyester (Mylar, Dupont, Wilmington DE) of about 0.0048 inch (0.1219 mm) thick and polypropylene films of 0.001-0.003 inch (0.025-0.076 mm) thick perform well. Illustratively, pouch 510 is made of a clear material capable of transmitting approximately 80%-90% of incident light.

In the illustrative embodiment, the materials are moved between blisters by the application of pressure, illustratively pneumatic pressure, upon the blisters and channels. Accordingly, in embodiments employing pressure, the pouch material illustratively is flexible enough to allow the pressure to have the desired effect. The term "flexible" is herein used to describe a physical characteristic of the material of pouch. The term "flexible" is herein defined as readily deformable by the levels of pressure used herein without cracking, breaking, crazing, or the like. For example, thin plastic sheets, such as Saran™ wrap and Ziploc® bags, as well as thin metal foil, such as aluminum foil, are flexible. However, only certain regions of the blisters and channels need be flexible, even in embodiments employing pneumatic pressure. Further, only one side of the blisters and channels need to be flexible, as long as the blisters and channels are readily deformable. Other regions of the pouch 510 may be made of a rigid material or may be reinforced with a rigid material.

Illustratively, a plastic film is used for pouch 510. A sheet of metal, illustratively aluminum, or other suitable material, may be milled or otherwise cut, to create a die having a pattern of raised surfaces. When fitted into a pneumatic press (illustratively A-5302-PDS, Janesville Tool Inc., Milton WI), illustratively regulated at an operating temperature of 195°C, the pneumatic press works like a printing press, melting the sealing surfaces of plastic film only where the die contacts the film. Various components, such as PCR primers (illustratively spotted onto the film and dried), antigen binding substrates, magnetic beads, and zirconium silicate beads may be sealed inside various blisters as the pouch 510 is formed. Reagents for sample processing can be spotted onto the film prior to sealing, either collectively or separately. In one embodiment, nucleotide tri-phosphates (NTPs) are spotted onto the film separately from polymerase and primers, essentially eliminating activity of the polymerase until the reaction is hydrated by an aqueous sample. If the aqueous sample has been heated prior to hydration, this creates the conditions for a true hot-start PCR and reduces or eliminates the need for expensive chemical hot-start components.

Pouch 510 may be used in a manner similar to that described in U.S. Patent No. 8,895,295. In one illustrative embodiment, a 300 μl mixture comprising the sample to be tested (100 μl) and lysis buffer (200 μl) is injected into an injection port (not shown) in fitment 590 near entry channel 515a, and the sample mixture is drawn into entry channel 515a. Water is also injected into a second injection port (not shown) of the fitment 590 adjacent entry channel 515l, and is distributed via a channel (not shown) provided in fitment 590, thereby hydrating up to eleven different reagents, each of which were previously provided in dry form in each of entry channels 515b through 515l. These reagents illustratively may include freeze-dried PCR reagents, DNA extraction reagents, wash solutions, immunoassay reagents, or other chemical entities. Illustratively, the reagents are for nucleic acid extraction, first-stage multiplex PCR, dilution of the multiplex reaction, and preparation of second-stage PCR reagents, as well as control reactions. In the embodiment shown in Fig. 16, all that need be injected is the sample solution in one injection port and water in the other injection port. After injection, the two injection ports may be sealed. For more information on various configurations of pouch 510 and fitment 590, see U.S. Patent No. 8,895,295.

After injection, the sample is moved from injection channel 515a to lysis blister 522 via channel 514. Lysis blister 522 is provided with beads or particles 534, such as ceramic beads, and is configured for vortexing via impaction using rotating blades or paddles provided within the FilmArray® instrument. Bead-milling, by shaking or vortexing the sample in the presence of lysing particles such as zirconium silicate (ZS) beads 534, is an effective method to form a lysate. It is understood that, as used herein, terms such as "lyse," "lysing," and "lysate" are not limited to rupturing cells, but that such terms include disruption of non-cellular particles, such as viruses. It is understood that a variety of devices may be used for milling, shaking, or vortexing the sample in lysis blister 522.

Once the cells have been adequately lysed, the sample is moved through channel 538, blister 544, and channel 543, to blister 546, where the sample is mixed with a nucleic acid-binding substance, such as silica-coated magnetic beads 533. The mixture is allowed to incubate for an appropriate length of time, illustratively approximately 10 seconds to 10 minutes. A retractable magnet located within the instrument adjacent blister 546 captures the magnetic beads 533 from the solution, forming a pellet against the interior surface of blister 546. The liquid is then moved out of blister 546 and back through blister 544 and into blister 522, which is now used as a waste receptacle. One or more wash buffers from one or more of injection channels 515c to 515e are provided via blister 544 and channel 543 to blister 546. Optionally, the magnet is retracted and the magnetic beads 533 are washed by moving the beads back and forth from blisters 544 and 546 via channel 543. Once the magnetic beads 533 are washed, the magnetic beads 533 are recaptured in blister 546 by activation of the magnet, and the wash solution is then moved to blister 522. This process may be repeated as necessary to wash the lysis buffer and sample debris from the nucleic acid-binding magnetic beads 533, illustratively including 3 or more washes, although one wash may be sufficient for some embodiments disclosed herein and any number of washes is within the scope of this disclosure.

After washing, elution buffer stored at injection channel 515f is moved to blister 548, and the magnet is retracted. The solution is cycled between blisters 546 and 548 via channel 552, breaking up the pellet of magnetic beads 533 in blister 546 and allowing the captured nucleic acids to dissociate from the beads and come into solution. The magnet is once again activated, capturing the magnetic beads 533 in blister 546, and the eluted nucleic acid solution is moved into blister 548.

First-stage PCR master mix from injection channel 515g is mixed with the nucleic acid sample in blister 548. Optionally, the mixture is mixed by forcing the mixture between 548 and 564 via channel 553. After several cycles of mixing, the solution is contained in blister 564, where a pellet of first-stage PCR primers is provided, at least one set of primers for each target, and first-stage multiplex PCR is performed. If RNA targets are present, an RT step may be performed prior to or simultaneously with the first-stage multiplex PCR. First-stage multiplex PCR temperature cycling in the FilmArray® instrument is illustratively performed for 15-20 cycles, although other levels of amplification may be desirable, depending on the requirements of the specific application. The first-stage PCR master mix may be any of various master mixes, as are known in the art. In one illustrative example, the first-stage PCR master mix may be any of the chemistries disclosed in US2015/0118715, for use with PCR protocols taking 20 seconds or less per cycle.

After first-stage PCR has proceeded for the desired number of cycles, the sample may be diluted, illustratively by forcing most of the sample back into blister 548, leaving only a small amount in blister 564, and adding second-stage PCR master mix from injection channel 515i. Alternatively, a dilution buffer from 515i may be moved to blister 566 then mixed with the amplified sample in blister 564 by moving the fluids back and forth between blisters 564 and 566. If desired, dilution may be repeated several times, using dilution buffer from injection channels 515j and 515k, or injection channel 515k may be reserved for sequencing or for other post-PCR analysis, and then adding second-stage PCR master mix from injection channel 515h to some or all of the diluted amplified sample. It is understood that the level of dilution may be adjusted by altering the number of dilution steps or by altering the percentage of the sample discarded prior to mixing with the dilution buffer or second-stage PCR master mix comprising components for amplification, illustratively a polymerase, dNTPs, and a suitable buffer, although other components may be suitable, particularly for non-PCR amplification methods. It is understood that dilution not only has the effect of diluting the target amplicons, but it also dilutes inhibitors and nonspecific amplification from first-stage amplification. If desired, this mixture of the sample and second-stage PCR master mix may be pre-heated in blister 564 prior to movement to second-stage wells 582 for second-stage amplification. Such preheating may obviate the need for a hot-start component (antibody, chemical, or otherwise) in the second-stage PCR mixture.

The illustrative second-stage PCR master mix is incomplete, lacking primer pairs, and each of the 102 second-stage wells 582 is pre-loaded with a specific PCR primer pair. If desired, second-stage PCR master mix may lack other reaction components, and these components may be pre-loaded in the second-stage wells 582 as well. Each primer pair may be similar to or identical to a first-stage PCR primer pair or may be nested within the first-stage primer pair. Movement of the sample from blister 564 to the second-stage wells 582 completes the PCR reaction mixture. Once high density array 581 is filled, the individual second-stage reactions are sealed in their respective second-stage blisters by any number of means, as is known in the art. Illustrative ways of filling and sealing the high density array 58l without cross-contamination are discussed in U.S. Patent No. 8,895,295. Illustratively, the various reactions in wells 582 of high density array 581 are simultaneously thermal cycled, illustratively with one or more Peltier devices, although other means for thermal cycling are known in the art.

In certain embodiments, second-stage PCR master mix contains the dsDNA binding dye LCGreen® Plus (BioFire Defense, LLC) to generate a signal indicative of amplification. However, it is understood that this dye is illustrative only, and that other signals may be used, including other dsDNA binding dyes and probes that are labeled fluorescently, radioactively, chemiluminescently, enzymatically, or the like, as are known in the art. Alternatively, wells 582 of array 581 may be provided without a signal, with results reported through subsequent processing.

Success of the secondary PCR reactions is dependent upon the template generated by the multiplex first-stage reaction. Typically, PCR is performed using DNA of high purity. Methods such as phenol extraction or commercial DNA extraction kits provide DNA of high purity. Samples processed through the pouch 510 may require accommodations be made to compensate for a less pure preparation. PCR may be inhibited by components of biological samples, which is a potential obstacle. Illustratively, hot-start PCR, higher concentration of Taq polymerase enzyme, adjustments in $MgCl_2$ concentration, adjustments in primer concentration, and addition of adjuvants (such as DMSO, TMSO, or glycerol) optionally may be used to compensate for lower nucleic acid purity. While purity issues are likely to be more of a concern with first-stage amplification, it is understood that similar adjustments may be provided in the second-stage amplification as well.

Instruments suitable for use with pouch 510 are described in U.S. Patent Nos. 8,394,608, 8,895,295, and U.S. Patent Application Nos. 62/298,311, 62/330,701, and 62/368,095. Further, it is understood that pouch 510 is illustrative, and other sample vessels or containers may be used herein.

EXAMPLE 1

Unless otherwise indicated, PCR was performed in 5 $\mu$l reaction volumes containing 50 mM Tris (pH 8.3, at 25°C), 3 mM $MgCl_2$, 200 $\mu$M each dNTP (dATP, dCTP, dGTP, dTTP), 500 $\mu$g/ml non-acetylated bovine serum albumin (Sigma), 2% (v/v) glycerol (Sigma), 50 ng of purified human genomic DNA, and 1X LCGreen® Plus (BioFire Diagnostics). The concentration of the primers and the polymerase varied according to the specific experimental protocols. Klentaq1™ DNA polymerase was obtained from either AB Peptides, St. Louis, MO, or from Wayne Barnes at Washington University (St. Louis). The molecular weight of KlenTaq is 62.1 kD with an extinction coefficient at 280 nm of 69,130 $M^{-1}cm^{-1}$, as calculated from the sequence (U.S. Patent No. 5,436,149). Mass spectrometry confirmed a predominate molecular weight of 62 kD, and denaturing polyacrylamide gels showed that the major band was greater than 80% pure by integration. Using the absorbance and purity to calculate the concentration indicated an 80 $\mu$M stock in 10% glycerol. Final polymerase concentrations were typically 0.25-16 $\mu$M. One $\mu$M KlenTaq is the equivalent of 0.75 U/$\mu$l, with a unit defined as 10 nmol of product synthesized in 30 min at 72°C with activated salmon sperm DNA. Primers were synthesized by the University of Utah core facility, desalted, and concentrations determined by $A_{260}$. The final concentrations of each primer typically varied from 2.5-20 $\mu$M.

A 45 bp fragment of *KCNE1* was amplified from human genomic DNA using primers CCCATTCAACGTCTA CATCGAGTC (SEQ ID NO:1) and TCCTTCTCTTGCCAGGCAT (SEQ ID NO:2). The primers bracketed the variant rs#1805128 (c.253G>A) and amplified the sequence: CCCATTCAACGTCTACATCGAGTCC(G/A)ATGCCTGGCAAG AGAAGGA (SEQ ID NO:3).

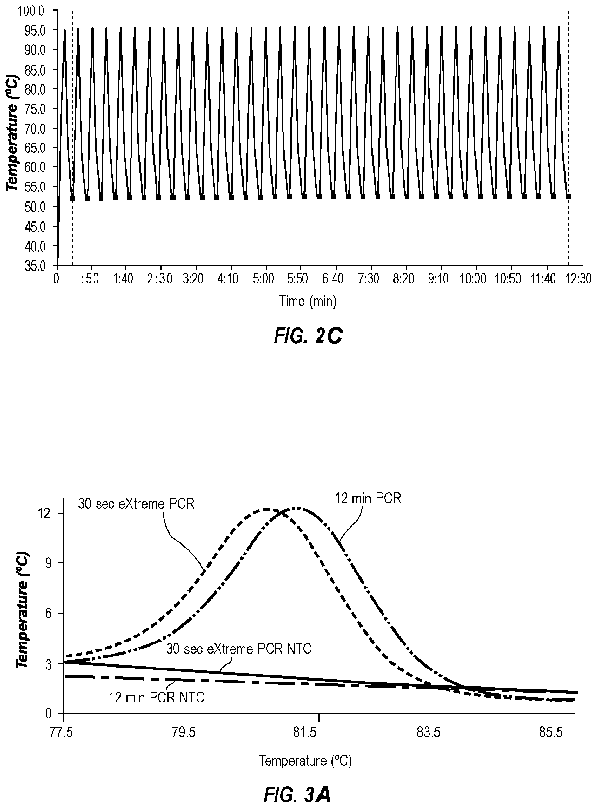

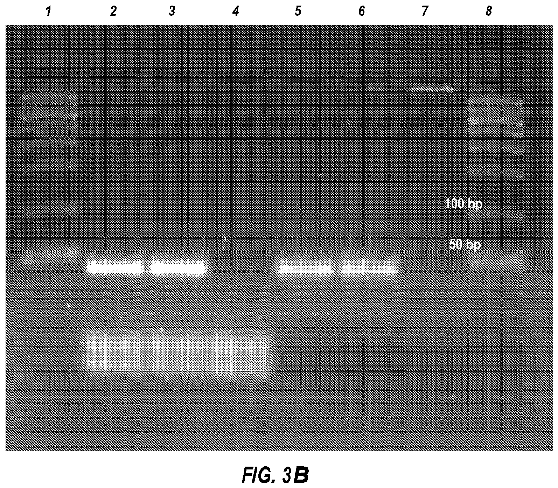

Fig. 3A shows a melting curve of the PCR product generated by extreme PCR using the device shown in Fig. 1A, where 0.64 $\mu$M KlenTaq and 10 $\mu$M of each primer were used, and cycled between 91°C and 50°C, as shown in Fig. 2B, for 35 cycles and a total amplification time of 28 seconds. Each cycle required 0.8 seconds. Also shown in Fig. 3A is a melting curve of the same amplicon generated by rapid cycling in the LightCycler, where 0.064 $\mu$M KlenTaq and 0.5 $\mu$M of each primer were used, and cycling was between 90°C and 50°C for 35 cycles and a total amplification time of 12 minutes (Fig. 2C). Each cycle required 10.3 seconds. Note that because of the different time scales in Fig. 2B and Fig. 2C, the entire extreme PCR protocol of Fig. 2B is completed in less than 2 cycles of its rapid cycle counterpart. Both reactions produced amplicons having similar Tms and strong bands on gel electrophoresis (Fig. 3B), whereas neither negative control showed amplification by either melting analysis or gel electrophoresis. In this illustrative example, extreme PCR conditions showed greater yield than rapid cycle PCR conditions when analyzed on gels (Fig. 3B). The 0.5°C difference in Tm on the melting curves is believed to be due to the different amounts of glycerol in each reaction, arising from the glycerol content in the polymerase storage buffer (final concentration of glycerol in the PCR was 1.3% under extreme conditions and 0.1% under rapid conditions). Fig. 3B also confirms that the size of the amplicons were similar and as predicted. In addition, despite the high concentrations of polymerase and primers, the reaction appears specific with no indication of nonspecific products. However, high resolution melting analysis was unable to distinguish the 3 genotypes. The stoichiometric percentage of polymerase to total primer concentration was 3% for extreme PCR and 6.4% for rapid cycle PCR.

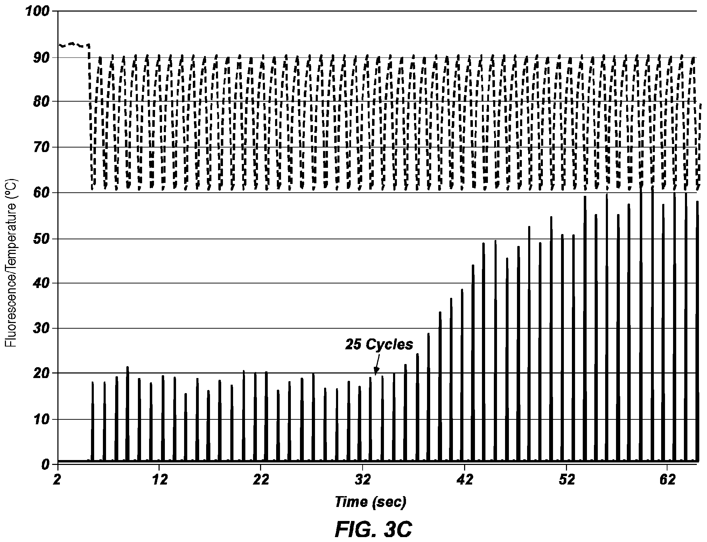

Real-time monitoring of the 45 bp *KCNE1* reaction was performed using 1 $\mu$M polymerase, 10 $\mu$M of each primer, and 1.3% glycerol. The sample was monitored each cycle in air between the 2 water baths using the device of Fig. 1A. The enclosed chamber air temperature was held at 70°C and the sample was interrogated for 0.2 seconds each cycle. As measured by the temperature reference capillary, samples were cycled between 60 and 90°C, as shown in Fig. 3C. The cycle time increased from 0.8 seconds to 1.12 seconds because of the added time for positioning and measuring. Thus, fifty cycles were completed in 56 seconds. Amplification was apparent from an increase in fluorescence at about 30 cycles or after about 34 seconds (Fig. 3C). The temperature remained near 60°C while the sample was in air for measurement, limiting the extension rate of the polymerase.

As seen in Fig. 3C, this reaction has a quantification cycle (Cq) of about 25 cycles, but it does not seem to plateau until at least 50 cycles. Also, because the reaction was stopped after 64 cycles, it is possible that the quantity of amplicon may continue to increase and not plateau until significantly later. Without being bound to theory, it is believed that the increase in primer concentration allows for improved yield and delayed plateau, illustratively 20 cycles after Cq, and more illustratively 25 cycles or more after Cq.

EXAMPLE 2

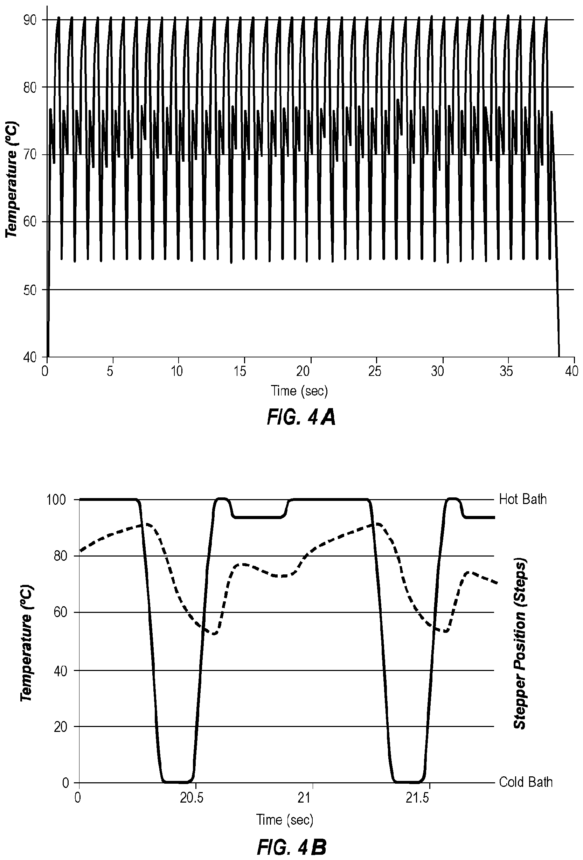

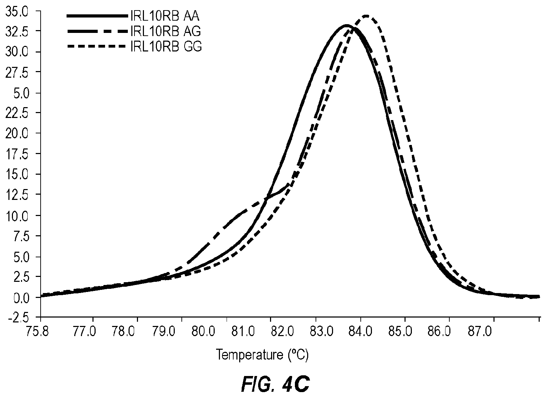

In this example, a 58 bp fragment bracketing an A>G variant (rs#2834167) in the interleukin 10 beta receptor was amplified with primers CTACAGTGGGAGTCACCTGC (SEQ ID NO:4) and GGTACTGAGCTGTGAAAGTCAGGTT (SEQ ID NO:5) to generate the following amplicon:
CTACAGTGGGAGTCACCTGCTTTTGCC(A/G)AAGGGAACCTGACTTTCACAGCTCAGT ACC (SEQ ID NO:6). Extreme PCR was performed as described in Example 1 using the instrument shown in Fig. 1A. One $\mu$M polymerase, 10 $\mu$M each primer and 1.3% glycerol were used (polymerase to total primer percentage = 5%). In order to increase the temperature for polymerase extension to 70-80°C, where the polymerase has higher extension rates, a different positioning protocol was used. After reaching the annealing temperature, instead of immediately positioning in air for monitoring, the sample was transferred to the hot water bath until the extension temperature was reached. Then the sample was positioned in air just above the hot water bath, producing the temperature cycles shown in Figs. 4a and 4b, and enabling faster polymerase extension at optimal temperatures between 70 and 77°C. The 3 different genotypes were each amplified by extreme PCR using 0.97 second cycles, completing 39 cycles in 38 seconds. After extreme PCR, high resolution melting curves were obtained for each genotype on an HR-1 instrument modified to accept LC24 capillaries. Fig. 4C reveals that all three genotypes were amplified and distinguished, as expected.

EXAMPLE 3

The reaction mixtures in Example 1 were the same for both the extreme PCR and rapid cycle PCR, except for the amounts of polymerase and primers, and a minor difference in glycerol concentration that apparently caused the shift in Tm seen in Fig. 3A. In this and all future examples, the glycerol concentration was held at 2% by equalizing its concentration as necessary. For extreme PCR, 1 $\mu$M polymerase and 10 $\mu$M of each primer were used, while for rapid cycle PCR, 0.064 $\mu$M polymerase and 0.5 $\mu$M of each primer were used. As discussed above, it is believed that faster annealing times provide for improved primer specificity. With this improved specificity, increased concentrations of primers may be used, which is believed to favor primer binding and allow reduced annealing times. Similarly, increased polymerase concentrations favor binding to the annealed primer, and also favor rebinding to the incomplete amplicon if a polymerase falls off prior to complete extension. In addition, because of the higher polymerase concentration, a greater proportion of the primed templates can be extended at once even late in PCR, reducing the number of templates that a single polymerase must extend and reducing the overall extension time.

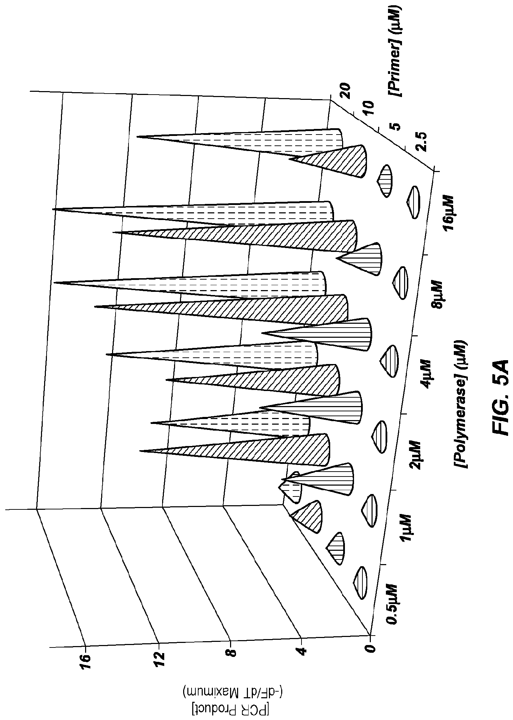

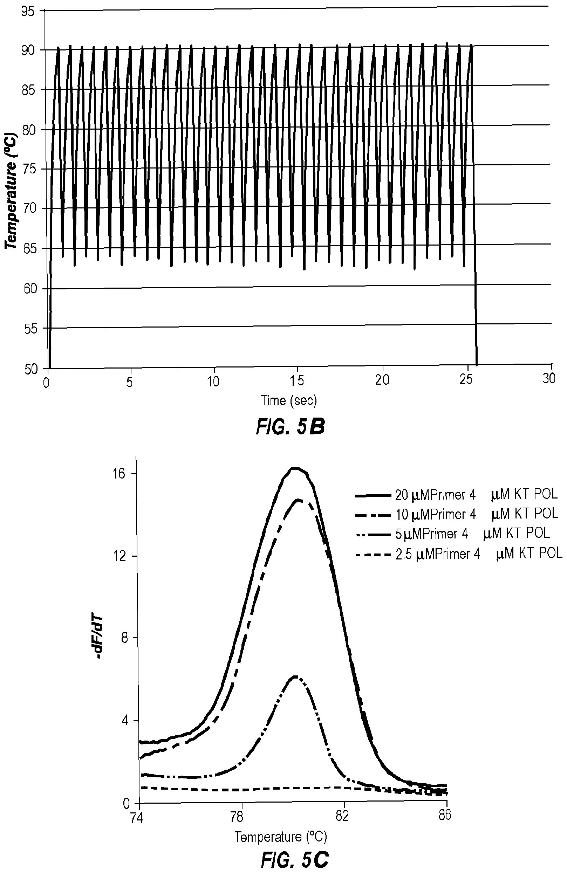

Fig. 5A summarizes the results of extreme PCR cycling with various polymerase and primer concentrations. In this example, a 49 bp fragment of the interleukin 10 beta receptor was amplified with primers GGGAGTCACCTGCTT TTGCC (SEQ ID NO:7) and TACTGAGCTGTGAAAGTCAGGTTCC (SEQ ID NO:8) and 3 mM MgCl$_2$, to generate: GGGAGTCACCTGCTTTTGCCAAAGGGAACCTGACTTTCACAGCTCAGTA (SEQ ID NO:9). For each extreme PCR reaction, the device shown in Figs. 1b was used without real time monitoring. The temperature was cycled between 90°C and 63°C for 35 cycles, for a total reaction time of just under 26 seconds (0.73 second cycles) as shown in Fig 5B. Reaction conditions were as discussed in Example 1, except that the amounts of polymerase and primers were varied, as shown in Fig. 5A. The vertical axis in Fig. 5A is quantified as the peak of the negative derivative plot of the melting curve, obtained without normalization on the HR-1 instrument. At 0.5 $\mu$M polymerase, virtually no amplification was seen at any level of primer concentration. However, at 1.0 $\mu$M polymerase, discernible levels of amplification were seen at primer concentrations of 5 $\mu$M and above. As the polymerase levels increase, so do the amount of amplicon, up to levels of about 4 $\mu$M. At 8 $\mu$M polymerase, the amount of amplicon plateaued or dropped off, depending on the primer concentration, with a significant drop off at 16 $\mu$M at lower primer concentrations. It appears that under these extreme temperature cycling conditions for a 49 bp product, the polymerase has a favored concentration range between about 1 and 8 $\mu$M, and more specifically between 2 and 8 $\mu$M, depending on the primer concentration.

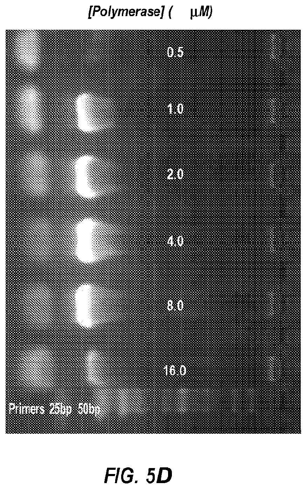

Similarly, little amplification was seen with primer concentrations of 2.5 $\mu$M. However, amplification was successful at 5 $\mu$M primer, with KlenTaq concentrations of 2-8 $\mu$M, and amplification continued to improve with increasing concentrations. Excellent amplification was achieved with primer concentrations of about 10-20 $\mu$M primer. Fig. 5C shows melting curves for various primer concentrations at 4 $\mu$M KlenTaq, while Fig. 5D verifies the size of the product as the polymerase concentration varies while the primer concentration is held at 10 $\mu$M. Despite the high concentrations of polymerase and primers, no nonspecific amplification is seen.

Without being bound to theory, it appears that the ratio between the amount of enzyme and amount of primer is important for extreme PCR cycling, provided that both are above a threshold amount. It is noted that the above amounts are provided based on each primer. Given that the polymerase binds to each of the duplexed primers, the total primer concentration may be the most important. For KlenTaq, suitable ratios are 0.03-0.4 (3-40% enzyme to total primer concentration), with an illustrative minimum KlenTaq concentration of about 0.5 $\mu$M, and more illustratively about 1.0 $\mu$M, for extreme PCR. The primers may be provided in equimolar amounts, or one may be provided in excess, as for asymmetric PCR. The optimal polymerase:primer percentage may also depend on the temperature cycling conditions and the product size. For example, standard (slow) temperature cycling often uses a much lower polymerase to primer percentage, typically 1.5 nM (0.04 U/$\mu$l) polymerase (49) and 1,000 nM total primer concentration, for a percentage of 0.15%, over 10 times lower than the percentages found effective for extreme PCR.

EXAMPLE 4

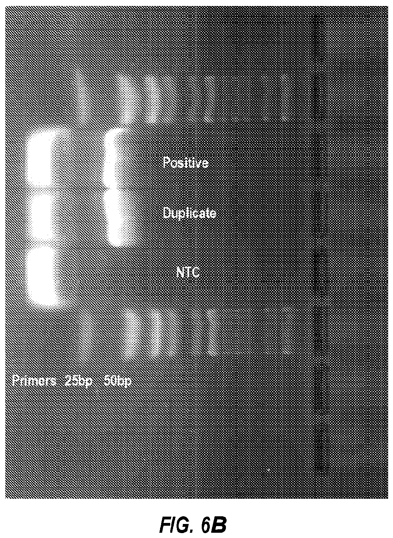

The same PCR target as in Example 3 was amplified with 8 $\mu$M polymerase and 20 $\mu$M each primer in a 19 gauge steel hypodermic needle, to increase thermal transfer and cycling speeds. The polymerase to total primer percentage was 20%. Amplification was performed on the instrument of Fig. 1B and was completed in 16 seconds using 35 cycles of 0.46 seconds each (Fig. 6A), cycling between 91°C and 59-63°C. The maximum heating rate during cycling was 407°C/s and the maximum cooling rate was 815°C/s, demonstrating that PCR can occur with ramp rates of greater than 400°C/s with no holds. Analysis of the products on a 4% NuSieve 3:1 agarose gel revealed strong specific bands of the correct size (Fig. 6B). The no template control showed no product at 49 bp, but did show a prominent primer band similar to the positive samples.

EXAMPLE 5

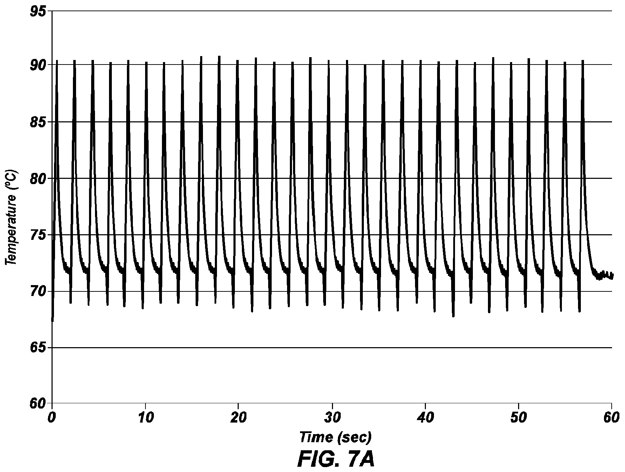

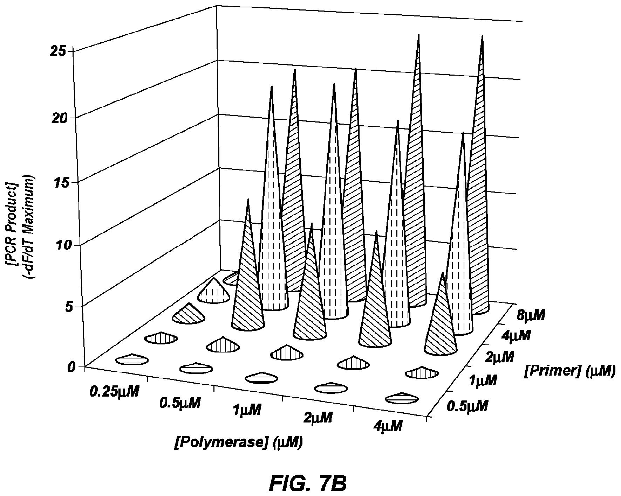

A 102 bp fragment of the NQO1 gene was amplified using primers CTCTGTGCTTTCTGTATCCTCAGAGTG GCATTCT (SEQ ID NO:10) and CGTCTGCTGGAGTGTGCCCAATGCTATA (SEQ ID NO:11) and the instrument of Fig. 1B without the real-time components. The polymerase concentration was varied between 0.25 and 4 $\mu$M, while each primer concentration was varied between 0.5 and 8 $\mu$M. The primers were designed to anneal at higher temperatures (low 70s) so that extension at a combined annealing/extension phase would be at a more optimal temperature for the polymerase. Greater polymerization rates at these temperatures were expected to enable amplification of longer products. The cooler water bath was controlled at 72°C and the end of the annealing/extension phase triggered by time (1 second), rather than temperature. Cycling between 72 and 90°C for 30 cycles required 58 seconds using 1.93 second cycles (Fig. 7A). As seen in Fig. 7A, the sample temperature drops about 3°C below the annealing/extension temperature while it travels through the air to the hot water bath. Fig. 7B shows the amount of product amplified by quantifying the melting curves as in Fig. 5A. Melting curve analysis showed only a single product of Tm 84°C. Very little product was observed at 0.25 $\mu$M polymerase or at 1 $\mu$M each primer. Some amplification occurs at 2 $\mu$M each primer, with the best amplification at 2-4 $\mu$M polymerase and 8 $\mu$M each primer. At primer concentrations of 2-4 $\mu$M, yield decreases as the polymerase concentration increases, although this was not seen at 8 $\mu$M primer concentration. Although the thermal cycling and target length are different from Example 3, the best amplification occurs at polymerase to total primer concentrations of 3.1 to 50%.

EXAMPLE 6

Extreme PCR was used to amplify 135 bp and 337 bp fragments of the *BBS2* gene using the instrument shown in Fig. 1B with real time monitoring. In order to study the effect of product length on extreme PCR and control for possible confounding effects of different primers, the fragments were first amplified from genomic DNA using primers with common 5'-end extensions. For the 135 bp fragment the primers were

ACACACACACACACACACACACACACACACACACACAAAAATTCAGTGGCATTAAA
TACG (SEQ ID NO:12)

and

GAGAGAGAGAGAGAGAGAGAGAGAGAGAGAGAGAGAGAGAGAGAGAGAAAAACCAG AGCTAAAGGGAAG (SEQ ID NO:13).

For the 337 bp fragment the primers were

ACACACACACACACACACACACACACACACACACAAAAAGCTGGTGTCTGCTAT AGAACTGATT (SEQ ID NO:14)

and

GAGAGAGAGAGAGAGAGAGAGAGAGAGAGAGAGAGAGAGAGAGAGAGAAAAAGTTG CCAGAGCTAAAGGGAAGG (SEQ ID NO:15).

. After standard PCR amplification from genomic DNA, primers and dNTPs were degraded by ExoSAP-IT (Affymetrix, CA), followed by PCR product purification using the QuickStep™ 2 PCR Purification Kit (Catalog # 33617, Edge BioSystems, Gaithersburg, MD). PCR products were diluted approximately 1 million-fold and adjusted to equal concentrations by equalizing the Cq obtained by standard real-time PCR to obtain a Cq of 25 cycles (approximately 10,000 copies/10 $\mu$l reaction).

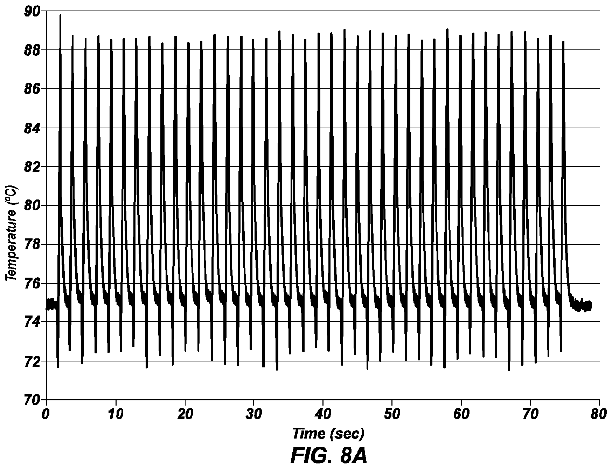

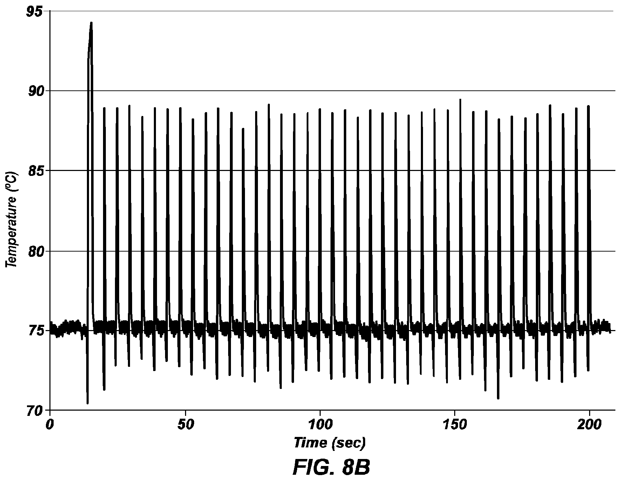

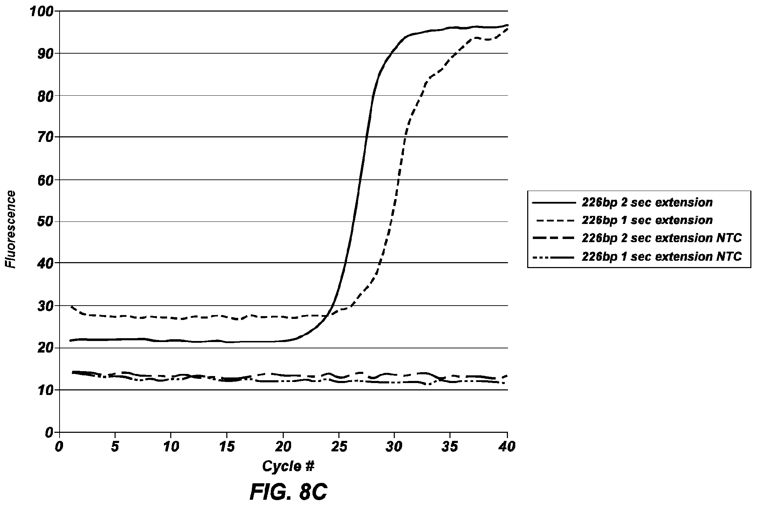

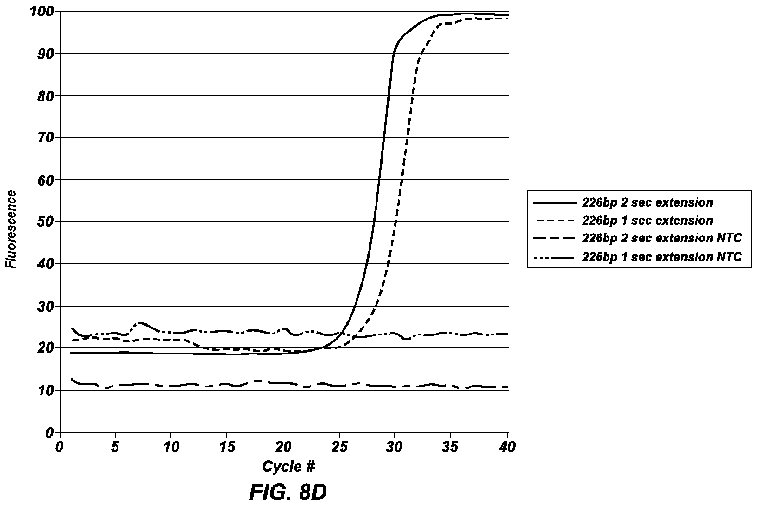

Extreme PCR was performed on 1,000 copies of the amplified templates in a total volume of 5 $\mu$l using the common primers ACACACACACACACACACACACACACACACACACAAAAA(SEQ ID NO:16) and GAGAGAGA GAGAGAGAGAGAGAGAGAGAGAGAGAGAGAGAGAGAAAAA (SEQ ID NO:17) each at 2 $\mu$M with 2 $\mu$M polymerase and 2% glycerol. The 135 bp BBS2 fragment resulted in a 226 bp product requiring extension of 176 or 185 bases (depending on the primer), while the 337 bp BBS2 fragment resulted in a 428 bp PCR product requiring extension of 378 or 387 bases. Specific amplification was verified on agarose gels and by melting analysis. The extreme PCR temperature profile used for the 226 bp product is shown in Fig. 8A, which included a 1 second combined annealing/extension at 75°C and denaturation at 87°C. Also performed was a 2 second annealing/extension phase at the same temperature (trace not shown). Real time PCR results for these amplifications are shown in Fig. 8C, revealing about a 5 cycle shift to higher Cq with the 1 second extension as compared to the 2 second extension, presumably reflecting a decrease in efficiency as the extension time is decreased. The extreme PCR temperature profile used for the 428 bp product is shown in Fig. 8B, showing a 4 second combined annealing/extension at 75°C and denaturation at 87°C. Also performed was a 5 second annealing/extension phase at the same temperature (trace not shown). Real time PCR results for these amplifications are shown in Fig. 8D, revealing about a 2 cycle shift to higher Cq with the 4 second extension as compared to the 5 second extension, presumably reflecting a decrease in efficiency as the extension time is decreased.

EXAMPLE 7

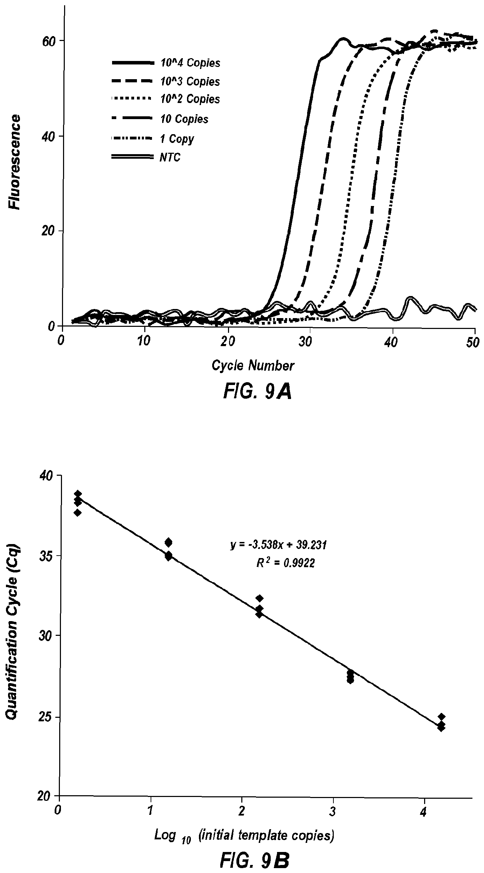

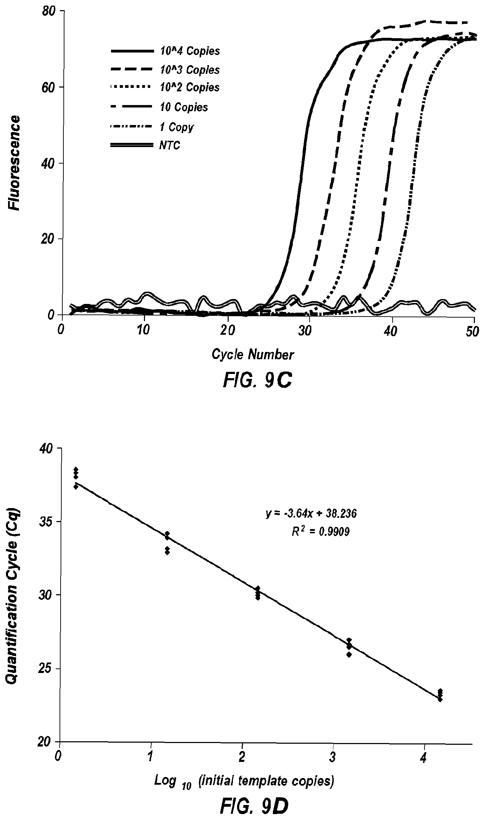

Quantitative performance of PCR was assessed using the real-time instrument of Fig. 1B for the 102 bp fragment of NQO1 of Example 5 and the 45 bp fragment of KCNE1 of Example 1 using a dilution series of human genomic DNA, using 2 $\mu$M KlenTaq and 8 $\mu$M each primer for NQO1 and 8 $\mu$M KlenTaq and 20 $\mu$M each primer for KNCE1. With a dynamic range of at least 4 decades, as seen in Figs. 9a and 9b, the amplification efficiencies calculated from the standard curves were 95.8% for NQO1 and 91.7% for KCNE1. Control reactions without template did not amplify after 50 cycles and single copy replicates (mean copy number of 1.5 copies per reaction) were similar in amplification curve shape and intensity to higher concentrations (Figs. 9A and 9C). At a mean copy number of 0.15 copies/reaction, 2 reactions were positive out of 17 (combining both NQO1 and KCNE1 trials), with a calculated expectation of 0.13 copies/reaction by binomial expansion.

EXAMPLE 8

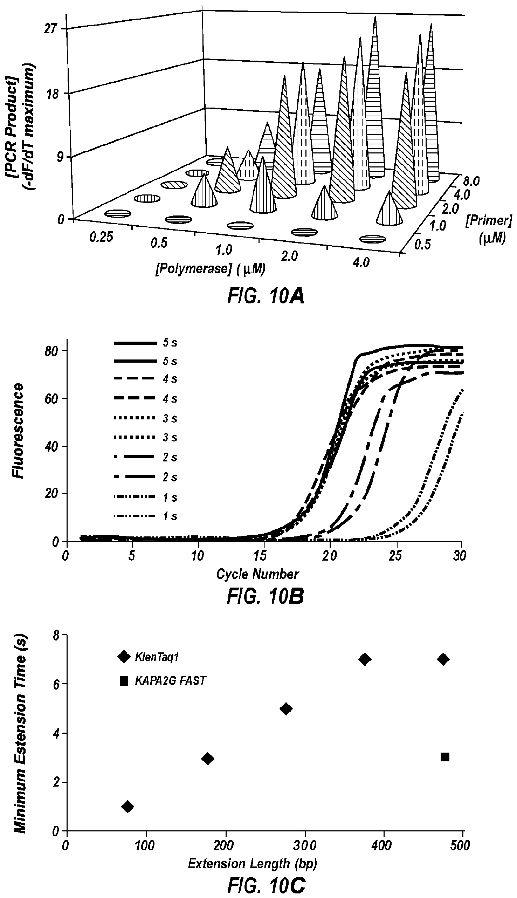

The extension time required for different product lengths using real-time PCR (Fig. 10A-C). To control for the possible confounding effects of different primers, synthetic templates of 100-500 bp using the following common high Tm (77 °C) primers:

ACTCGCACGAACTCACCGCACTCC (SEQ ID NO:18) and
GCTCTCACTCGCACTCTCACGCACA (SEQ ID NO:19).

The synthetic template sequences were:

100 bp Template:

ACTCGCACGAACTCACCGCACTCCGGATGGATTGTGAAGAGGCCCAAGATACTGGT CATATTATCCTTTGATCTAGCTCTCACTCGCACTCTCACGCACA (SEQ ID NO:20).

200 bp Template:

ACTCGCACGAACTCACCGCACTCCTCAATGCTGACAAATCGAAAGAATAGGAATAG CGTAATTACTAGAGGACTCCAATATAGTATATTACCCTGGTGACCGCCTGTACTGTA GGAACACTACCGCGGTTATATTGACAGCTTAGCAATCTACCCTGTTGGGATCTGTTT AAGTGGCTCTCACTCGCACTCTCACGCACA (SEQ ID NO:21).

300 bp Template:

ACTCGCACGAACTCACCGCACTCCCCTTCGAATATAAAGTACGACATTACTAGCAAT GACAGTTCCAGGATTTAAGAAAGTAGTGTTCCACATCAATGCATATCCAGTGAAAGC ATAACGTCAAAAAAAGCCTGGCACCGTTCGCGATCTGGACTTACTTAGATTTGTTGT AGTCAAGCCGGCTATCAGCGATTTATCCCGGAAACACATACTAGTGAGTTATTTGTA TGTTACCTAGAATAGCTGTCACGAATCACTAATACATTCACCCACCAGCTCTCACTC GCACTCTCACGCACA (SEQ ID NO:22).

400 bp Template:

ACTCGCACGAACTCACCGCACTCCTGAATACAAGACGACAGTCCTGATTATATTTTC ATTTAATTACGCCAATTTAATTATGATGAATATTAACGGAATTAAATATGTATTGAT AAGTACTAAGTAATGGTTTACCCACGGCGATCTATATGCAAGGGAAACATTAACAA ATTTAAACATCTGATGTGGACAAAACTTGTAATGTGGTATAGTTAAAAATATAGGTT TCAGGGACACGTAAGTATCTATCTTGAATGTTTAAGTAGGTCCTGTCTACCATTCTG AAATTTAGAAATCGCGTTCATCGGGCTGTCGGCTACACCTCAGAAAACCATTTCGT GTTGCACAGGAGGAACTTTCGAGGGTTCGTATGAGCTCTCACTCGCACTCTCACGCA CA (SEQ ID NO:23).

500 bp Template:

ACTCGCACGAACTCACCGCACTCCACCGCTTGACGACGTAGGGTATTTGGTATCTGA
ATCTACTCATTTACCTACATACTGAAGATTTTGCGATCGTCTAATATATTGGACTAAT
GCCCGATTTCTGATCAATTACTCTAGGCGATACTTCATCGCTGGCCTTATTTGGATTT
TGCTCAAGTGCTAAACTCTCTGCGCGTCAATACTAGTCTGACATCAGTCAAGACCTG
CTATCTGAAAACTACTAGAGAGATATACCTAACAACTTAGTGGATAAATCAGGTCT
GGAGATTGTCATATAATGCCACTAGGGTCAGAAGGCTGTGTCAAAGTTAGTGGTTAG
TAGGTCTCCGCTCTGCGGTACTATTCTTATATTCTCTTACTATGCATCAAACAAAATA
GAATGCATAGACAAACCGCCTGCCAAGTTTACAAGATAACTTGCGTATAGGTTTATA
AGGGTTCTTCTGTATCGCTCTCACTCGCACTCTCACGCACA (SEQ ID NO:24).

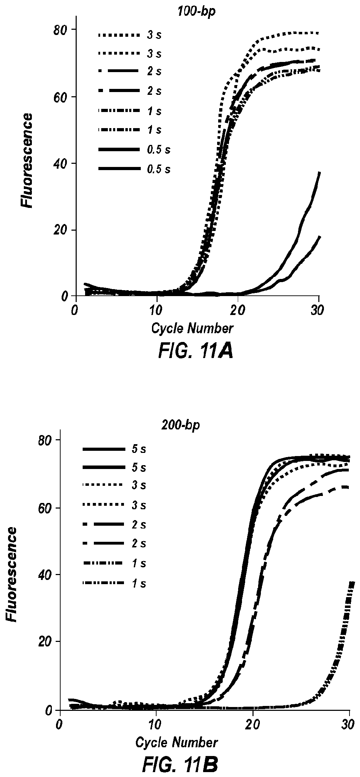

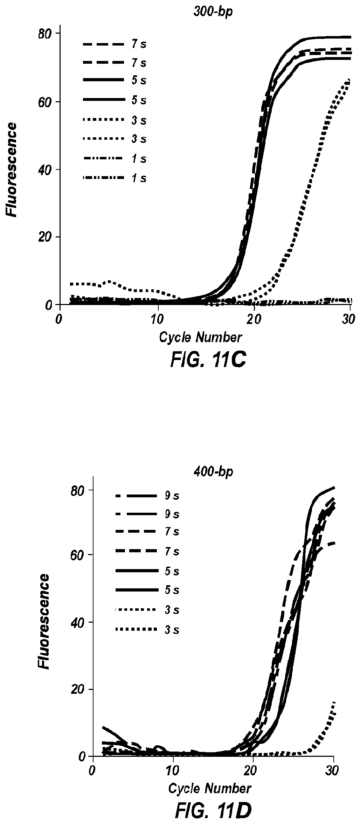

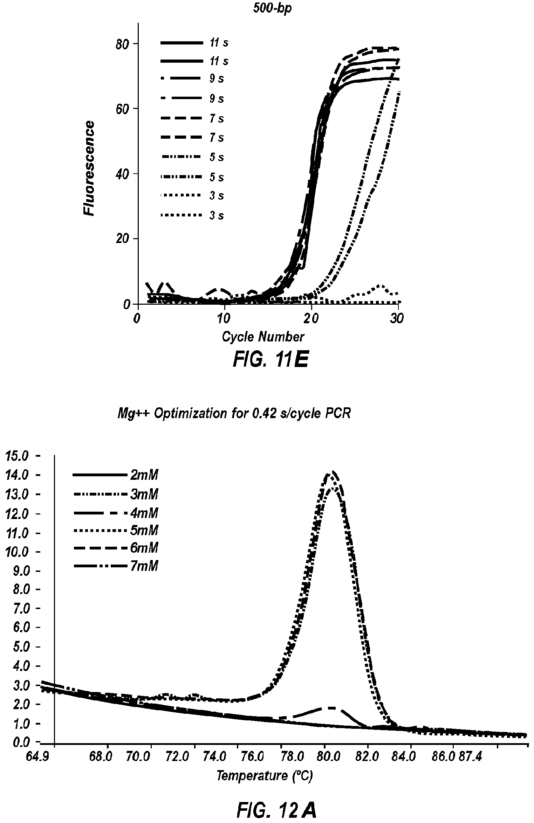

Optimal concentrations of primers and polymerase were first determined for the intermediate length 300-bp product using a 4 second combined annealing/extension segment with 4.9 seconds per cycles (Fig. 10A). Identical primer (4 µM) and polymerase (2 µM) concentrations were then used for all product lengths and minimum extension times were determined (Fig. 11A-E). Depending on the product length, increased extension times resulted in decreased fractional quantification cycles (Cq) until no further change was observed, reflecting the minimum extension time required for efficient PCR. For example, amplification curves using the KAPA2G™ FAST polymerase (Kapa Biosystems) for the 500 bp product are shown in Fig. 10B. The minimum extension time using KAPA2G FAST polymerase was 3 s, compared to 7 s using KlenTaq1 (a deletion mutant of Taq polymerase, AB Peptides). When the identity of the polymerase is kept constant, longer products required longer extension times (Fig. 10C). For KlenTaq1 polymerase, about 1 second is required for each 60 bps, while for KAPA2G FAST, 1 second is required for each 158 bp. It is noted that these two polymerases were chosen because they are commercially available at sufficient concentrations, while most other polymerases are not commercially available at such high concentrations. It is understood that the required time for extension depends directly and linearly with the length to be extended, and inversely with the concentration of polymerase and the polymerase speed. A proportionality constant (k2) can be defined that relates these 3 parameters:

$$\text{Required Extension Time} = k2*(\text{extension length})/([\text{polymerase}]*(\text{polymerase speed}))$$

EXAMPLE 9

Extreme PCR times can also be reduced with high Mg++ concentrations. A 60 bp fragment of AKAP10 was amplified with primers:

GCTTGGAAGATTGCTAAAATGATAGTCAGTG (SEQ ID NO:25) and
TTGATCATACTGAGCCTGCTGCATAA (SEQ ID NO:26), to generate the amplicon

GCTTGGAAGATTGCTAAAATGATAGTCAGTGAC(**A/G**)TTATGCAGCAGGCTCAGTAT
GATCAA (SEQ ID NO:27).

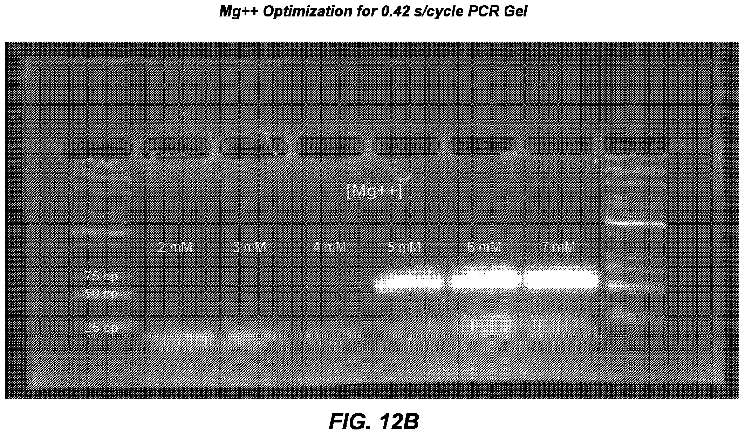

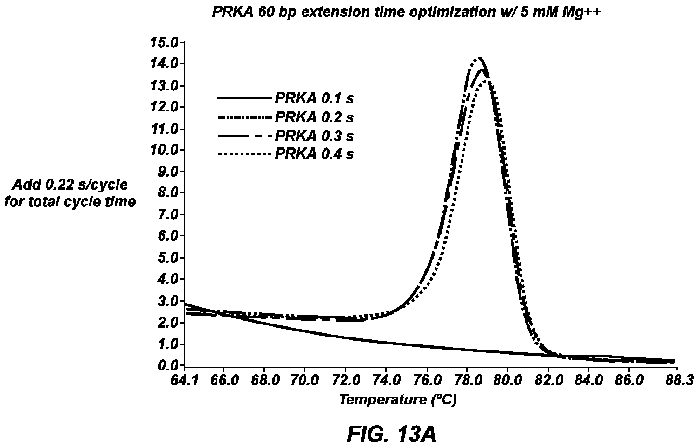

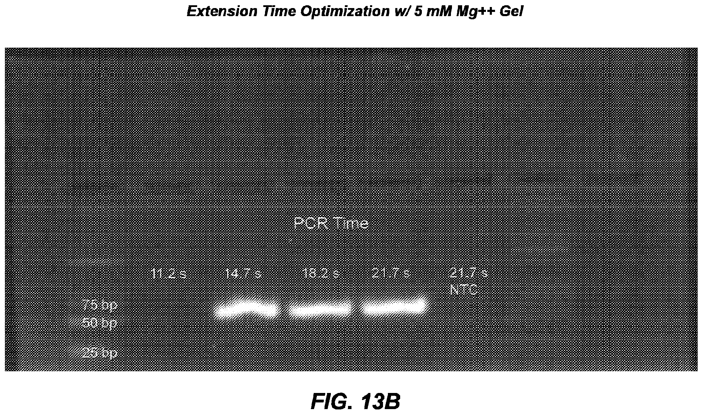

Each reaction was in a 1 µl volume with time based control (0.07 seconds in a 94°C water bath, 0.1-0.4 seconds in a 60°C water bath) for 35 cycles using 2-7 mM MgCl$_2$. The sample volume was 1 µl, with 5 ng human genomic DNA, 20 µM primers, and 8 µM polymerase. Using a 0.42 second per cycle protocol, when the MgCl$_2$ was 2-3 mM, no product was observed on melting curves (Fig. 12A) or gels (Fig. 12B). Minimal product was present at 4 mM, but a large amount of product was observed after amplification with 5-7 mM MgCl$_2$. At 5 mM MgCl$_2$, no products were observed on melting curves (Fig. 13A) or gels (Fig. 13B) with cycle times of 0.32 seconds, but large amounts of product were present at cycle times of 0.42 seconds, 0.52 seconds, and 0.62 seconds, demonstrating that specific, high yield 60 bp products can be obtained in PCR performed in under 15 seconds (35 cycles in 14.7 seconds). Thus, illustrative Mg++ concentrations are at least 4 mM, at least 5 mM, at least 6 mM, at least 7 mM, or more, and it is understood that these illustrative Mg++ concentrations may be used with any of the embodiments described herein.

EXAMPLE 10

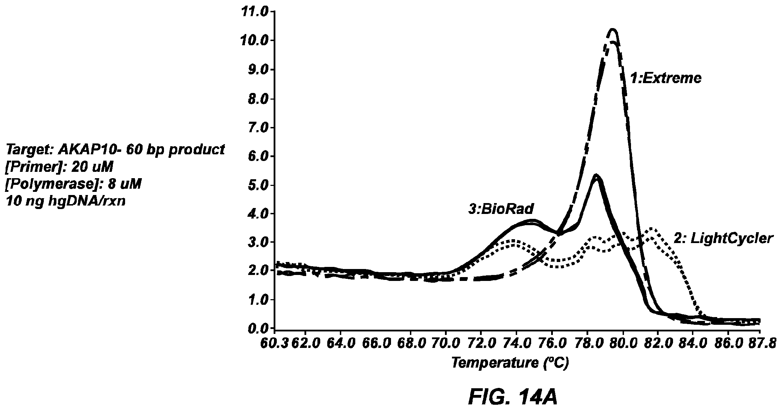

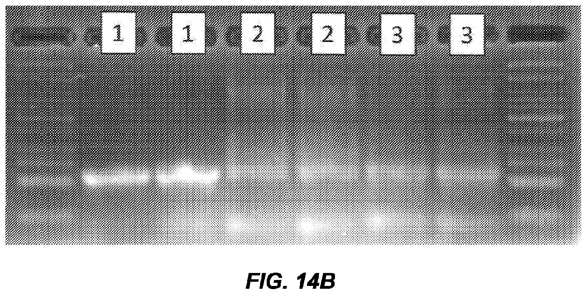

The high concentrations of primer and polymerase used in extreme PCR can have detrimental effects when used at slower cycling speeds. Non-specific products were obtained on rapid cycle or block based instruments that are 32- or 106-fold slower, respectively. Fig. 14A-B shows the results comparing amplification of the AKAP10 60 bp product used in Example 9, wherein amplification was performed using 20 $\mu$M of each primer, 8 $\mu$M KlenTaq and 10 ng human genomic DNA for 40 cycles using: (1) extreme PCR with set times of 0.5 s at 94°C and 0.2 seconds at 60°C, giving a total time of approximately 17 seconds, (2) Rapid cycle PCR (Roche LightCycler) using set times of 10 s at 94°C for an initial denaturation, followed by cycles of 85°C for 0 seconds, and 60°C for 0 seconds, giving a total time of approximately 9 minutes, and (3) Legacy (block) temperature cycling (Bio-Rad CFX96) with a 10 s initial denaturation at 94°C, following by temperature cycling for 0 s at 85°C and 5 s at 60°C with a total time of approximately 30 minutes. As can be seen, even the rapid cycling of the LightCycler resulted in quite a bit of non-specific amplification, while the extreme cycling conditions resulted in a single melting peak and minimal non-specific amplification on the gel.

It also noted that the yield is enhanced in extreme PCR, resulting from high primer and polymerase concentrations. Extreme PCR produced over 30-fold the amount of product compared to rapid cycle PCR, using quantitative PCR for comparison (data not shown).

Examples 1 - 10 were all performed using one or more of the devices described in Figs. 1a - 1d, or minor variations on those configurations, with certain steps performed on the LightCycler, to confirm qPCR results. However, it is understood that the methods and reactions described herein may take place in a variety of instruments. The water baths and tubes used in these examples allow for sufficiently rapid temperature change to study the effects of elevated concentrations of primers and polymerase. However, other embodiments may be more suitable commercially. Microfluidics systems, with low volume and high surface area to volume ratios, may be well suited to extreme PCR. Such systems allow for rapid temperature changes required by the high concentrations of primers and polymerase that are used in extreme PCR. Microfluidics systems include micro-flow systems (35, 53) that incorporate miniaturized channels that repeatedly carry the samples through denaturation, annealing, and extension temperature zones. Some of these systems have already demonstrated effective PCR with cycle times as fast as 3 seconds for lower complexity targets. It is expected that more complex targets may be amplified in such systems if the polymerase is provided at a concentration of at least 0.5 $\mu$M and primers are each provided at a concentration of at least 2 $\mu$M. Stationary PCR chips and PCR droplet systems (54) may also benefit from increased primer and probe concentrations, as the volumes may be as small as 1 nl or smaller and may be low enough to permit very fast cycling. It is understood that the exact instrumentation is unimportant to the present invention, provided that the instrumentation temperature cycles fast enough to take advantage of increased primer and polymerase concentrations without suffering from the loss of specificity associated with higher primer concentrations at slower cycle speeds.

While the above examples all employ PCR, it is understood that PCR is illustrative only, and increased primer and enzyme concentrations combined with shorter amplification times are envisioned for nucleic acid amplification methods other than PCR. Illustrative enzymatic activities whose magnitude may be increased include polymerization (DNA polymerase, RNA polymerase or reverse transcriptase), ligation, helical unwinding (helicase), or exonuclease activity (5' to 3' or 3' to 5'), strand displacement and/or cleavage, endonuclease activity, and RNA digestion of a DNA/RNA hybrid (RNAse H). Amplification reactions include without limitation the polymerase chain reaction, the ligase chain reaction, transcription medicated amplification (including transcription-based amplification system, self-sustained sequence replication, and nucleic acid sequence-based amplification), strand displacement amplification, whole genome amplification, multiple displacement amplification, antisense RNA amplification, loop-mediated amplification, linear-linked amplification, rolling circle amplification, ramification amplification, isothermal oligonucleotide amplification, helicase chain reaction, and serial invasive signal amplification.

In general, as the enzyme activity is varied, the amplification time varies inversely by the same factor. For reactions that include primers, as the primer concentration is varied, the amplification time varies inversely by the same factor. When both primers and enzymes are required for amplification, both enzyme and primer concentrations should be varied in order to maximize the reaction speed. If primer annealing occurs in a unique segment of the amplification cycle (for example, a unique temperature during 3-temperature PCR), then the time required for satisfactory completion of primer annealing in that segment is expected to be inversely related to the primer concentration. Similarly, if the enzyme activity is required in a unique segment of the amplification cycle (for example, a unique temperature during 3-temperature PCR), then the time required for satisfactory completion of the enzymatic process in that segment is expected to be inversely related to the enzyme concentration within a certain range. Varying the primer or enzyme concentrations can be used to change the required times of their individual segments, or if both occur under the same conditions (such as in 2-temperature PCR or during an isothermal reaction process), it is expected that a change in both concentrations may be necessary to prevent one reaction from limiting the reaction speed. Increased Mg$^{++}$ concentration can also be used in combination with increased enzyme and primer concentrations to further speed amplification processes. Higher Mg$^{++}$ concentrations both increase the speed of primer annealing and reduce the time for many enzymatic reactions used in nucleic acid amplification.

Higher concentrations of Mg$^{++}$, enzymes, and primers are particularly useful when they are accompanied by shorter amplification times or segments. When higher concentrations are used without shortening times, non-specific amplification products may occur in some cases, as the "stringency" of the reaction has been reduced. Reducing the amplification time or segment time(s) introduces a higher stringency that appears to counterbalance the loss of stringency from increased reactant concentrations. Conversely, reagent costs can be minimized by reducing the concentration of the reactants if these lower concentrations are counterbalanced by increased amplification times or segment times.

Increasing polymerase concentrations can reduce the time necessary for long-range PCR, illustratively where the target is 5-50 kb. Typically, 10 min to 30 min extension periods are used to amplify large targets because the target is so long that such times are needed: 1) for the polymerase to complete extension of a single target, and 2) for enzyme recycling to polymerize additional primed templates. This recycling of polymerase is not needed at the beginning of PCR, when the available enzyme outnumbers the primed template molecules. However, even before the exponential phase is finished, the number of polymerase molecules often becomes limiting and enzyme recycling is necessary. By increasing the concentration of the polymerase, the required extension period can be reduced to less than 5 minutes and possibly less than 2 minutes, while maintaining increased yield due to the high primer concentration. Although the actual enzyme speed is not increased, less recycling is necessary, affecting the minimum time required, approximately in a linear fashion with the enzyme concentration.

Cycle sequencing times can also be reduced by increasing primer and polymerase concentrations. Typically, standard cycle sequencing primer concentrations are 0.16 μM and the combined annealing/extension period is 10 min at 50-60 degrees C. By increasing the primer and polymerase concentrations by 10-fold, the time required for annealing/extension can be reduced approximately 10-fold. In both long PCR and cycle sequencing, the expected time required is inversely proportional to the polymerase or primer concentration, whichever is limiting.

PCR of fragments with ligated linkers that are used as primers in preparation for massively parallel sequencing can be completed in much less time than currently performed by combining extreme temperature cycling with higher concentrations of primers, polymerase, and/or Mg$^{++}$.

In all of the above applications, it is expected that the specificity of the reaction is maintained by shorter amplification times. Although high primer and polymerase concentrations are expected by those well versed in the art to cause difficulty from non-specific amplification, minimizing the overall cycle time and/or individual segment times results in high specificity and efficiency of the PCR.

**Table** 2. Extreme PCR conditions for different targets.

| Target | KCNEI | KCNE1 | IRL10RB | IRL10RB | IRL10RB | NQO1 | AKAP10 | Synthetic | Synthetic | Synthetic | Synthetic | Synthetic | Synthetic | Synthetic |
|---|---|---|---|---|---|---|---|---|---|---|---|---|---|---|
| Amplicon Size (bp) | 45 | 45 | 49 | 49 | 58 | 102 | 60 | 100 | 200 | 300 | 300 | 400 | 500 | 500 |
| Polymerase | KlenTaq1 | KlenTaq1 | KlenTaq1 | KlenTaq1 | KlenTaq1 | KlenTaq1 | KlenTaq1 | KlenTaq1 | KlenTaq1 | KlenTaq1 | KlenTaq1 | KlenTaq1 | KlenTaq1 | KAPA2G FAST |
| [Polymerase] | 1 | 8 | 4 | 8 | 2 | 2 | 8 | 2 | 2 | 2 | 2 | 2 | 2 | 2 |
| [Primers] | 10 | 20 | 10 | 20 | 10 | 8 | 20 | 4 | 4 | 4 | 4 | 4 | 4 | 4 |
| # Cycles | 35 | RT | 35 | 35 | 39 | 30 | 35 | RT | RT | 20 | RT | RT | RT | RT |
| Cycle Time (s) | 0.8 | 0.91 | 0.73 | 0.45 | 0.97 | 1.93 | 0.42 | 1.9 | 3.9 | 4.9 | 5.9 | 7.9 | 7.9 | 3.9 |
| PCR Time (s) | 28 | RT | 26 | 16 | 38 | 58 | 14.7 | RT | RT | 98 | RT | RT | RT | RT |
| Hot Water Temp (°C) | 95.5 | 95.5 | 95.5 | 95.5 | 95.5 | 95.5 | 95.5 | 95.5 | 95.5 | 95.5 | 95.5 | 95.5 | 95.5 | 95.5 |
| Cold Water Temp (°C) | 20 | 58 | 30 | 30 | 30 | 72 | 59 | 76 | 76 | 76 | 76 | 76 | 76 | 76 |
| Hot Trigger Temp (°C) | 90 | 85 | 90 | 90 | 90 | 90 | Time | 92 | 92 | 92 | 92 | 92 | 92 | 92 |
| Cold Trigger Temp (°C) | 70 | 62 | 70 | 70 | 70 | Time | Time | Time | Time | Time | Time | Time | Time | Time |
| Denaturation (°C) | 90 | 85 | 90 | 90 | 90 | 90 | (82-85) w/ TC | 92 | 92 | 92 | 92 | 92 | 92 | 92 |
| Ann/Ext (°C) | 60 | 60 | 65 | 65 | 65 | 72 | 60 | 76 | 76 | 76 | 76 | 76 | 76 | 76 |
| Ann/Ext Time (s) | 0 | 0 | 0 | 0 | 0 | 1 | 0.1-0.4 | 0.5-3 | 1-5 | 4 | 1-7 | 3-9 | 3-11 | 1-5 |
| Figure | 9a | 9a | 5a | 5a | 4c | 7a | 12a, | 11a | 11b | 10a, 11c | 11c | 11d | 11e | 10b |
| Tm | 81 | 81 | 80 | 80 | 83 | 85 | 79 | 85 | 85 | 85 | 85 | 81/87 (2 domains) | 84 | 84 |
| Mg$^{++}$ | 3 | 3 | 3 | 3 | 3 | 3 | 2-7 | 3 | 3 | 3 | 3 | 3 | 3 | 3 |

Time = time-based segment control does not have a temperature trigger
RT = real-time acquisition

Table 3. Derivation of rate constants (k1 for primer annealing and k2 for polymerase extension) using A) historical ranges, B), the equation for primer annealing, and C) the equation for polymerase extension.

| A) | [Primer] (μM) | [Polymerase] (μM) | Polymerase Speed (nt/s) | Extension Length (bp) | Cycle Time (s) | Anneal/Extend Time (s) | [Mg++] |
|---|---|---|---|---|---|---|---|
| Standard | 0.05 - 0.5 | 0.0026 - 0.026 | 10 - 45 | 20 - 980 | 120 - 480 | 15 - 60 | 1.5 |
| Rapid Cycle | 0.2 - 1.0 | 0.063 | 55 - 90 | 20 - 480 | 20-60 | 1 - 10 | 3 |
| Extreme | 1-16 | 0.5 - 8 | 50 - 100 | 20 - 280 | 0.5-5 | <0.1- 5 | 3 - 7 |
| Opt Extreme #1 | 10 | 2.50 | 60 | 29 | 0.73 | <0.1 | 3 |
| Opt Extreme #2 | 4 | 0.50 | 60 | 82 | 1.93 | 1 | 3 |
| Opt Extreme #3 | 4 | 0.75 | 60 | 280 | 4.9 | 4 | 3 |

B) If Required Annealing time = k1/[primer]

| | [Primer] (μM) | Anneal/Extend Time (s) | k1 (s * μM) | | k1 range (s*μM) |
|---|---|---|---|---|---|
| Min Standard | 0.05 | 15 | 0.75 | Standard | 0.75 - 30 |
| Max Standard | 0.5 | 60 | 30 | Rapid Cycle | 0.2 - 10 |
| Min Rapid Cycle | 0.2 | 1 | 0.2 | Extreme | 1 - 20 |
| Max Rapid Cycle | 1 | 10 | 10 | | |
| Opt Extreme #1 | 10 | 0.1 | 1 | | |
| Opt Extreme #2 | 4 | 1 | 4 | | |
| Opt Extreme #3 | 4 | 5 | 20 | | |

C) If required extension time = k2*product length/(polymerase speed*[polymerase])

| | [Polymerase] (μM) | Polymerase Speed (nt/s) | Extension Length (bp) | Anneal/Extend Time (s) | k2 (1/μM) |
|---|---|---|---|---|---|
| Opt Extreme #1 | 2.5 | 60 | 29 | 0.1 | 0.52 |
| Opt Extreme #2 | 0.5 | 60 | 82 | 1 | 0.37 |
| Opt Extreme #3 | 0.75 | 60 | 280 | 4 | 0.64 |

Specific conditions for extreme PCR are shown in Table 2. All data are presented except for the simultaneous optimization experiments for polymerase and primer concentrations for 3 of the targets. In Table 3, the quantitative relationships between variables are detailed. The inverse proportionality that relates the required annealing time to the primer concentration is approximately constant (k1) and defined by the equation (Required annealing time) = k1/[primer]. Using a range of typical values for these variables under conditions of legacy (standard) PCR, rapid cycle PCR, and extreme PCR produces ranges for the inverse proportionality constant that largely overlap (legacy 0.75 - 30, rapid cycle 0.2 - 10, and extreme 1 - 20). Because of this constant inverse proportionality, desired annealing times outside of those currently performed can be used to predict the required primer concentrations for the desired time. For example, using a constant of 5 (s * μM), for an annealing time of 0.01 s, a primer concentration of 500 μM can be calculated. Conversely, if a primer concentration of 0.01 μM were desired, the required annealing time would be 500 seconds. Although these conditions are outside the bounds of both legacy and extreme PCR, they predict a relationship between primer concentrations and annealing times that is useful for PCR success. Reasonable bounds for k1 across legacy, rapid cycle and extreme PCR are 0.5 - 20 (s x μM), more preferred 1 - 10 (s x μM) and most preferred 3 - 6 (s x μM).

Similar calculations can be performed to relate desired extension times to polymerase concentration, polymerase speed, and the length of the product to be amplified. However, because of many additional variables that affect PCR between legacy, rapid cycle and extreme PCR (polymerase, Mg$^{++}$, buffers), performed in different laboratories over time, it may be best to look at the well-controlled conditions of extreme PCR presented here to establish an inverse proportionality between variables. This allows a quantitative expression between polymerase concentration, polymerase speed, product length, and the required extension time under extreme PCR conditions. The defining equation is (Required Extension Time) = k2(product length)/([polymerase]*(polymerase speed)). The experimentally determined k2 is defined as the proportionality constant in the above equation under conditions of constant temperature, Mg$^{++}$, type of polymerase, buffers, additives, and concentration of dsDNA dye. For the 3 extreme PCR targets with two dimensional optimization of [polymerase] and [primer], the [polymerase] at the edge of successful amplification can be discerned across primer concentrations and related to the other 3 variables. As shown in Table 3, the values of k2 for these 3 different targets vary by less than a factor of 2, from which it is inferred that k2 is a constant and can be used to predict one variable if the others are known. The required extension time is proportional to the extension length (product length minus the primer length) and inversely proportional to the polymerase speed and concentration of polymerase. k2 has units of (1/$\mu$M) and an optimal value for the extreme PCR conditions used here of 0.5 (1/$\mu$M) with a range of 0.3 - 0.7 (1/$\mu$M). Similar values for k2 could be derived for other reaction conditions that vary in polymerase type, Mg$^{++}$ concentration or different buffer or dye conditions.

Extreme PCR can be performed in any kind of container, as long as the temperature of the sample(s) can be changed quickly, and preferably homogeneously. Both intra-sample, and inter-sample homogeneity is important in many applications, illustratively for quantitative PCR where different PCR efficiencies of different samples translate directly to quantification errors. In addition to standard tubes and capillaries, micro-droplets of aqueous reactions suspended in an oil stream or thin 2-dimensional wafers provide good thermal contact. Continuous flow PCR of a sample stream (either dispersed as droplets, separated by bubbles, or other means to prevent mixing) past spatial segments at different temperatures is a good method for the temperature control needed for the speeds of extreme PCR. Induction heating, as described in WO 2015/069743, may provide suitable methods and devices for extreme PCR.

EXAMPLE 11

Molecular crowding agents (or "molecular crowders") are high molecular weight molecules that, when used in sufficient concentration, can alter the properties of other molecules in that solution. Molecular crowders occupy volume and can concentrate other molecules in solution, illustratively by absorbing or locking up available water, thereby increasing the effective concentration of the other molecules. Molecular crowders can also affect the folding and binding of a variety of molecules. While molecular crowders have been used with isothermal amplification (66), prior work with PCR (67) has suggested that molecular crowders can improve the efficiency of PCR, but such efficiency improved the most under prolonged cycling times, with holds of up to 30 min. It is believed that molecular crowders can be used to concentrate PCR reactants to allow for increased efficiency with faster cycle times. Also, because molecular crowders usually increase the boiling temperature of solutions, it is believed that molecular crowders may aid in protecting PCR reactions from temperature overshoots that can sometimes happen in PCR instruments and in sterilization of products or components, particularly if the temperature exceeds 100°C. This and the following examples explore use of molecular crowders in fast PCR amplification.

In this example, a test pouch 510 was used in a FilmArray instrument, where the test pouch included 11 target assays plus one first-stage PCR control and one second-stage PCR control, wherein the targets are a combination of natural non-pathogenic and synthetic sequences that were designed to mimic the performance of commercial FilmArray pouches. Thus, various performance characteristics may be studied without risk of contamination to commercial or potentially commercial assays. Each amplicon was between 50 and 255 bp for inner amplicons (second-stage), with a median size of 107 bp, and between 105 and 474 bp for outer amplicons, with a median size of 245 bp (first-stage).

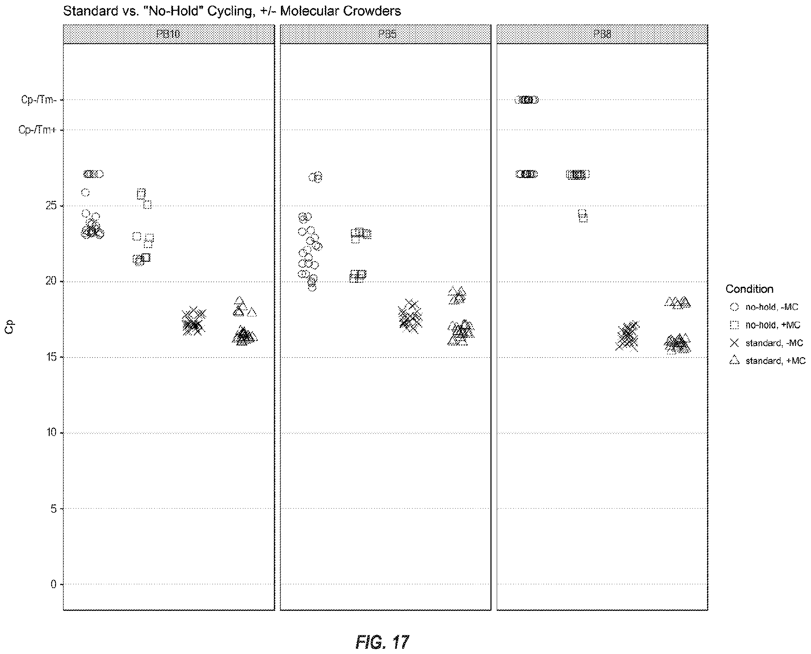

In a typical FilmArray run using pouch 510, first-stage amplification takes place with cycle times of 50 sec/cycle for 26 cycles, and second-stage amplification takes place with cycle times of 46 sec/cycle for 30 cycles. Algorithms that calculate Cp from real-time fluorescence amplification curves often have difficulty making a correct call when the amplification occurs very late (near the last cycle of PCR), since there are few data points after the positive signal. As a consequence, in a test version of the FilmArray software, the the Cp value for late amplification curves (25 to to 27.1 In Fig. 17, positives with a late Cp, i.e. a Cp later than 27, are compresed and are plotted as having a Cp of 27, whereas assays that did not have an Cp after 30 cycles but had a melt curve showing proper Tm were plotted as Cp-/Tm+ (presumably very low level amplication), and those assays that did not have Cp and did not show a melt curve were plotted as Cp-/Tm- (no amplification).

In this Example, the test pouch was run with yeast only, provided at 7.63E+04 copies/pouch, and tested for three different yeast assays at these cycling times. When cycling speeds were shortened by 19 s per cycle, the average Cp increased by at least 4 cycles (see Fig. 17, compare standard -MC with no-hold -MC, where -MC refers to pouches run without molecular crowders). In addition there were several instances of replicates failing to amplify, as indicated by the a Y-axis value of Cp-/Tm- (neither Cp nor Tm observed). It is understood that references to Cp in these examples are references to the Cp in second-stage amplification, not total amplification. It is also understood that there is a substantial dilution step between first-stage amplification and second-stage amplification. The data in Figure 17 demonstrate that the chemical composition of the first and second stage amplification reactions of this pouch are not sufficient to support robust amplification with faster cycling conditions.

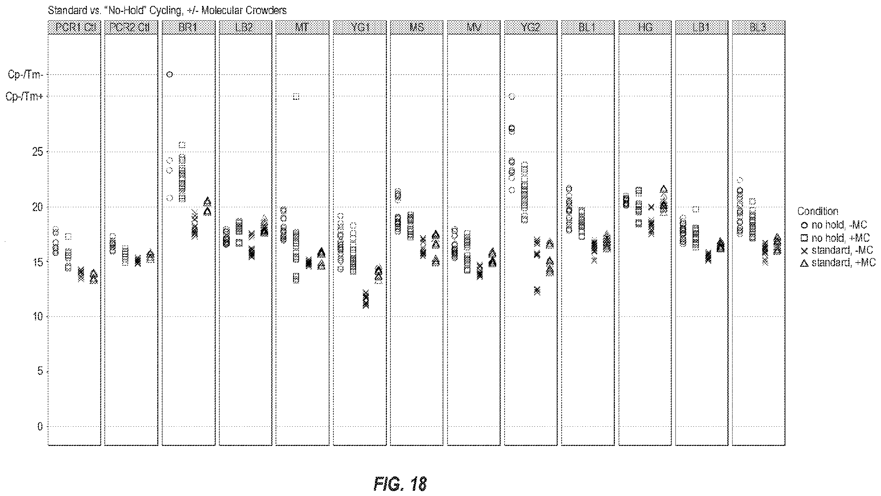

In a high order multiplex system such as the test pouch and commercial FilmArray pouches, it may not be desirable to increase the primer concentration to 2 $\mu$M or more for each of the primers, as the number of primers in first-stage amplification is quite high, and such an increase in concentration of primers can be expensive and difficult to manufacture. Moreover, one might expect to find an increase in the amount of non-specific amplification when 20 or more pairs of primers are present, each at a concentration of 2 $\mu$M, although it is understood that first-stage non-specific amplification may be mitigated illustratively by dilution and nesting second-stage primers or by other means. Even with dilution and nesting, it is understood that nonspecific amplification in the first-stage amplification reaction may be detrimental to the overall system sensitivity in that it diverts resources in the reaction (primers, dNTPs, DNA Polymerase) away from amplifying the specfic out products. In this example, rather than increasing the overall concentration of primers, molecular crowders were provided to increase the local concentration of the primers. In the test pouch, it is possible to inject fluid directly into one or more injection channels 515, rather than by inserting the fluid only into the two injection ports. In one illustrative example, a solution containing 15% w/v Ficoll 70 and 7.5% w/v Ficoll 400 (g/100mL of final solution) was laterally injected into injection channels 515e to 515j, and this mixture was used to hydrate the reagents provided in these injection channels. By providing the molecular crowder mixture in injection channels 515e to 515j, first-stage amplification may be performed in the presence of this mixture, and dilution for second-stage amplification will be performed using mixtures having the same concentration of molecular crowders, along with the rehydrated reagents. Yeast *(S.pombe)* was provided as a freeze dried component in injection channel 515a, and injection channel 515a was hydrated with FilmArray sample buffer 1A. Injection channels 515a through 515d were hydrated with water. In the test pouch, no reagents are provided in injection wells 515k or 515l and those injection wells are not used. As seen in Fig. 17, when the test pouch was run at standard conditions, Cp values were in the 15-20 range for each of the three assays. When replicates of the same pouch were run at the faster cycling protocols, many replicates had late Cps or failed to amplify. However, when the test pouch was run at the faster speed in the presence of the Ficoll mixture ("no hold +MC"), the Cp values improved, although they were still delayed relative to the test pouches run with the slower cycling time. Fig. 18 shows similar results for eleven assays in the test pouch 510 when all targets are present.

The improved Cp values suggest that molecular crowders may be used to recover at least a portion of the amplification efficiency lost when cycle times are decreased, even when the absolute concentration of the primers is not increased. Without being bound by theory, it is believed that molecular crowders concentrate regents present in the solution, thereby increasing the effective concentration of primers and/or polymerase present. Thus, molecular crowders can be used to increase PCR efficiency when cycle times are reduced.

While a mixture of 15% Ficoll 70 and 7.5% Ficoll 400 was used in this and other examples herein, it is understood that this mixture is illustrative only. A variety of other Ficoll mixtures performed similarly (data not shown). In addition, it is expected that other molecular crowders, including but not limited to other Ficolls, polyethylene glycols, dextran, sucrose, other sugars, ovalbumin, other proteins, all of varying molecular weights based on the specifics of the reaction, may be used in combination with various fast PCR reactions.

EXAMPLE 12

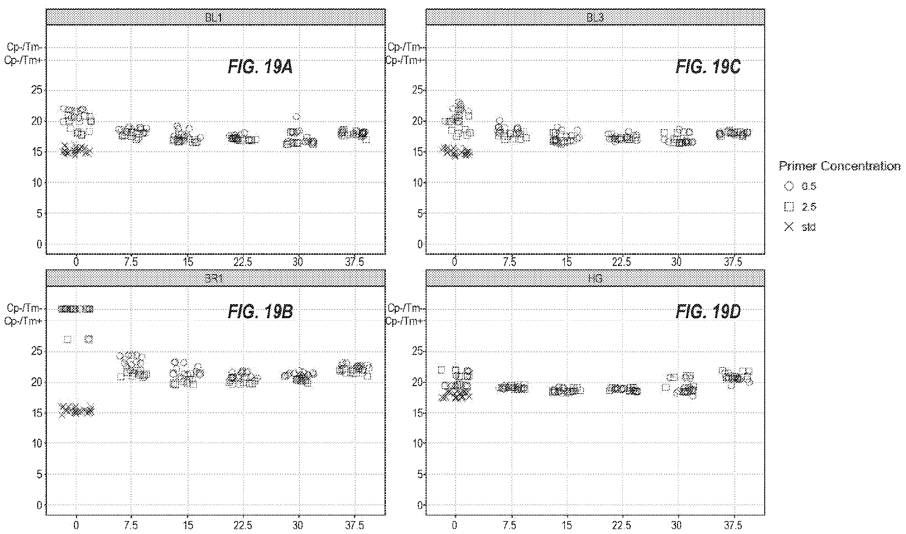

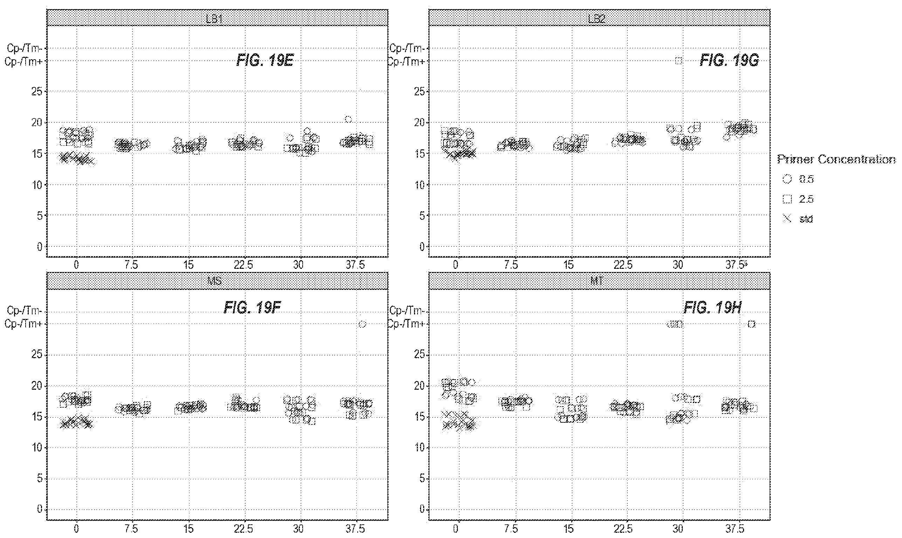

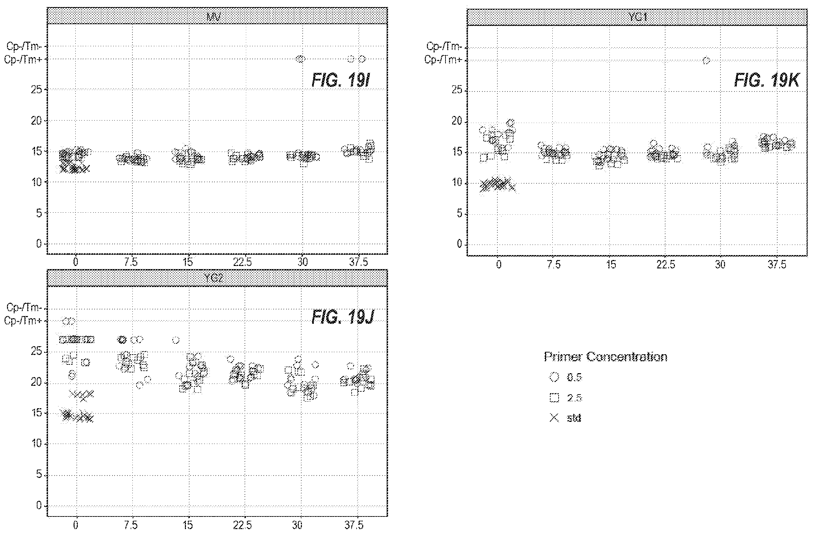

The effect of concentration of molecular crowders was studied using the test pouch of Example 11 and a mixture of Ficoll 70 and Ficoll 400. Eleven different targets were studied in two different pouch formats, one with each second-stage primer provided at the standard concentration of 0.5$\mu$M ($\bigcirc$) and one with each second-stage primer provided at a higher concentration of 2.5$\mu$M ($\square$). Each pouch with molecular crowders was cycled at the faster cycle times described in Example 11. Since synthetic templates were used, the sample prep steps in a standard FilmArray protocol were omitted. These data are displayed in Figs. 19a-19k, with the total concentration of the Ficol mixture indicated on the X-axis. Without molecular crowders present ("0" on the X-axis), all of the assays had a Cp greater than the Cp obtained with the standard primer concentration and the standard cycling times ($\times$). As expected from the work above, many of these assays have improved Cps when the higher concentration of primers are used, although some of the more robust assays are not rate limited by their primer concentration, and therefore showed no primer concentration effect. However, all of the assays showed improvement with 7.5% total concentration of molecular crowders, with plateaus for most assays at 15-30% molecular crowders. While 7.5% molecular crowders is the lowest amount tested, it is expected that at least 3% molecular crowders will have a positive effect on many assays, with most assays showing improvement at 5%. It is also expected that as cycle times decrease below 10 seconds per cycle, and more particularly below 5 seconds per cycle, Cp will be improved (decreased) in the presence of higher concentrations of primers and polymerase, although the amounts of polymerase and primers needed will be reduced from that discussed above due to the localized concentration effect of the molecular crowders. Certain assays, such as BL3 in figure 19b, seem to be rate limited by second stage primer concentration. There is a significant difference between performance of the $0.5\mu$M primer reactions and the $2.5\mu$M primer reactions when this assay is run at the faster cycling time without molecular crowders present. However, once molecular crowders are added, the $0.5\mu$M primer reactions are as efficient as the $2.5\mu$M reactions, indicating that the molecular crowders are able to act specifically on the primers in the second stage PCR. It is understood that other primer concentrations may be desired, and primer concentrations between $0.5\mu$M and $2.5\mu$M, may be desired, illustratively $1.0\mu$M, $1.5\mu$M, or $2.0\mu$M of each primer.

EXAMPLE 13

Without being bound by theory, it is expected that molecular crowders increase PCR efficiency in several ways. One way is to increase localized concentration such that formation of an initiation complex of a template, a primer, and a polymerase is favored. Another way is to increase local polymerase formation such that when a polymerase falls off during extension (as often happens with longer amplicons), polymerase binding is favored. The first example mechanism involves formation of a ternary complex and the components that occupy the smallest volume are the primers (MW in the range of 6.5 kDa to 10 kDa). In the second example mechanism, a binary complex is formed between the DNA polymerase (~66 kDa) and a partially double stranded amplicon. Other mechanisms are possible. Given these multiple mechanisms of action, one might expect a combination of different molecular crowders to provide different results, given differential diffusion and interaction of the components with various molecular weight crowders.

To test whether different combinations of molecular crowders have different effects, the following mixtures of Ficoll 70 and Ficoll 400 were tested:

2% Ficoll 70 / 30% Ficoll 400
24% Ficoll 70 / 0% Ficoll 400
18% Ficoll 70 / 0% Ficoll 400
28% Ficoll 70 / 28% Ficoll 400
5% Ficoll 70 / 5% Ficoll 400
21% Ficoll 70 / 6% Ficoll 400

All Ficoll amounts are provided in final w/v percentages. Test pouches 510, each using one of these Ficoll mixtures, were run using the fast protocol described in Example 11. Each mixture was run in three pouches.

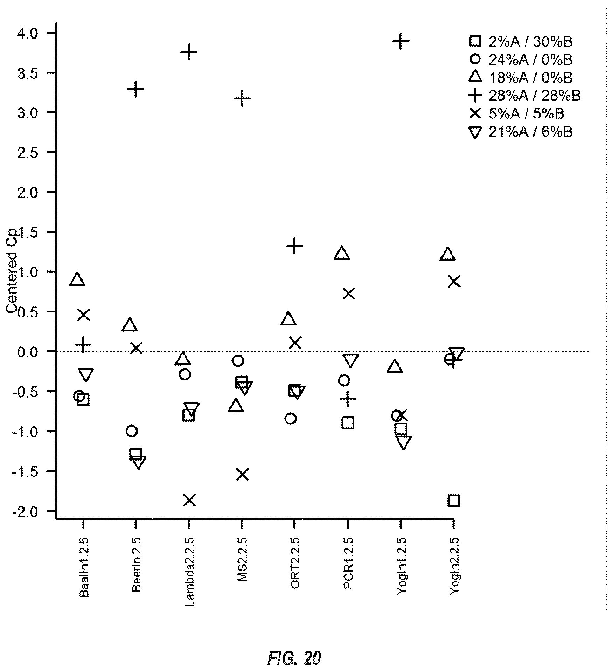

As can be seen in Fig. 20, the 28% Ficoll 70 / 28% Ficoll 400 mixture (+) had significantly delayed Cps compared to the other mixtures for many of the assays shown. This mixture had the highest overall percentage of molecular crowders. Without being bound to theory, it is believed that the concentration of crowders was too high, thereby retarding diffusion of the reactants. However, there was little difference in Cp between the other Ficoll mixtures, although results were far less consistent with the 5%/5% ($\times$) mixture and the 18%/0% mixture ($\Delta$). Thus, it is believed that there is a fairly wide range of concentrations and molecular weights that are effective in increasing localized concentrations without significantly limiting diffusion of the reactants. Based on the results in Examples 12-13, 7.5% molecular crowders is sufficient (although smaller amounts may be useful), 32% total w/v molecular crowders is effective, but 56% total w/v of Ficoll mixtures may inhibit effective diffusion. However, it is understood that other concentrations of other molecular crowders may be acceptable. This experiment also showed that the total concentration of crowders may be more important than the specific ratio of the two molecular weight components.

EXAMPLE 14

In this example, a prototype biothreat panel was used, where the panel includes assays for 30 targets, plus first-stage and second-stage controls. The sample injected into pouch 510 included a mixture of synthetic templates for each of the 30 targets, and the control templates are provided within pouch 510, so all assays should show amplification in second-stage PCR. The run protocol included a shortened sample prep, with 2 min of bead beating, followed by a reverse transcription step at 57°C for 60 sec. First-stage amplification and second-stage amplification were performed under the conditions described in Example 11. The following three Ficoll mixtures were used:

6% Ficoll 70 / 21% Ficoll 400 (+)
2% Ficoll 70 / 30% Ficoll 400 ($\times$)
21% Ficoll 70 / 6% Ficoll 400 ($\Delta$)

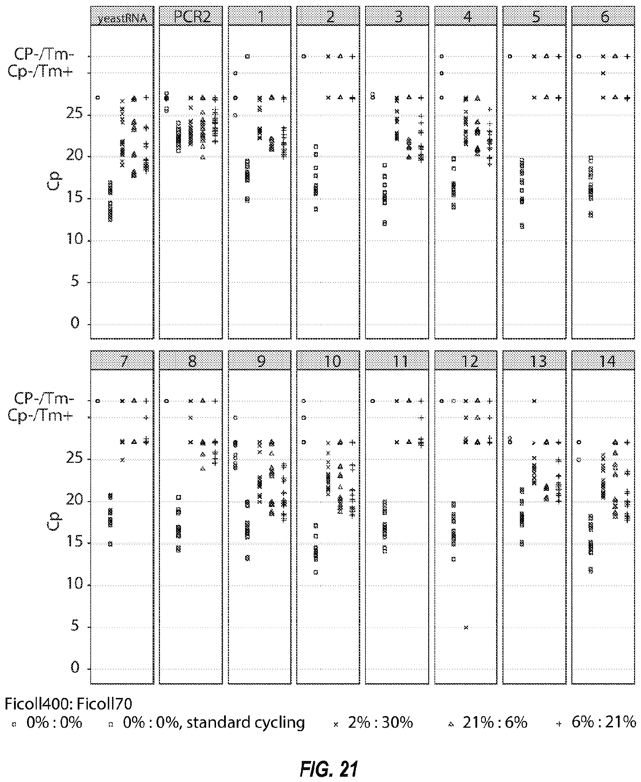

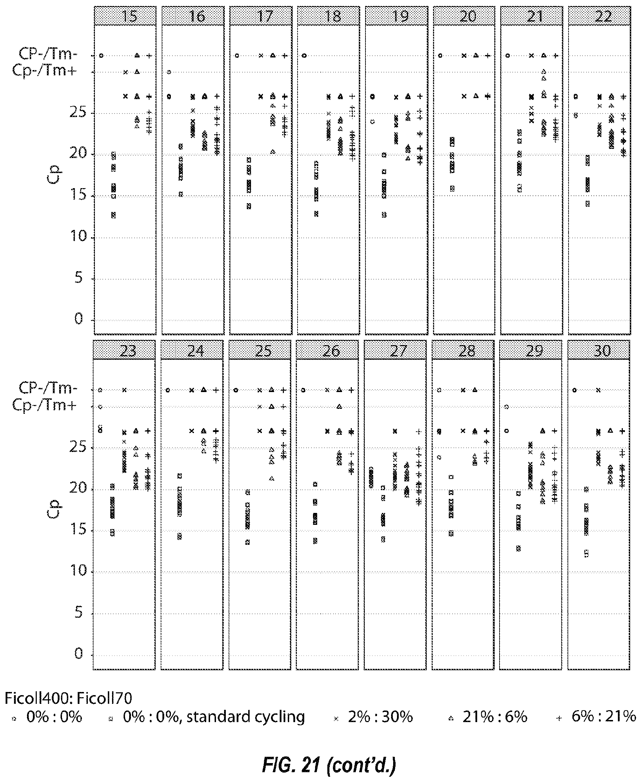

As seen in Fig. 21, at the standard cycling conditions, most replicates of all of the assays had Cps in the 13-22 cycle range, although assay 1 (a second-stage control) had later Cp values. It is understood that these assays were optimized for this slower protocol, with a number of the assays designed to amplify target variants with mismatches near the 3'-ends of some of the primers. It is expected that "weaker" assays, e.g. those with mismatches near the 3'-end of at least one of the primers and requiring more time for formation of the initiation complex, are not optimized well for the annealing and denaturation temperatures, or generate lengthy amplicons and may provide more opportunities for the polymerase to disassociate, would perform more poorly at faster cycling speeds. As above, when cycling times are decreased by 19 s and everything else is held constant, many of the assays failed. Only assays 7 and 28 continued to amplify well, and even those assays had Cps that were delayed by about 5 cycles. When molecular crowders are used with fast cycling, many of the assays, once again, showed positive results with improved performance over their counterparts without molecular crowders, but with a delay of about 5 cycles as compared to the standard cycling conditions. Weaker assays 22-25, and 29-30 and the yeast process control did not recover even with molecular crowders. The three different ratios of Ficoll 70 and Ficoll 400 all showed similar results, thus suggesting that the concentration of the molecular crowders is more important than the size of the molecular crowder molecules.

Assay optimization is often empirical. Bioinformatics tools can produce tens to hundreds of different oligonucleotide sequences as candidate primers. From these candidate primers, tens of combinations of primers can be tested under any given cycling condition and the combination giving the lowest Cp and the greatest sensitivity in the full assay may become the primer set of choice. Thus, it should be possible to reoptimize assays that are efficient under slow cycling conditons to ones that are efficient under fast cycling conditions in the presence of molecular crowders.

EXAMPLE 15

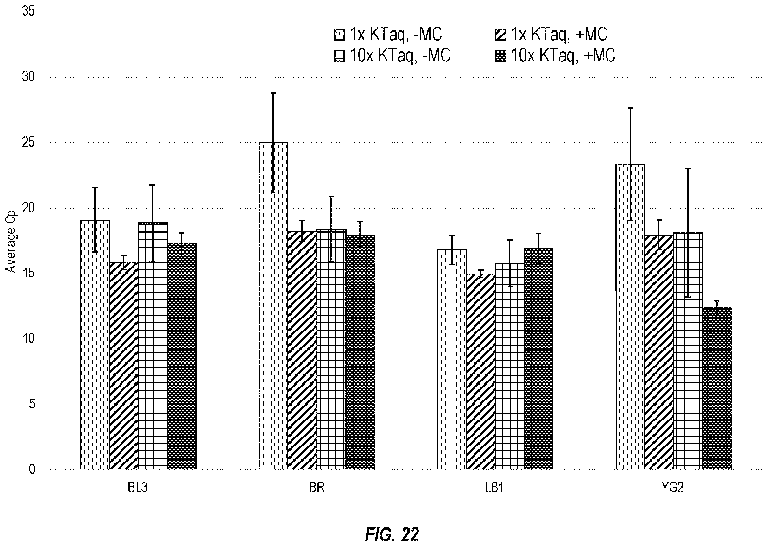

The effect of increased polymerase concentration in combination with molecular crowders was studied. The test pouch of Example 11 was used at the faster cycling speeds. All pouches were injected with a mixture of targets. In examples using molecular crowders, the mixture of Example 11 was injected as described above. Pouches with and without molecular crowders were tested with standard 1x concentration of KlenTaq (0.2U/ μL) and a 10x concentration of KlenTaq in both first-stage amplification and second-stage amplification. Fig. 22 shows the results for several assays. BR and YG2 assays performed as expected, with either molecular crowders or increased polymerase providing a substantial shift in Cp. All assays performed better in the presence of molecular crowders, but many assays were unaffected by the additional polymerase, perhaps because the polymerase was not rate limiting for these assays. The key process identified in this experiment was demonstrated by the BR and YG2 assays. For those assays, polymerase concentration may be the rate limiting step, as shown by crossing point recovery when additional polymerase is spiked in. This example shows that molecular crowders are effective in boosting the performance of reactions containing 1x polymerase and rescuing these assays. As noted in Example 11, the second-stage amplification is performed at 26 seconds/cycle. It is expected that many assays will benefit from both molecular crowders and enhanced primer/polymerase concentrations with faster cycle times. It is expected that with cycle times of 10 seconds or less, acceptable PCR yield will be obtained with 2 to 10 times standard KlenTaq concentrations and 2 to 10 times standard primer concentrations.

It is believed that the addition of molecular crowders to various commercial assays will result in good amplification when protocols are altered to include faster cycling times. A method including the addition of one or more of the molecular crowders in the illustrative mixtures to commercial assays, followed by amplification with cycle times reduced by 5 to 50%, will yield performance (sensitivity and specificity of the assays) equivalent to that achieved with the slow cycling times. In one illustrative embodiment, other than the addition of molecular crowders, no changes are made to the chemistry of the assay to permit faster cycling times. The addition of molecular crowders may allow assays that were optimized for slower PCR instruments to be used on fast PCR instruments, without change to the manufactured product.

Additionally, it is believed that molecular crowders can be selectively and strategically applied to a single manufactured commercial assay such that, the assay can be used in one of two ways: 1) optimized for speed with a certain combination of chemistry, molecular crowders, and faster cycling times using faster cycling instrumentation, or 2) without molecular crowders and compatible with slower cycling instrumentation. The addition by the user of the molecular crowders in hydration buffer (for dried reagents) or in dilution buffer (for wet reagents) provides flexibility in instrumentation used for a single assay.

REFERENCES

1. Wittwer CT, Reed GB, Ririe KM. Rapid cycle DNA amplification. In: Mullis IK, Ferre F, Gibbs R, eds. The polymerase chain reaction, Vol. Deerfield Beach, FL: 174-181, 1994.

2. Wittwer CT, Fillmore GC, Hillyard DR. Automated polymerase chain reaction in capillary tubes with hot air. Nucleic Acids Res 1989;17:43 53-7.

3. Wittwer CT, Fillmore GC, Garling DJ. Minimizing the time required for DNA amplification by efficient heat transfer to small samples. Anal Biochem 1990;186:328-31.

4. Wittwer CT, Garling DJ. Rapid cycle DNA amplification: time and temperature optimization. Biotechniques 1991;10:76-83.

5. Wittwer CT, Marshall BC, Reed GH, Cherry JL. Rapid cycle allele-specific amplification: studies with the cystic fibrosis delta F508 locus. Clin Chem 1993;39:804-9.

6. Schoder D, Schmalwieser A, Schauberger G, Hoorfar J, Kuhn M, Wagner M. Novel approach for assessing performance of PCR cyclers used for diagnostic testing. J Clin Microbiol 2005;43:2724-8.

7. Herrmann MG, Durischi JD, Wittwer CT, Voelkerding KV. Expanded instrument comparison of amplicon DNA melting analysis for mutation scanning and genotyping. Clin Chem 2007;53:1544-8.

8. Herrmann MG, Durtschi JD, Bromley LK, Wittwer CT, Voelkerding KV. Amplicon DNA melting analysis for mutation scanning and genotyping: cross-platform comparison of instruments and dyes. Clin Chem 2006;52:494-503.

9. Raja S, El-Hefnawy T, Kelly LA, Chestney ML, Luketich JD, Godfrey TE. Temperature-controlled primer limit for multiplexing of rapid, quantitative reverse transcription-PCR assays: application to intraoperative cancer diagnostics. Clin Chem 2002;48:1329-37.

10. Wittwer CT, Ririe KM, Andrew RV, David DA, Gundry RA, Balis UJ. The LightCycler: a microvolume multisample fluorimeter with rapid temperature control. Biotechniques 1997;22:176-81.

11. Wittwer CT, Ririe KM, Rasmussen RP. Fluorescence monitoring of rapid cycle PCR for quantification. In: Ferre F, ed. Gene Quantification, New York: Birkhauser, 1998:129-44.

12. Elenitoba-Johnson O, David D, Crews N, Wittwer CT. Plastic vs glass capillaries for rapid-cycle PCR. Biotechniques 2008;44:487-8,490,492.

13. Roper MG, Easley CJ, Landers JP. Advances in polymerase chain reaction on microfluidic chips. Anal Chem 2005;77:3887-93.

14. Zhang C, Xing D. Miniaturized PCR chips for nucleic acid amplification and analysis: latest advances and future trends. Nucleic Acids Res 2007;35:4223-37.

15. Cheng J, Shoffner MA, Hvichia GE, Kricka LJ, Wilding P. Chip PCR. II. Investigation of different PCR amplification systems in microfabricated silicon-glass chips. Nucleic Acids Res 1996;24:380-5.

16. Woolley AT, Hadley D, Landre P, deMello AJ, Mathies RA, Northrup MA. Functional integration of PCR amplification and capillary electrophoresis in a microfabricated DNA analysis device. Anal Chem 1996;68:4081-6.

17. Neuzil P, Zhang C, Pipper J, Oh S, Zhuo L. Ultra fast miniaturized real-time PCR: 40 cycles in less than six minutes. Nucleic Acids Res 2006;34:e77.

18. Oda RP, Strausbauch MA, Huhmer AF, Borson N, Jurrens SR, Craighead J, et al. Infrared-mediated thermocycling for ultrafast polymerase chain reaction amplification of DNA. Anal Chem 1998;70:4361-8.

19. Roper MG, Easley CJ, Legendre LA, Humphrey JA, Landers JP. Infrared temperature control system for a completely noncontact polymerase chain reaction in microfluidic chips. Anal Chem 2007;79:1294-300.

20. Friedman NA, Meldrum DR. Capillary tube resistive thermal cycling. Anal Chem 1998;70:2997-3002.

21. Heap DM, Herrmann MG, Wittwer CT. PCR amplification using electrolytic resistance for heating and temperature monitoring. Biotechniques 2000;29:1006-12.

22. Kopp MU, Mello AJ, Manz A. Chemical amplification: continuous-flow PCR on a chip. Science 1998;280:1046-8.

23. Hashimoto M, Chen PC, Mitchell MW, Nikitopoulos DE, Soper SA, Murphy MC. Rapid PCR in a continuous flow device. Lab Chip 2004;4:638-45.

24. Crews N, Wittwer C, Gale B. Continuous-flow thermal gradient PCR. Biomed Microdevices 2008;10;187-95.

25. Chiou JT, Matsudaira PT, Ehrlich DJ. Thirty-cycle temperature optimization of a closed-cycle capillary PCR machine. Biotechniques 2002;33:557-8, 60, 62.

26. Frey O, Bonneick S, Hierlemann A, Lichtenberg J. Autonomous microfluidic multichannel chip for real-time PCR with integrated liquid handling. Biomed Microdevices 2007;9:711-8.

27. Chen J, Wabuyele M, Chen H, Patterson D, Hupert M, Shadpour H, et al. Electrokinetically synchronized polymerase chain reaction microchip fabricated in polycarbonate. Anal Chem 2005;77:658-66.

28. Sun Y, Kwok YC, Nguyen NT. A circular ferrofluid driven microchip for rapid polymerase chain reaction. Lab Chip 2007;7:1012-7.

29. Agrawal N, Hassan YA, Ugaz VM. A pocket-sized convective PCR thermocycler. Angew Chem Int Ed Engl 2007;46:4316-9.

30. Zhang C, Xu J, Ma W, Zheng W. PCR microfluidic devices for DNA amplification. Biotechnol Adv 2006;24:243-84.

31. Wheeler EK, Benett W, Stratton P, Richards J, Chen A, Christian A, et al. Convectively driven polymerase chain reaction thermal cycler. Anal Chem 2004;76:4011-6.

32. Belgrader P, Benett W, Hadley D, Long G, Mariella R, Jr., Milanovich F, et al. Rapid pathogen detection using a microchip PCR array instrument. Clin Chem 1998;44:2191-4.

33. Terazona H, Takei, H, Hattori A, Yasuda K. Development of a high-speed real-time polymerase chain reaction system using a circulating water-based rapid heat exchange. Jap J Appl Phys 2010;49:06GM05.

34. Wheeler EK, Hara CA, Frank J, Deotte J, Hall SB, Benett W, Spadaccini C, Beer NR. Under-three minute PCR:

Probing the limits of fast amplification. Analyst 2011;136(16):3707-12.

35. Fuchiwaki Y, Nagai H, Saito M, Tamiya E. Ultra-rapid flow-through polymerase chain reaction microfluidics using vapor pressure. Biosens Bioelect 2011;27:88-94.

36. Maltezos G, Johnston M, Taganov K, Srichantaratsamee C, Gorman J, Baltimore D, Chantratita W and Scherer A, Exploring the limits of ultrafast polymerase chain reaction using liquid for thermal heat exchange: A proof of principle Appl. Phys. Lett., 2010; 97: 264101.

37. Wilhelm J, Hahn M, Pingoud A. Influence of DNA target melting behavior on real-time PCR quantification. Clin Chem 2000;46:1738-43.

38. Zuna J, Muzikova K, Madzo J, Krejci O, Trka J. Temperature non-homogeneity in rapid airflow-based cycler significantly affects real-time PCR. Biotechniques 2002;33:508, 10, 12.

39. von Kanel T, Adolf F, Schneider M, Sanz J, Gallati S. Sample number and denaturation time are crucial for the accuracy of capillary-based LightCyclers. Clin Chem 2007;53:1392-4.

40. Wittwer CT, Herrmann MG. Rapid thermal cycling and PCR kinetics. In: Innis M, Gelfand D, Sninsky J, eds. PCR Methods Manual, Vol. San Diego: Academic Press, 1999:211-29.

41. Wittwer CT, Reed GH, Gundry CN, Vandersteen JG, Pryor RJ. High-resolution genotyping by amplicon melting analysis using LCGreen. Clin Chem 2003;49:853-60.

42. von Ahsen N, Wittwer CT, Schutz E. Oligonucleotide melting temperatures under PCR conditions: nearest-neighbor corrections for Mg(2+), deoxynucleotide triphosphate, and dimethyl sulfoxide concentrations with comparison to alternative empirical formulas. Clin Chem 2001;47:1956-61.

43. Ririe KM, Rasmussen RP, Wittwer CT. Product differentiation by analysis of DNA melting curves during the polymerase chain reaction. Anal Biochem 1997;245:154-60.

44. Wittwer CT, Herrmann MG, Moss AA, Rasmussen RP. Continuous fluorescence monitoring of rapid cycle DNA amplification. Biotechniques 1997;22:130-1, 4-8.

45. Weis JH, Tan SS, Martin BK, Wittwer CT. Detection of rare mRNAs via quantitative RT-PCR. Trends Genet 1992;8:263-4.

46. Brown RA, Lay MJ, Wittwer CT. Rapid cycle amplification for construction of competitive templates. In: Horton RM, Tait RC, eds. Genetic Engineering with PCR, Vol. Norfolk: Horizon Scientific Press, 1998:57-70.

47. Ausubel et al., Current Protocols in Molecular Biology, John Wiley & Sons, New York, NY, 1998

48. Whitney SE, "Analysis of rapid thermocycling for the polymerase chain reaction," Ph.D. thesis, University of Nebraska, 2004.

49. Lawyer FC, Stoffel S, Saiki RK, Chang SY, Landre PA, Abramson RD, Gelfand DH. High-level expression, purification and enzymatic characterization of full-length Thermus aquaticus DNA polymerase and a truncated form deficient of 5' to 3' exonuclease activity. PCR Meth Appl. 1993;2:275-287.

50. Innis MA, Myamo KB, Gelfand DH, Brow MAD. DNA sequencing with Thermus aquaticus DNA polymerase and direct sequencing of polymerase chain reaction-amplified DNA. Proc. Natl. Acad. Sci USA 1988;85:9436-40.

51. Terazono H, Hattori A, Takei H, Takeda K, Yasuda K. Development of 1480 nm photothermal high-speed real-time polymerase chain reaction system for rapid nucleotide recognition. Jpn J Appl Phys. 2008;47:5212-6.

52. Wittwer CT, Rasmussen RP, Ririe KM. Rapid PCR and melting curve analysis. In: The PCR Revolution: Basic Technologies and Applications, Bustin SA, ed. Cambridge Univ Press, New York, 48-69, 2010.

53. Fuchiwaki Y, Saito M, Wakida S, Tamiya E, Nagai H. A practical liquid plug flow-through polymerase chain-reaction system based on a heat-resistant resin chip. Anal Sci. 2011;27:225-30.

54. Kim H, Dixit S, Green CJ, Faris GW. Nanodroplet real-time PCR system with laser assisted heating. Optics Express 2009;17:218-27.

55. Obeid PJ, Christopoulos TK, Crabtree HJ, Backhouse CJ. Microfabricated device for DNA and RNA amplification by continuous-flow polymerase chain reaction and reverse transcription-polymerase chain reaction with cycle number selection. Anal Chem 2003;75:288-95.

56. Giordano BC, Ferrance J, Swedberg S, Huhmer AFR, Landers JP. Polymerase chain reaction in polymeric microchips: DNA amplification in less than 240 seconds. Anal Biochem 2001;291:124-132.

57. Pal, D., Venkataraman, V., Mohan, K. N., Chandra, H. S., & Natarajan, V. (2004). A power-efficient thermocycler based on induction heating for DNA amplification by polymerase chain reaction. Review of Scientific Instruments, 75(9), 2880 2883.

58. Yuanzhi Lao, F. E. H., Tay, F. E. H., Guolin Xu, Hartono, D., & Lee, Y. Y. (2003). A Non-Contact Micro Thermocycling Chip for Polymerase Chain Reactions. International Journal of Computational Engineering Science, 4(3), 651-654.

59. Shen F ,Sun, B, Kreutz JE. Davydova EK, Du W, Reddy PL, Joseph LJ, and. Ismagilov RF. Multiplexed Quantification of Nucleic Acids with Large Dynamic

60. Range Using Multivolume Digital RT-PCR on a Rotational SlipChip Tested with HIV and Hepatitis C Viral Load. *J. Am. Chem. Soc.* 2011, 133, 17705-17712.

61. Bustin, S. et al., Variability of the reverse transcription step: practical implications, Clinical Chemistry, 61, 201-12, 2015.

62. Montgomery JL and Wittwer CT, Influence of PCR reagents on DNA polymerase extension rates measured on real-time PCR instruments, Clin Chem 60:334-340 (2014).

63. Mifatovic-Rustempasic S et al., Sensitive and specific quantitative detection of rotavirus A by one-step real-time reverse transcription-PCR assay without antecedent double-stranded-RNA denaturation, J Clin Microbiol (2013), 51, 3047-54.

64. Carninci P et al., Thermostabilization and thermoactivation of thermolabele enzymes by trehalose and its application for the synthesis of full length cDNA, PNAS, 1998:95:520-4 and AN Spiess et al., Therhalose is a potent enhancer: Lowering of DNA melting temperature and thermal stabilizationof Taq polymerase by the disaccharide trihalose, Clin Chem, 2004; 50: 1256-9.

65. Gerard GF, D'Alessio JM. Chapter 6 (73-93) From: Methods in Molecular Biology, Vol. 16, Enzymes of Molecular Biology, Edited by MM Burell, 1993, Humana Press, Inc., Totowa, NJ.

66. Tong Y et al., Multiple strategies to improve sensitivity, speed and robustness of isothermal nucleic acid amplification for rapid pathogen detection, BMC Biotechnology (2011) 11:50.

67. Sasaki Y et al., Effect of molecular crowding on DNA polymerase Activity, Biotechnol J, 2006:1:440-6.

**Claims**

1. A method for amplifying a target nucleic acid from a biological sample in a container, the container comprising:

   a flexible material defining a plurality of fluidly connected reaction zones fluidly connected by channels, the fluidly connected reaction zones including at least a first-stage PCR reaction zone;
   the container comprising an amplification mixture for first-stage PCR in the first-stage PCR reaction zone, wherein the amplification mixture includes a thermostable polymerase, primers configured for amplification of a target nucleic acid, and a molecular crowder for increasing the effective concentration of primers and polymerase, the method comprising the steps of:

   introducing the biological sample into the first-stage PCR reaction zone of the container;
   mixing the thermostable polymerase, the primers configured for amplification of the target nucleic acid, and the molecular crowder with the biological sample to create the amplification mixture, wherein the molecular crowder is provided in an amount that is at least 3% w/v of the amplification mixture; and
   amplifying the target nucleic acid by polymerase chain reaction by thermally cycling the amplification mixture through a plurality of at least two-step amplification cycles comprising at least a denaturation temperature and an annealing temperature, wherein each cycle is completed in a cycle time less than 40 seconds per cycle;
   wherein the molecular crowder is a Ficoll or a mixture of Ficolls.

2. The method of claim 1, wherein the molecular crowder is provided at an amount between 5% and 50% w/v of the amplification mixture.

3. The method of claim 1 or 2, wherein the molecular crowder is provided in an amount that is at least 7.5% w/v of the amplification mixture.

4. The method of claim 1, wherein the molecular crowder is a mixture of a first Ficoll having a first molecular weight and a second Ficoll having a second molecular weight that is different from the first molecular weight.

5. The method of any one of claims 1 to 4, wherein the polymerase is provided at an amount of not more than 2.5μM in the amplification mixture, preferably at an amount of not more than 1.0μM in the amplification mixture, more preferably at an amount of not more than 0.5μM in the amplification mixture.

6. The method of any one of claims 1 to 5, wherein the denaturation temperature exceeds 100°C for at least one cycle.

7. The method of claim 1, wherein:

   each cycle is completed in a cycle time less than 30 seconds per cycle,
   the molecular crowder is provided in an amount between 5% and 50% w/v of the amplification mixture,
   the primers are each provided at a concentration of at least 0.5 μM in the amplification mixture, and

the polymerase is provided at a concentration of at least 0.4U/μL of the amplification mixture.

8.  The method of any one of claims 1 to 7 wherein the cycle time is no more than 10 seconds.

**Patentansprüche**

1.  Verfahren zum Amplifizieren einer Zielnukleinsäure aus einer biologischen Probe in einem Behälter, wobei der Behälter Folgendes umfasst:

    ein nachgiebiges Material, das eine Vielzahl von fluidisch verbundenen Reaktionsbereichen definiert, die fluidisch durch Kanäle verbunden sind, wobei die fluidisch verbundenen Reaktionsbereiche mindestens einen PRC-Reaktionsbereich der ersten Stufe einschließen;
    wobei der Behälter eine Amplifikationsmischung für die PCR der ersten Stufe im PRC-Reaktionsbereich der ersten Stufe umfasst, wobei die Amplifikationsmischung eine thermostabile Polymerase, Primer, die zur Amplifikation einer Zielnukleinsäure konfiguriert sind, und einen molekularen Crowder zum Erhöhen der wirksamen Konzentration von Primern und Polymerase einschließt,
    wobei das Verfahren die folgenden Schritte umfasst:

    Einführen der biologischen Probe in den PCR-Reaktionsbereich der ersten Stufe des Behälters;
    Mischen der thermostabilen Polymerase, der Primer, die zur Amplifikation der Zielnukleinsäure konfiguriert sind, und des molekularen Crowders mit der biologischen Probe, um die Amplifikationsmischung zu erzeugen, wobei der molekulare Crowder in einer Menge bereitgestellt wird, die mindestens 3 Gew.-% der Amplifikationsmischung beträgt; und
    Amplifizieren der Zielnukleinsäure durch Polymerasekettenreaktion durch thermisches Zirkulieren der Amplifikationsmischung durch eine Vielzahl von mindestens zweistufigen Amplifikationszyklen, die mindestens eine Denaturierungstemperatur und eine Annealing-Temperatur umfassen, wobei jeder Zyklus in einer Zykluszeit von weniger als 40 Sekunden pro Zyklus abgeschlossen wird;
    wobei der molekulare Crowder ein Ficoll oder eine Mischung aus Ficolls ist.

2.  Verfahren nach Anspruch 1, wobei der molekulare Crowder in einer Menge zwischen 5 Gew.-% und 50 Gew.-% der Amplifikationsmischung bereitgestellt wird.

3.  Verfahren nach Anspruch 1 oder 2, wobei der molekulare Crowder in einer Menge bereitgestellt wird, die mindestens 7,5 Gew.-% der Amplifikationsmischung beträgt.

4.  Verfahren nach Anspruch 1, wobei der molekulare Crowder eine Mischung aus einem ersten Ficoll, das ein erstes Molekulargewicht aufweist, und einem zweiten Ficoll ist, das ein zweites Molekulargewicht aufweist, das sich vom ersten Molekulargewicht unterscheidet.

5.  Verfahren nach einem der Ansprüche 1 bis 4, wobei die Polymerase in einer Menge von nicht mehr als 2,5 μM in der Amplifikationsmischung, vorzugsweise in einer Menge von nicht mehr als 1,0 μM in der Amplifikationsmischung, bevorzugter in einer Menge von nicht mehr als 0,5 μM in der Amplifikationsmischung bereitgestellt wird.

6.  Verfahren nach einem der Ansprüche 1 bis 5, wobei die Denaturierungstemperatur für mindestens einen Zyklus 100 °C übersteigt.

7.  Verfahren nach Anspruch 1, wobei:

    jeder Zyklus in einer Zykluszeit von weniger als 30 Sekunden pro Zyklus abgeschlossen wird;
    der molekulare Crowder in einer Menge zwischen 5 Gew.-% und 50 Gew.-% der Amplifikationsmischung bereitgestellt wird,
    die Primer jeweils bei einer Konzentration von mindestens 0,5 μM in der Amplifikationsmischung bereitgestellt werden, und
    die Polymerase bei einer Konzentration von mindestens 0,4 U/μL in der Amplifikationsmischung bereitgestellt wird.

8.  Verfahren nach einem der Ansprüche 1 bis 7, wobei die Zykluszeit nicht mehr als 10 Sekunden beträgt.

**Revendications**

1. Procédé pour amplifier un acide nucléique cible provenant d'un échantillon biologique dans un récipient, le récipient comprenant :

   un matériau flexible définissant une pluralité de zones de réaction fluidiquement reliées qui sont fluidiquement reliées par des canaux, les zones de réaction fluidiquement reliées incluant au moins une zone de réaction à PCR de premier étage ;
   le récipient comprenant un mélange d'amplification pour PCR de premier étage dans la zone de réaction à PCR de premier étage, dans lequel le mélange d'amplification inclut une polymérase thermostable, des amorces configurées pour amplification d'un acide nucléique cible et un amasseur moléculaire pour augmenter la concentration efficace d'amorces et de polymérase,
   le procédé comprenant les étapes consistant à :

   introduire l'échantillon biologique dans la zone de réaction à PCR de premier étage du récipient ;
   mélanger la polymérase thermostable, les amorces configurées pour amplification de l'acide nucléique cible et l'amasseur moléculaire avec l'échantillon biologique pour créer le mélange d'amplification, dans lequel l'amasseur moléculaire est prévu en une quantité qui fait au moins 3 % en poids/volume du mélange d'amplification ; et
   amplifier l'acide nucléique cible par réaction en chaîne par polymérase par le cyclage thermique du mélange d'amplification sur une pluralité de cycles d'amplification à au moins deux étapes comprenant au moins une température de dénaturation et une température de recuit, dans lequel chaque cycle est achevé dans un temps de cycle de moins de 40 secondes par cycle ;
   dans lequel l'amasseur moléculaire est un Ficoll ou un mélange de Ficoll.

2. Procédé selon la revendication 1, dans lequel l'amasseur moléculaire est prévu en une quantité faisant entre 5 % et 50 % en poids/volume du mélange d'amplification.

3. Procédé selon la revendication 1 ou la revendication 2, dans lequel l'amasseur moléculaire est prévu en une quantité qui fait au moins 7,5 % en poids/volume du mélange d'amplification.

4. Procédé selon la revendication 1, dans lequel l'amasseur moléculaire est un mélange d'un premier Ficoll présentant un premier poids moléculaire et d'un second Ficoll présentant un second poids moléculaire qui est différent du premier poids moléculaire.

5. Procédé selon l'une quelconque des revendications 1 à 4, dans lequel la polymérase est prévue en une quantité de pas plus de 2,5 $\mu$M dans le mélange d'amplification, préférablement en une quantité de pas plus de 1,0 $\mu$M dans le mélange d'amplification, plus préférablement en une quantité de pas plus de 0,5 $\mu$M dans le mélange d'amplification.

6. Procédé selon l'une quelconque des revendications 1 à 5, dans lequel la température de dénaturation dépasse 100 °C pendant au moins un cycle.

7. Procédé selon la revendication 1, dans lequel :

   chaque cycle est achevé dans un temps de cycle de moins de 30 secondes par cycle,
   l'amasseur moléculaire est prévu en une quantité faisant entre 5 % et 50 % en poids/volume du mélange d'amplification,
   les amorces sont chacune prévues à une concentration d'au moins 0,5 $\mu$M dans le mélange d'amplification, et
   la polymérase est prévue à une concentration d'au moins 0,4 U/$\mu$L du mélange d'amplification.

8. Procédé selon l'une quelconque des revendications 1 à 7 dans lequel le temps de cycle ne fait pas plus de 10 secondes.

FIG. 1A

FIG. 1B

**FIG. 1C**

EP 3 562 592 B1

**FIG. 1D**

*FIG. 2A*

*FIG. 2B*

*FIG. 2C*

*FIG. 3A*

FIG. 3B

*FIG. 3C*

FIG. 4A

FIG. 4B

*FIG. 4C*

FIG. 5A

**FIG. 5B**

20 μMPrimer 4   μM KT POL
10 μMPrimer 4   μM KT POL
5 μMPrimer 4   μM KT POL
2.5 μMPrimer 4   μM KT POL

**FIG. 5C**

FIG. 5D

**FIG. 6A**

*FIG. 6B*

Temperature (°C)

Time (sec)

FIG. 7A

FIG. 7B

FIG. 8A

FIG. 8B

226bp 2 sec extension
226bp 1 sec extension
226bp 2 sec extension NTC
226bp 1 sec extension NTC

Fluorescence

Cycle #

FIG. 8C

**FIG. 8D**

**FIG. 9A**

$y = -3.538x + 39.231$
$R^2 = 0.9922$

**FIG. 9B**

FIG. 9C

$y = -3.64x + 38.236$
$R^2 = 0.9909$

Log $_{10}$ (initial template copies)

FIG. 9D

FIG. 10A

FIG. 10B

FIG. 10C

*FIG. 11A*

*FIG. 11B*

FIG. 11C

FIG. 11D

**500-bp**

FIG. 11E

**Mg++ Optimization for 0.42 s/cycle PCR**

FIG. 12A

*Mg++ Optimization for 0.42 s/cycle PCR Gel*

*FIG. 12B*

**PRKA 60 bp extension time optimization w/ 5 mM Mg++**

*Add 0.22 s/cycle for total cycle time*

Legend:
- PRKA 0.1 s
- PRKA 0.2 s
- PRKA 0.3 s
- PRKA 0.4 s

Temperature (°C)

**FIG. 13A**

*Extension Time Optimization w/ 5 mM Mg++ Gel*

*FIG. 13B*

*Target: AKAP10- 60 bp product*
*[Primer]: 20 uM*
*[Polymerase]: 8 uM*
*10 ng hgDNA/rxn*

*FIG. 14A*

*FIG. 14B*

**FIG. 15B**

**FIG. 15A**

**FIG. 16**

FIG. 17

FIG. 18

FIG. 19A

FIG. 19B

FIG. 19C

FIG. 19D

Primer Concentration
○ 0.5
□ 2.5
✕ std

EP 3 562 592 B1

FIG. 19E

FIG. 19G

FIG. 19F

FIG. 19H

Primer Concentration

○ 0.5
□ 2.5
× std

FIG. 19I

FIG. 19K

FIG. 19J

Primer Concentration

○ 0.5

□ 2.5

× std

EP 3 562 592 B1

FIG. 20

FIG. 21

Ficoll400: Ficoll70
○ 0% : 0%    ▫ 0% : 0%, standard cycling    × 2% : 30%    ▵ 21% : 6%    + 6% : 21%

*FIG. 21 (cont'd.)*

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6706617 B
- US 6210882 B
- US 20090275014 A1
- US 6472186 B
- WO 2008018839 A
- US 8895295 B
- US 8895295 A

- US 20150118715 A
- US 8394608 B
- US 62298311
- US 62330701 B
- US 62368095 B
- US 5436149 A
- WO 2015069743 A

### Non-patent literature cited in the description

- Rapid cycle DNA amplification.. **WITTWER CT** ; **REED GB** ; **RIRIE KM.** The polymerase chain reaction,. 1994, 174-181
- **WITTWER CT** ; **FILLMORE GC** ; **HILLYARD DR.** Automated polymerase chain reaction in capillary tubes with hot air.. *Nucleic Acids Res*, 1989, vol. 17, 4353-7
- **WITTWER CT** ; **FILLMORE GC** ; **GARLING DJ.** Minimizing the time required for DNA amplification by efficient heat transfer to small samples.. *Anal Biochem*, 1990, vol. 186, 328-31
- **WITTWER CT** ; **GARLING DJ.** Rapid cycle DNA amplification: time and temperature optimization.. *Biotechniques*, 1991, vol. 10, 76-83
- **WITTWER CT** ; **MARSHALL BC** ; **REED GH** ; **CHERRY JL.** Rapid cycle allele-specific amplification: studies with the cystic fibrosis delta F508 locus. *Clin Chem*, 1993, vol. 39, 804-9
- **SCHODER D** ; **SCHMALWIESER A** ; **SCHAUBERGER G** ; **HOORFAR J** ; **KUHN M** ; **WAGNER M.** Novel approach for assessing performance of PCR cyclers used for diagnostic testing. *J Clin Microbiol*, 2005, vol. 43, 2724-8
- **HERRMANN MG** ; **DURISCHI JD** ; **WITTWER CT** ; **VOELKERDING KV.** Expanded instrument comparison of amplicon DNA melting analysis for mutation scanning and genotyping.. *Clin Chem*, 2007, vol. 53, 1544-8
- **HERRMANN MG** ; **DURTSCHI JD** ; **BROMLEY LK** ; **WITTWER CT** ; **VOELKERDING KV.** Amplicon DNA melting analysis for mutation scanning and genotyping: cross-platform comparison of instruments and dyes.. *Clin Chem*, 2006, vol. 52, 494-503

- **RAJA S** ; **EL-HEFNAWY T** ; **KELLY LA** ; **CHESTNEY ML** ; **LUKETICH JD** ; **GODFREY TE.** Temperature-controlled primer limit for multiplexing of rapid, quantitative reverse transcription-PCR assays: application to intraoperative cancer diagnostics.. *Clin Chem*, 2002, vol. 48, 1329-37
- **WITTWER CT** ; **RIRIE KM** ; **ANDREW RV** ; **DAVID DA** ; **GUNDRY RA** ; **BALIS UJ.** The LightCycler: a microvolume multisample fluorimeter with rapid temperature control.. *Biotechniques*, 1997, vol. 22, 176-81
- Fluorescence monitoring of rapid cycle PCR for quantification. **WITTWER CT** ; **RIRIE KM** ; **RASMUSSEN RP.** Gene Quantification. Birkhauser, 1998, 129-44
- **ELENITOBA-JOHNSON O** ; **DAVID D** ; **CREWS N** ; **WITTWER CT.** Plastic vs glass capillaries for rapid-cycle PCR.. *Biotechniques*, 2008, vol. 44, 487-8, 490, 492
- **ROPER MG** ; **EASLEY CJ** ; **LANDERS JP.** Advances in polymerase chain reaction on microfluidic chips.. *Anal Chem*, 2005, vol. 77, 3887-93
- **ZHANG C** ; **XING D.** Miniaturized PCR chips for nucleic acid amplification and analysis: latest advances and future trends.. *Nucleic Acids Res*, 2007, vol. 35, 4223-37
- **CHENG J** ; **SHOFFNER MA** ; **HVICHIA GE** ; **KRICKA LJ** ; **WILDING P.** Chip PCR. II. Investigation of different PCR amplification systems in microfabricated silicon-glass chips.. *Nucleic Acids Res*, 1996, vol. 24, 380-5
- **WOOLLEY AT** ; **HADLEY D** ; **LANDRE P** ; **DEMELLO AJ** ; **MATHIES RA** ; **NORTHRUP MA.** Functional integration of PCR amplification and capillary electrophoresis in a microfabricated DNA analysis device.. *Anal Chem*, 1996, vol. 68, 4081-6

- **NEUZIL P** ; **ZHANG C** ; **PIPPER J** ; **OH S** ; **ZHUO L.** Ultra fast miniaturized real-time PCR: 40 cycles in less than six minutes.. *Nucleic Acids Res*, 2006, vol. 34, e77
- **ODA RP** ; **STRAUSBAUCH MA** ; **HUHMER AF** ; **BORSON N** ; **JURRENS SR** ; **CRAIGHEAD J et al.** Infrared-mediated thermocycling for ultrafast polymerase chain reaction amplification of DNA.. *Anal Chem*, 1998, vol. 70, 4361-8
- **ROPER MG** ; **EASLEY CJ** ; **LEGENDRE LA** ; **HUMPHREY JA** ; **LANDERS JP.** Infrared temperature control system for a completely noncontact polymerase chain reaction in microfluidic chips.. *Anal Chem*, 2007, vol. 79, 1294-300
- **FRIEDMAN NA** ; **MELDRUM DR.** Capillary tube resistive thermal cycling.. *Anal Chem*, 1998, vol. 70, 2997-3002
- **HEAP DM** ; **HERRMANN MG** ; **WITTWER CT.** PCR amplification using electrolytic resistance for heating and temperature monitoring.. *Biotechniques*, 2000, vol. 29, 1006-12
- **KOPP MU** ; **MELLO AJ** ; **MANZ A.** Chemical amplification: continuous-flow PCR on a chip.. *Science*, 1998, vol. 280, 1046-8
- **HASHIMOTO M** ; **CHEN PC** ; **MITCHELL MW** ; **NIKITOPOULOS DE** ; **SOPER SA** ; **MURPHY MC.** Rapid PCR in a continuous flow device.. *Lab Chip*, 2004, vol. 4, 638-45
- **CREWS N** ; **WITTWER C** ; **GALE B.** Continuous-flow thermal gradient PCR.. *Biomed Microdevices*, 2008, vol. 10, 187-95
- **CHIOU JT** ; **MATSUDAIRA PT** ; **EHRLICH DJ.** Thirty-cycle temperature optimization of a closed-cycle capillary PCR machine.. *Biotechniques*, 2002, vol. 33, 557-8, 60, 62
- **FREY O** ; **BONNEICK S** ; **HIERLEMANN A** ; **LICHTENBERG J.** Autonomous microfluidic multichannel chip for real-time PCR with integrated liquid handling.. *Biomed Microdevices*, 2007, vol. 9, 711-8
- **CHEN J** ; **WABUYELE M** ; **CHEN H** ; **PATTERSON D** ; **HUPERT M** ; **SHADPOUR H et al.** Electrokinetically synchronized polymerase chain reaction microchip fabricated in polycarbonate. *Anal Chem*, 2005, vol. 77, 658-66
- **SUN Y** ; **KWOK YC** ; **NGUYEN NT.** A circular ferrofluid driven microchip for rapid polymerase chain reaction.. *Lab Chip*, 2007, vol. 7, 1012-7
- **AGRAWAL N** ; **HASSAN YA** ; **UGAZ VM.** A pocket-sized convective PCR thermocycler.. *Angew Chem Int Ed Engl*, 2007, vol. 46, 4316-9
- **ZHANG C** ; **XU J** ; **MA W** ; **ZHENG W**. PCR microfluidic devices for DNA amplification.. *Biotechnol Adv*, 2006, vol. 24, 243-84
- **WHEELER EK** ; **BENETT W** ; **STRATTON P** ; **RICHARDS J** ; **CHEN A** ; **CHRISTIAN A et al.** Convectively driven polymerase chain reaction thermal cycler.. *Anal Chem*, 2004, vol. 76, 4011-6
- **BELGRADER P** ; **BENETT W** ; **HADLEY D** ; **LONG G** ; **MARIELLA R, JR.** ; **MILANOVICH F et al.** Rapid pathogen detection using a microchip PCR array instrument. *Clin Chem*, 1998, vol. 44, 2191-4
- **TERAZONA H** ; **TAKEI, H** ; **HATTORI A** ; **YASUDA K.** Development of a high-speed real-time polymerase chain reaction system using a circulating water-based rapid heat exchange.. *Jap J Appl Phys*, 2010, vol. 49
- **WHEELER EK** ; **HARA CA** ; **FRANK J** ; **DEOTTE J** ; **HALL SB** ; **BENETT W** ; **SPADACCINI C** ; **BEER NR.** Under-three minute PCR: Probing the limits of fast amplification.. *Analyst*, 2011, vol. 136 (16), 3707-12
- **FUCHIWAKI Y** ; **NAGAI H** ; **SAITO M** ; **TAMIYA E.** Ultra-rapid flow-through polymerase chain reaction microfluidics using vapor pressure.. *Biosens Bioelect*, 2011, vol. 27, 88-94
- **MALTEZOS G** ; **JOHNSTON M** ; **TAGANOV K** ; **SRICHANTARATSAMEE C** ; **GORMAN J** ; **BALTIMORE D** ; **CHANTRATITA W** ; **SCHERER A**. Exploring the limits of ultrafast polymerase chain reaction using liquid for thermal heat exchange: A proof of principle. *Appl. Phys. Lett.*, 2010, vol. 97, 264101
- **WILHELM J** ; **HAHN M** ; **PINGOUD A.** Influence of DNA target melting behavior on real-time PCR quantification.. *Clin Chem*, 2000, vol. 46, 1738-43
- **ZUNA J** ; **MUZIKOVA K** ; **MADZO J** ; **KREJCI O** ; **TRKA J.** Temperature non-homogeneity in rapid airflow-based cycler significantly affects real-time PCR.. *Biotechniques*, 2002, vol. 33, 508, 10, 12
- **VON KANEL T** ; **ADOLF F** ; **SCHNEIDER M** ; **SANZ J** ; **GALLATI S.** Sample number and denaturation time are crucial for the accuracy of capillary-based LightCyclers.. *Clin Chem*, 2007, vol. 53, 1392-4
- Rapid thermal cycling and PCR kinetics.. **WITTWER CT** ; **HERRMANN MG.** PCR Methods Manual. Academic Press, 1999, 211-29
- **WITTWER CT** ; **REED GH** ; **GUNDRY CN** ; **VANDERSTEEN JG** ; **PRYOR RJ.** High-resolution genotyping by amplicon melting analysis using LCGreen.. *Clin Chem*, 2003, vol. 49, 853-60
- **VON AHSEN N** ; **WITTWER CT** ; **SCHUTZ E.** Oligonucleotide melting temperatures under PCR conditions: nearest-neighbor corrections for Mg(2+), deoxynucleotide triphosphate, and dimethyl sulfoxide concentrations with comparison to alternative empirical formulas. *Clin Chem*, 2001, vol. 47, 1956-61
- **RIRIE KM** ; **RASMUSSEN RP** ; **WITTWER CT.** Product differentiation by analysis of DNA melting curves during the polymerase chain reaction.. *Anal Biochem*, 1997, vol. 245, 154-60

- **WITTWER CT** ; **HERRMANN MG** ; **MOSS AA** ; **RASMUSSEN RP.** Continuous fluorescence monitoring of rapid cycle DNA amplification.. *Biotechniques*, 1997, vol. 22, 130-1, 4-8
- **WEIS JH** ; **TAN SS** ; **MARTIN BK** ; **WITTWER CT.** Detection of rare mRNAs via quantitative RT-PCR.. *Trends Genet*, 1992, vol. 8, 263-4
- Rapid cycle amplification for construction of competitive templates.. **BROWN RA** ; **LAY MJ** ; **WITTWER CT.** Genetic Engineering with PCR. Horizon Scientific Press, 1998, 57-70
- **AUSUBEL et al.** Current Protocols in Molecular Biology. John Wiley & Sons, 1998
- Analysis of rapid thermocycling for the polymerase chain reaction. **WHITNEY SE**. Ph.D. thesis. University of Nebraska, 2004
- **LAWYER FC** ; **STOFFEL S** ; **SAIKI RK** ; **CHANG SY** ; **LANDRE PA** ; **ABRAMSON RD** ; **GELFAND DH**. High-level expression, purification and enzymatic characterization of full-length Thermus aquaticus DNA polymerase and a truncated form deficient of 5' to 3' exonuclease activity.. *PCR Meth Appl.*, 1993, vol. 2, 275-287
- **INNIS MA** ; **MYAMO KB** ; **GELFAND DH** ; **BROW MAD.** DNA sequencing with Thermus aquaticus DNA polymerase and direct sequencing of polymerase chain reaction-amplified DNA.. *Proc. Natl. Acad. Sci USA*, 1988, vol. 85, 9436-40
- **TERAZONO H** ; **HATTORI A** ; **TAKEI H** ; **TAKEDA K** ; **YASUDA K.** Development of 1480 nm photothermal high-speed real-time polymerase chain reaction system for rapid nucleotide recognition.. *Jpn J Appl Phys.*, 2008, vol. 47, 5212-6
- Rapid PCR and melting curve analysis.. **WITTWER CT** ; **RASMUSSEN RP** ; **RIRIE KM.** The PCR Revolution: Basic Technologies and Applications. Cambridge Univ Press, 2010, 48-69
- **FUCHIWAKI Y** ; **SAITO M** ; **WAKIDA S** ; **TAMIYA E** ; **NAGAI H.** A practical liquid plug flow-through polymerase chain-reaction system based on a heat-resistant resin chip.. *Anal Sci.*, 2011, vol. 27, 225-30
- **KIM H** ; **DIXIT S** ; **GREEN CJ** ; **FARIS GW.** Nanodroplet real-time PCR system with laser assisted heating.. *Optics Express*, 2009, vol. 17, 218-27
- **OBEID PJ** ; **CHRISTOPOULOS TK** ; **CRABTREE HJ** ; **BACKHOUSE CJ.** Microfabricated device for DNA and RNA amplification by continuous-flow polymerase chain reaction and reverse transcription-polymerase chain reaction with cycle number selection. *Anal Chem*, 2003, vol. 75, 288-95
- **GIORDANO BC** ; **FERRANCE J** ; **SWEDBERG S** ; **HUHMER AFR** ; **LANDERS JP.** Polymerase chain reaction in polymeric microchips: DNA amplification in less than 240 seconds.. *Anal Biochem*, 2001, vol. 291, 124-132
- **PAL, D.** ; **VENKATARAMAN, V.** ; **MOHAN, K. N.** ; **CHANDRA, H. S.** ; **NATARAJAN, V.** A power-efficient thermocycler based on induction heating for DNA amplification by polymerase chain reaction.. *Review of Scientific Instruments*, 2004, vol. 75 (9), 2880-2883
- **YUANZHI LAO, F. E. H.** ; **TAY, F. E. H.** ; **GUOLIN XU** ; **HARTONO, D.** ; **LEE, Y. Y.** A Non-Contact Micro Thermocycling Chip for Polymerase Chain Reactions.. *International Journal of Computational Engineering Science*, 2003, vol. 4 (3), 651-654
- **BUSTIN, S et al.** Variability of the reverse transcription step: practical implications. *Clinical Chemistry*, 2015, vol. 61, 201-12
- **MONTGOMERY JL** ; **WITTWER CT**. Influence of PCR reagents on DNA polymerase extension rates measured on real-time PCR instruments. *Clin Chem*, 2014, vol. 60, 334-340
- **MIFATOVIC-RUSTEMPASIC S et al.** Sensitive and specific quantitative detection of rotavirus A by one-step real-time reverse transcription-PCR assay without antecedent double-stranded-RNA denaturation. *J Clin Microbiol*, 2013, vol. 51, 3047-54
- **CARNINCI P et al.** Thermostabilization and thermoactivation of thermolabele enzymes by trehalose and its application for the synthesis of full length cDNA. *PNAS*, 1998, vol. 95, 520-4
- **AN SPIESS et al.** Therhalose is a potent enhancer: Lowering of DNA melting temperature and thermal stabilizationof Taq polymerase by the disaccharide trihalose. *Clin Chem*, 2004, vol. 50, 1256-9
- Enzymes of Molecular Biology. **GERARD GF** ; **D'ALESSIO JM.** Methods in Molecular Biology. Humana Press, Inc., 1993, vol. 16
- **TONG Y et al.** Multiple strategies to improve sensitivity, speed and robustness of isothermal nucleic acid amplification for rapid pathogen detection. *BMC Biotechnology*, 2011, vol. 11, 50
- **SASAKI Y et al.** Effect of molecular crowding on DNA polymerase Activity. *Biotechnol J*, 2006, vol. 1, 440-6